# EUROPEAN PATENT SPECIFICATION

(11) **EP 1 480 671 B1**
(45) Date of publication and mention of the grant of the patent: **08.08.2007**
(21) Application number: 01986054.3
(22) Date of filing: 28.11.2001
(51) Int. Cl.: A61K 39/155, C07K 14/135, C12N 7/04

(54) **RECOMBINANT RSV VIRUS EXPRESSION SYSTEMS AND VACCINES**
REKOMBINANTE RSV-VIRUS-EXPRESSIONSSYSTEME UND -IMPFSTOFFE
SYSTEMES D'EXPRESSION DE VIRUS RSV DE RECOMBINAISON ET VACCINS

(30) Priority: 28.11.2000 US 724416
(43) Date of publication of application: 01.12.2004
(73) Proprietor: MedImmune Vaccines, Inc., Mountain View, CA 94043 (US)
(72) Inventor: JIN, Hong, Cupertino, CA 95014 (US); TANG, Roderick, San Mateo, CA 94403-1273 (US); LI, Shengqiang, Los Altos, CA 94024 (US); BRYANT, Martin, Carlisle, MA 01741 (US)
(74) Representative: Chapman, Paul Gilmour
(86) International application number: PCT/US2001/044819
(87) International publication number: WO 2002/044334

(56) References cited:
- WO-A-01/04321
- WO-A-99/15631
- WO-A1-97/12032
- BERMINGHAM ALISON ET AL: "The M2-2 protein of human respiratory syncytial virus is a regulatory factor involved in the balance between RNA replication and transcription" PROCEEDINGS OF THE NATIONAL ACADEMY OF SCIENCES OF USA, NATIONAL ACADEMY OF SCIENCE. WASHINGTON, US, vol. 96, no. 20, 28 September 1999 (1999-09-28), pages 11259-11264, XP002154525 ISSN: 0027-8424
- JIN H ET AL: "Recombinant Respiratory Syncytial Viruses with Deletions in the NS1, NS2, SH, and M2-2 Genes Are Attenuated in Vitro and in Vivo" VIROLOGY, ACADEMIC PRESS,ORLANDO, US, vol. 273, no. 1, 20 July 2000 (2000-07-20), pages 210-218, XP004435993 ISSN: 0042-6822
- CHENG XING ET AL: "Chimeric subgroup A respiratory syncytial virus with the glycoproteins substituted by those of subgroup B and RSV without the M2-2 gene are attenuated in African green monkeys" VIROLOGY, vol. 283, no. 1, 23 April 2001 (2001-04-23), pages 59-68, XP002322975 ISSN: 0042-6822
- COLLINS ET AL.: 'Production of infectious human respiratory syncytial virus from cloned cDNA confirms an essential role for the transcription elongation factor from the 5' proximal open reading frame of the M2 mRNA in gene expression and provides a capability for ....' PROC. NATL. ACAD. SCI. USA vol. 92, December 1995, pages 11563 - 11567, XP002950497
- WHITEHEAD ET AL.: 'Replacement of the F and G proteins of respiratory syncytial virus (RSV) subgroup A with those of subgroup B generates chimeric live attenuated RSV subgroup B vaccine candidates' JOURNAL OF VIROLOGY vol. 73, no. 12, December 1999, pages 9773 - 9780, XP002950498

### SEQUENCE LISTING

<110> Aviron, Inc.
<120> Recombinant RSV virus Expression Systems and vaccines
<130> PC/P12940EP
<140> EP01986054
   <141> 2001-11-28
<150> PCT/US01/44819
   <151> 2001-11-28
<150> 09/724,416
   <151> 2000-11-28
<160> 66
<170> PatentIn version 3.0
<210> 1
   <211> 46
   <212> DNA
   <213> ARTIFICIAL SEQUENCE
<220>
   <223> Description of Artificial Sequence: Primer
<400> 1
   cgacgcatat tacgcgaaaa aatgcgtaca acaaacttgc ataaac 46
<210> 2
   <211> 50
   <212> DNA
   <213> ARTIFICIAL SEQUENCE
<220>
   <223> Description of Artificial sequence: Primer
<400> 2
   caaaaaaatg gggcaaataa gaatttgata agtaccactt aaatttaact 50
<210> 3
   <211> 24
   <212> DNA
   <213> ARTIFICIAL SEQUENCE
<220>
   <223> Description of Artificial Sequence: Primer
<400> 3
   ctagagttaa atttaagtgg tact 24
<210> 4
   <211> 50
   <212> DNA
   <213> ARTIFICIAL SEQUENCE
<220>
   <223> Description of Artificial Sequence: Primer
<400> 4
   tatcaaattc ttatttgccc catttttttg gtttatgcaa gtttgttgta 50
<210> 5
   <211> 30
   <212> DNA
   <213> ARTIFICIAL SEQUENCE
<220>
   <223> Description of Artificial Sequence: Primer
<400> 5
   cgcatttttt cgcgtaatat gcgtcggtac 30
<210> 6
   <211> 50
   <212> DNA
   <213> ARTIFICIAL SEQUENCE
<220>
   <223> Description of Artificial Sequence: Primer
<400> 6
   gtattcaatt atagttatta aaaattaaaa atcatataat tttttaaata 50
<210> 7
   <211> 50
   <212> DNA
   <213> ARTIFICIAL SEQUENCE
<220>
   <223> Description of Artificial Sequence: Primer
<400> 7
   acttttagtg aactaatcct aaagttatca ttttaatctt ggaggaataa 50
<210> 8
   <211> 50
   <212> DNA
   <213> ARTIFICIAL SEQUENCE
<220>
   <223> Description of Artificial sequence: Primer
<400> 8
   atttaaaccc taatctaatt ggtttatatg tgtattaact aaattacgag 50
<210> 9
   <211> 46
   <212> DNA
   <213> ARTIFICIAL SEQUENCE
<220>
   <223> Description of Artificial sequence: Primer
<400> 9
   atattagttt ttgacacttt ttttctcgtt atagtgagtc gtatta 46
<210> 10
   <211> 25
   <212> DNA
   <213> ARTIFICIAL SEQUENCE
<220>
   <223> Description of Artificial Sequence: Primer
<400> 10
   agcttaatac gactcactat aacga 25
<210> 11
   <211> 50
   <212> DNA
   <213> ARTIFICIAL SEQUENCE
<220>
   <223> Description of Artificial Sequence: Primer
<400> 11
   gaaaaaaagt gtcaaaaact aatatctcgt aatttagtta atacacatat 50
<210> 12
   <211> 50
   <212> DNA
   <213> ARTIFICIAL SEQUENCE
<220>
   <223> Description of Artificial Sequence: Primer
<400> 12
   aaaccaatta gattagggtt taaatttatt cctccaagat taaaatgata 50
<210> 13
   <211> 50
   <212> DNA
   <213> ARTIFICIAL SEQUENCE
<220>
   <223> Description of Artificial Sequence: Primer
<400> 13
   actttaggat tagttcacta aaagttattt aaaaaattat atgattttta 50
<210> 14
   <211> 29
   <212> DNA
   <213> ARTIFICIAL SEQUENCE
<220>
   <223> Description of Artificial Sequence: Primer
<400> 14
   atttttaata actataattg aatactgca 29
<210> 15
   <211> 40
   <212> DNA
   <213> ARTIFICIAL SEQUENCE
<220>
   <223> Description of Artificial sequence: olionucleotide
<400> 15
   ggtggccggc atggtcccag cgctggggac catgccggcc 40
<210> 16
   <211> 48
   <212> DNA
   <213> ARTIFICIAL SEQUENCE
<220>
   <223> Description of Artificial sequence: olionucleotide
<400> 16
   ctcgctggcg ccggctgggc aacagtgtgc ccagccggcg ccagcgag 48
<210> 17
   <211> 48
   <212> DNA
   <213> ARTIFICIAL SEQUENCE
<220>
   <223> Description of Artificial Sequence: Olionucleotide
<400> 17
   ttccgagggg accgtcccct cggtaccgag gggacggtcc cctcggaa 48
<210> 18
   <211> 48
   <212> DNA
   <213> ARTIFICIAL SEQUENCE
<220>
   <223> Description of Artificial Sequence: olionucleotide
<400> 18
   aatggcgaat gggacgtcga cagcgctgtc gacgtcccat tcgccatt 48
<210> 19
   <211> 42
   <212> DNA
   <213> ARTIFICIAL SEQUENCE
<220>
   <223> Description of Artificial Sequence: olionucleotide
<400> 19
   taacaaagcc cgaaggaagc tagcttcctt cgggctttgt ta 42
<210> 20
   <211> 42
   <212> DNA
   <213> ARTIFICIAL SEQUENCE
<220>
   <223> Description of Artificial Sequence: olionucleotide
<400> 20
   gagttgctgc tgccaccgtt gcaacggagg cagcagcaac tc 42
<210> 21
   <211> 46
   <212> DNA
   <213> ARTIFICIAL SEQUENCE
<220>
   <223> Description of Artificial Sequence: Olionucleotide
<400> 21
   agcaataact agataacctt gggcccaagg ttatctagtt attgct 46
<210> 22
   <211> 48
   <212> DNA
   <213> ARTIFICIAL SEQUENCE
<220>
   <223> Description of Artificial sequence: olionucleotide
<400> 22
   cctctaaacg ggtcttgagg gtctagaccc tcaagacccg tttagagg 48
<210> 23
   <211> 42
   <212> DNA
   <213> ARTIFICIAL SEQUENCE
<220>
   <223> Description of Artificial Sequence: olionucleotide
<400> 23
   ttttgctgaa aggaggaact atagttcctc ctttcagcaa aa 42
<210> 24
   <211> 42
   <212> DNA
   <213> ARTIFICIAL SEQUENCE
<220>
   <223> Description of Artificial sequence: Olionucleotide
<400> 24
   tatgcggccg cgtcgacggt ataccgtcga cgcggccgca ta 42
<210> 25
   <211> 36
   <212> DNA
   <213> ARTIFICIAL SEQUENCE
<220>
   <223> Description of Artificial sequence: olionucleotide
<400> 25
   ccgggcccgc cttcgaagct tcgaaggcgg gcccgg 36
<210> 26
   <211> 21
   <212> DNA
   <213> ARTIFICIAL SEQUENCE
<220>
   <223> Description of Artificial sequence: Primer
<400> 26
   caccacctac cttactcaag t 21
<210> 27
   <211> 24
   <212> DNA
   <213> ARTIFICIAL SEQUENCE
<220>
   <223> Description of Artificial Sequence: Primer
<400> 27
   tttgtttgtg ggtttgatgg ttgg 24
<210> 28
   <211> 35
   <212> DNA
   <213> ARTIFICIAL SEQUENCE
<220>
   <223> Description of Artificial Sequence: Primer
<400> 28
   gatatcaaga tctacaataa cattggggca aatgc 35
<210> 29
   <211> 31
   <212> DNA
   <213> ARTIFICIAL SEQUENCE
<220>
   <223> Description of Artificial Sequence: Primer
<400> 29
   gctaagagat ctttttgaat aactaagcat g 31
<210> 30
   <211> 46
   <212> DNA
   <213> ARTIFICIAL SEQUENCE
<220>
   <223> Description of Artificial Sequence: Primer
<400> 30
   tcttgactgt tgtggattgc agggttgact tgactccgat cgatcc 46
<210> 31
   <211> 49
   <212> DNA
   <213> ARTIFICIAL SEQUENCE
<220>
   <223> Description of Artificial Sequence: Primer
<400> 31
   cttgtgttgt tgttgtatgg tgtgtttctg attttgtatt gatcgatcc 49
<210> 32
   <211> 33
   <212> DNA
   <213> ARTIFICIAL SEQUENCE
<220>
   <223> Description of Artificial Sequence: Primer
<400> 32
   tcacgaagga atcctggcaa atttgaaatt cga 33
<210> 33
   <211> 33
   <212> DNA
   <213> ARTIFICIAL SEQUENCE
<220>
   <223> Description of Artificial Sequence: Primer
<400> 33
   gaaattcgag gtcatggttt aaatggtaag agg 33
<210> 34
   <211> 36
   <212> DNA
   <213> ARTIFICIAL SEQUENCE
<220>
   <223> Description of Artificial Sequence: Primer
<400> 34
   tgcttaaatg gtaagagggg acattttagt cataat 36
<210> 35
   <211> 33
   <212> DNA
   <213> ARTIFICIAL SEQUENCE
<220>
   <223> Description of Artificial Sequence: Primer
<400> 35
   actaaacaat cagcaggtgt tgccatgagc aaa 33
<210> 36
   <211> 52
   <212> DNA
   <213> ARTIFICIAL SEQUENCE
<220>
   <223> Description of Artificial Sequence: Primer
<400> 36
   gagctaaatt cacccaagat aagcttgtaa taaactgtca tatcatatat tg 52
<210> 37
   <211> 42
   <212> DNA
   <213> ARTIFICIAL SEQUENCE
<220>
   <223> Description of Artificial sequence: Primer
<400> 37
   caaactatcc atctgtaata aagcttgcca gcagacgtat tg 42
<210> 38
   <211> 50
   <212> DNA
   <213> ARTIFICIAL SEQUENCE
<220>
   <223> Description of Artificial sequence: Primer
<400> 38
   ccatcaacaa cccaaaataa taaagcttta gtgatacaaa tgaccatgcc 50
<210> 39
   <211> 36
   <212> DNA
   <213> ARTIFICIAL SEQUENCE
<220>
   <223> Description of Artificial Sequence: Primer
<400> 39
   atcaggatcc acaataacat tggggcaaat gcaacc 36
<210> 40
   <211> 36
   <212> DNA
   <213> ARTIFICIAL SEQUENCE
<220>
   <223> Description of Artificial Sequence: Primer
<400> 40
   ctggcattcg gatccgtttt atgtaactat gagttg 36
<210> 41
   <211> 40
   <212> DNA
   <213> ARTIFICIAL SEQUENCE
<220>
   <223> Description of Artificial Sequence: Primer
<400> 41
   gaggtgaggt acaatgcatt aatagcaaga tggaggaaga 40
<210> 42
   <211> 40
   <212> DNA
   <213> ARTIFICIAL SEQUENCE
<220>
   <223> Description of Artificial Sequence: Primer
<400> 42
   cagaagcaaa acaaaatgtg actgcagtga ggattgtggt 40
<210> 43
   <211> 17
   <212> DNA
   <213> ARTICIFICIAL SEQUENCE
<220>
   <223> Description of artificial sequence: Primer
<400> 43
   gtttaacacg tggtgag 17
<210> 44
   <211> 17
   <212> DNA
   <213> ARTICIFICIAL SEQUENCE
<220>
   <223> Description of artificial sequence: Primer
<400> 44
   acatataggc atgcacc 17
<210> 45
   <211> 17
   <212> DNA
   <213> ARTICIFICIAL SEQUENCE
<220>
   <223> Description of artificial sequence: Primer
<400> 45
   gcaaaatgga tcccatt 17
<210> 46
   <211> 18
   <212> DNA
   <213> ARTICIFICIAL SEQUENCE
<220>
   <223> Description of artificial sequence: Primer
<400> 46
   tggttggtat accagtgt 18
<210> 47
   <211> 18
   <212> DNA
   <213> ARTICIFICIAL SEQUENCE
<220>
   <223> Description of artificial sequence: Primer
<400> 47
   taccaagagc tcgagtca 18
<210> 48
   <211> 21
   <212> DNA
   <213> ARTICIFICIAL SEQUENCE
<220>
   <223> Description of artificial sequence: Primer
<400> 48
   ggtggccggc atggtcccag c 21
<210> 49
   <211> 20
   <212> DNA
   <213> ARTICIFICIAL SEQUENCE
<220>
   <223> Description of artificial sequence: Primer
<400> 49
   tttaccatat gcgctaatgt 20
<210> 50
   <211> 19
   <212> DNA
   <213> ARTICIFICIAL SEQUENCE
<220>
   <223> Description of artificial sequence: Primer
<400> 50
   acgcgaaaaa atgcgtaca 19
<210> 51
   <211> 18
   <212> DNA
   <213> ARTICIFICIAL SEQUENCE
<220>
   <223> Description of artificial sequence: Primer
<400> 51
   acgagaaaaa agtggcaa 18
<210> 52
   <211> 17
   <212> DNA
   <213> ARTICIFICIAL SEQUENCE
<220>
   <223> Description of artificial sequence: Primer
<400> 52
   ctcaccacgt gttaaac 17
<210> 53
   <211> 17
   <212> DNA
   <213> ARTICIFICIAL SEQUENCE
<220>
   <223> Description of artificial sequence: Primer
<400> 53
   ggtgcatgcc tatatgt 17
<210> 54
   <211> 19
   <212> DNA
   <213> ARTICIFICIAL SEQUENCE
<220>
   <223> Description of artificial sequence: Primer
<400> 54
   aatgggatcc attttgtcc 19
<210> 55
   <211> 19
   <212> DNA
   <213> ARTICIFICIAL SEQUENCE
<220>
   <223> Description of artificial sequence: Primer
<400> 55
   aacactggta taccaacca 19
<210> 56
   <211> 20
   <212> DNA
   <213> ARTICIFICIAL SEQUENCE
<220>
   <223> Description of artificial sequence: Primer
<400> 56
   acattagcgc atatggtaaa 20
<210> 57
   <211> 36
   <212> DNA
   <213> ARTIFICIAL SEQUENCE
<220>
   <223> Description of artificial sequence: Primer
<400> 57
   atcaggatcc acaataacat tggggcaaat gcaacc 36
<210> 58
   <211> 36
   <212> DNA
   <213> ARTIFICIAL SEQUENCE
<220>
   <223> Description of artificial sequence: Primer
<400> 58
   caactcatag ttacataaaa cggatccgaa tgccat 36
<210> 59
   <211> 2165
   <212> PRT
   <213> virus
<400> 59
   Met Asp Pro Ile Ile Asn Gly Asn Ser Ala Asn Val Tyr Leu Thr Asp
    1               5                  10                  15
   Ser Tyr Leu Lys Gly Val Ile Ser Phe Ser Glu Cys Asn Ala Leu Gly
               20                  25                  30
   Ser Tyr Ile Phe Asn Gly Pro Tyr Leu Lys Asn Asp Tyr Thr Asn Leu
           35                  40                  45
   Ile Ser Arg Gln Asn Pro Leu Ile Glu His Met Asn Leu Lys Lys Leu
       50                  55                  60
   Asn Ile Thr Gln Ser Leu Ile Ser Lys Tyr His Lys Gly Glu Ile Lys
   65                  70                  75                  80
   Leu Glu Glu Pro Thr Tyr Phe Gln Ser Leu Leu Met Thr Tyr Lys Ser
                   85                  90                  95
   Met Thr Ser Ser Glu Gln Ile Ala Thr Thr Asn Leu Leu Lys Lys Ile
               100                 105                 110
   Ile Arg Arg Ala Ile Glu Ile Ser Asp Val Lys Val Tyr Ala Ile Leu
               115                 120                 125
   Asn Lys Leu Gly Leu Lys Glu Lys Asp Lys Ile Lys Ser Asn Asn Gly
       130                 135                 140
   Gln Asp Glu Asp Asn Ser Val Ile Thr Thr Ile Ile Lys Asp Asp Ile
   145                 150                 155                 160
   Leu Ser Ala Val Lys Asp Asn Gln Ser His Leu Lys Ala Asp Lys Asn
                   165                 170                 175
   His Ser Thr Lys Gln Lys Asp Thr Ile Lys Thr Thr Leu Leu Lys Lys
               180                 185                 190
   Leu Met Cys Ser Met Gln His Pro Pro Ser Trp Leu Ile His Trp Phe
           195                 200                 205
   Asn Leu Tyr Thr Lys Leu Asn Asn Ile Leu Thr Gln Tyr Arg Ser Asn
       210                 215                 220
   Glu Val Lys Asn His Gly Phe Thr Leu Ile Asp Asn Gln Thr Leu Ser
   225                 230                 235                 240
   Gly Phe Gln Phe Ile Leu Asn Gln Tyr Gly Cys Ile Val Tyr His Lys
                   245                 250                 255
   Glu Leu Lys Arg Ile Thr Val Thr Thr Tyr Asn Gln Phe Leu Thr Trp
               260                 265                 270
   Lys Asp Ile Ser Leu Ser Arg Leu Asn Val Cys Leu Ile Thr Trp Ile
           275                 280                 285
   Ser Asn Cys Leu Asn Thr Leu Asn Lys Ser Leu Gly Leu Arg Cys Gly
       290                 295                 300
   Phe Asn Asn Val Ile Leu Thr Gln Leu Phe Leu Tyr Gly Asp Cys Ile
   305                 310                 315                 320
   Leu Lys Leu Phe His Asn Glu Gly Phe Tyr Ile Ile Lys Glu Val Glu
                   325                 330                 335
   Gly Phe Ile Met Ser Leu Ile Leu Asn Ile Thr Glu Glu Asp Gln Phe
               340                 345                 350
   Arg Lys Arg Phe Tyr Asn Ser Met Leu Asn Asn Ile Thr Asp Ala Ala
           355                 360                 365
   Asn Lys Ala Gln Lys Asn Leu Leu Ser Arg Val Cys His Thr Leu Leu
       370                 375                 380
   Asp Lys Thr Val Ser Asp Asn Ile Ile Asn Gly Arg Trp Ile Ile Leu
   385                 390                 395                 400
   Leu Ser Lys Phe Leu Lys Leu Ile Lys Leu Ala Gly Asp Asn Asn Leu
                   405                 410                 415
   Asn Asn Leu Ser Glu Leu Tyr Phe Leu Phe Arg Ile Phe Gly His Pro
               420                 425                 430
   Met Val Asp Glu Arg Gln Ala Met Asp Ala Val Lys Ile Asn Cys Asn
           435                 440                 445
   Glu Thr Lys Phe Tyr Leu Leu Ser Ser Leu Ser Met Leu Arg Gly Ala
       450                 455                 460
   Phe Ile Tyr Arg Ile Ile Lys Gly Phe Val Asn Asn Tyr Asn Arg Trp
   465                 470                 475                 480
   Pro Thr Leu Arg Asn Ala Ile Val Leu Pro Leu Arg Trp Leu Thr Tyr
                   485                 490                 495
   Tyr Lys Leu Asn Thr Tyr Pro Ser Leu Leu Glu Leu Thr Glu Arg Asp
               500                 505                 510
   Leu Ile Val Leu Ser Gly Leu Arg Phe Tyr Arg Glu Phe Arg Leu Pro
           515                 520                 525
   Lys Lys Val Asp Leu Glu Met Ile Ile Asn Asp Lys Ala Ile Ser Pro
       530                 535                 540
   Pro Lys Asn Leu Ile Trp Thr Ser Phe Pro Arg Asn Tyr Met Pro Ser
   545                 550                 555                 560
   His Ile Gln Asn Tyr Ile Glu His Glu Lys Leu Lys Phe Ser Glu Ser
                   565                 570                 575
   Asp Lys Ser Arg Arg Val Leu Glu Tyr Tyr Leu Arg Asp Asn Lys Phe
               580                 585                 590
   Asn Glu Cys Asp Leu Tyr Asn Cys Val Val Asn Gln Ser Tyr Leu Asn
           595                 600                 605
   Asn Pro Asn His Val Val Ser Leu Thr Gly Lys Glu Arg Glu Leu Ser
       610                 615                 620
   Val Gly Arg Met Phe Ala Met Gln Pro Gly Met Phe Arg Gln Val Gln
   625                 630                 635                 640
   Ile Leu Ala Glu Lys Met Ile Ala Glu Asn Ile Leu Gln Phe Phe Pro
                   645                 650                 655
   Glu Ser Leu Thr Arg Tyr Gly Asp Leu Glu Leu Gln Lys Ile Leu Glu
                   660                 665                 670
   Leu Lys Ala Gly Ile Ser Asn Lys Ser Asn Arg Tyr Asn Asp Asn Tyr
               675                 680                 685
   Asn Asn Tyr Ile Ser Lys Cys Ser Ile Ile Thr Asp Leu Ser Lys Phe
       690                 695                 700
   Asn Gln Ala Phe Arg Tyr Glu Thr Ser Cys Ile Cys Ser Asp Val Leu
   705                 710                 715                 720
   Asp Glu Leu His Gly Val Gln Ser Leu Phe Ser Trp Leu His Leu Thr
                   725                 730                 735
   Ile Pro His Val Thr Ile Ile Cys Thr Tyr Arg His Ala Pro Pro Tyr
               740                 745                 750
   Ile Gly Asp His Ile Val Asp Leu Asn Asn Val Asp Glu Gln Ser Gly
           755                 760                 765
   Leu Tyr Arg Tyr His Met Gly Gly Ile Glu Gly Trp Cys Gln Lys Leu
       770                 775                 780
   Trp Thr Ile Glu Ala Ile Ser Leu Leu Asp Leu Ile Ser Leu Lys Gly
   785                 790                 795                 800
   Lys Phe Ser Ile Thr Ala Leu Ile Asn Gly Asp Asn Gln Ser Ile Asp
                   805                 810                 815
   Ile Ser Lys Pro Ile Arg Leu Met Glu Gly Gln Thr His Ala Gln Ala
               820                 825                 830
   Asp Tyr Leu Leu Ala Leu Asn Ser Leu Lys Leu Leu Tyr Lys Glu Tyr
           835                 840                 845
   Ala Gly Ile Gly His Lys Leu Lys Gly Thr Glu Thr Tyr Ile Ser Arg
       850                 855                 860
   Asp Met Gln Phe Met Ser Lys Thr Ile Gln His Asn Gly Val Tyr Tyr
   865                 870                 875                 880
   Pro Ala Ser Ile Lys Lys Val Leu Arg Val Gly Pro Trp Ile Asn Thr
                   885                 890                 895
   Ile Leu Asp Asp Phe Lys Val Ser Leu Glu Ser Ile Gly Ser Leu Thr
               900                 905                 910
   Gln Glu Leu Glu Tyr Arg Gly Glu Ser Leu Leu Cys Ser Leu Ile Phe
           915                 920                 925
   Arg Asn Val Trp Leu Tyr Asn Gln Ile Ala Leu Gln Leu Lys Asn His
       930                 935                 940
   Ala Leu Cys Asn Asn Lys Leu Tyr Leu Asp Ile Leu Lys Val Leu Lys
   945                 950                 955                 960
   His Leu Lys Thr Phe Phe Asn Leu Asp Asn Ile Asp Thr Ala Leu Thr
                   965                 970                 975
   Leu Tyr Met Asn Leu Pro Met Leu Phe Gly Gly Gly Asp Pro Asn Leu
               980                 985                 990
   Leu Tyr Arg Ser Phe Tyr Arg Arg Thr Pro Asp Phe Leu Thr Glu Ala
           995                 1000                1005
   Ile Val His Ser Val Phe Ile Leu Ser Tyr Tyr Thr Asn His Asp Leu
       1010                1015                1020
   Lys Asp Lys Leu Gln Asp Leu Ser Asp Asp Arg Leu Asn Lys Phe Leu
   1025                1030                1035                1040
   Thr Cys Ile Ile Thr Phe Asp Lys Asn Pro Asn Ala Glu Phe Val Thr
                   1045                1050                1055
   Leu Met Arg Asp Pro Gln Ala Leu Gly Ser Glu Arg Gln Ala Lys Ile
               1060                1065                1070
   Thr Ser Glu Ile Asn Arg Leu Ala Val Thr Glu Val Leu Ser Thr Ala
           1075                1080                1085
   Pro Asn Lys Ile Phe Ser Lys Ser Ala Gln His Tyr Thr Thr Thr Glu
       1090                1095                1100
   Ile Asp Leu Asn Asp Ile Met Gln Asn Ile Glu Pro Thr Tyr Pro His
   1105                1110                1115                1120
   Gly Leu Arg Val Val Tyr Glu Ser Leu Pro Phe Tyr Lys Ala Glu Lys
                   1125                1130                1135
   Ile Val Asn Leu Ile Ser Gly Thr Lys Ser Ile Thr Asn Ile Leu Glu
               1140                1145                1150
   Lys Thr Ser Ala Ile Asp Leu Thr Asp Ile Asp Arg Ala Thr Glu Met
           1155                1160                1165
   Met Arg Lys Asn Ile Thr Leu Leu Ile Arg Ile Leu Pro Leu Asp Cys
       1170                1175                1180
   Asn Arg Asp Lys Arg Glu Ile Leu Ser Met Glu Asn Leu Ser Ile Thr
   1185                1190                1195                1200
   Glu Leu Ser Lys Tyr Val Arg Glu Arg Ser Trp Ser Leu Ser Asn Ile
                   1205                1210                1215
   Val Gly Val Thr Ser Pro Ser Ile Met Tyr Thr Met Asp Ile Lys Tyr
               1220                1225                1230
   Thr Thr Ser Thr Ile Ser Ser Gly Ile Ile Ile Glu Lys Tyr Asn Val
           1235                1240                1245
   Asn Ser Leu Thr Arg Gly Glu Arg Gly Pro Thr Lys Pro Trp Val Gly
       1250                1255                1260
   Ser Ser Thr Gln Glu Lys Lys Thr Met Pro Val Tyr Asn Arg Gln Val
   1265                1270                1275                1280
   Leu Thr Lys Lys Gln Arg Asp Gln Ile Asp Leu Leu Ala Lys Leu Asp
                   1285                1290                1295
   Trp Val Tyr Ala Ser Ile Asp Asn Lys Asp Glu Phe Met Glu Glu Leu
               1300                1305                1310
   Ser Ile Gly Thr Leu Gly Leu Thr Tyr Glu Lys Ala Lys Lys Leu Phe
           1315                1320                1325
   Pro Gln Tyr Leu Ser Val Asn Tyr Leu His Arg Leu Thr Val Ser Ser
       1330                1335                1340
   Arg Pro Cys Glu Phe Pro Ala Ser Ile Pro Ala Tyr Arg Thr Thr Asn
   1345                1350                1355                1360
   Tyr His Phe Asp Thr Ser Pro Ile Asn Arg Ile Leu Thr Glu Lys Tyr
                   1365                1370                1375
   Gly Asp Glu Asp Ile Asp Ile Val Phe Gln Asn Cys Ile Ser Phe Gly
               1380                1385                1390
   Leu Ser Leu Met Ser Val Val Glu Gln Phe Thr Asn Val Cys Pro Asn
           1395                1400                1405
   Arg Ile Ile Leu Ile Pro Lys Leu Asn Glu Ile His Leu Met Lys Pro
       1410                1415                1420
   Pro Ile Phe Thr Gly Asp Val Asp Ile His Lys Leu Lys Gln Val Ile
   1425                1430                1435                1440
   Gln Lys Gln His Met Phe Leu Pro Asp Lys Ile Ser Leu Thr Gln Tyr
                   1445                1450                1455
   Val Glu Leu Phe Leu Ser Asn Lys Thr Leu Lys Ser Gly Ser His Val
               1460                1465                1470
   Asn Ser Asn Leu Ile Leu Ala His Lys Ile Ser Asp Tyr Phe His Asn
           1475                1480                1485
   Thr Tyr Ile Leu Ser Thr Asn Leu Ala Gly His Trp Ile Leu Ile Ile
       1490                1495                1500
   Gln Leu Met Lys Asp Ser Lys Gly Ile Phe Glu Lys Asp Trp Gly Glu
   1505                1510                1515                1520
   Gly Tyr Ile Thr Asp His Met Phe Ile Asn Leu Lys Val Phe Phe Asn
                   1525                1530                1535
   Ala Tyr Lys Thr Tyr Leu Leu Cys Phe His Lys Gly Tyr Gly Lys Ala
               1540                1545                1550
   Lys Leu Glu Cys Asp Met Asn Thr Ser Asp Leu Leu Cys Val Leu Glu
           1555                1560                1565
   Leu Ile Asp Ser Ser Tyr Trp Lys Ser Met Ser Lys Val Phe Leu Glu
       1570                1575                1580
   Gln Lys Val Ile Lys Tyr Ile Leu Ser Gln Asp Ala Ser Leu His Arg
   1585                1590                1595                1600
   Val Lys Gly Cys His Ser Phe Lys Leu Trp Phe Leu Lys Arg Leu Asn
                   1605                1610                1615
   Val Ala Glu Phe Thr Val Cys Pro Trp Val Val Asn Ile Asp Tyr His
               1620                1625                1630
   Pro Thr His Met Lys Ala Ile Leu Thr Tyr Ile Asp Leu Val Arg Met
           1635                1640                1645
   Gly Leu Ile Asn Ile Asp Arg Ile His Ile Lys Asn Lys His Lys Phe
       1650                1655                1660
   Asn Asp Glu Phe Tyr Thr Ser Asn Leu Phe Tyr Ile Asn Tyr Asn Phe
   1665                1670                1675                1680
   Ser Asp Asn Thr His Leu Leu Thr Lys His Ile Arg Ile Ala Asn Ser
                   1685                1690                1695
   Glu Leu Glu Asn Asn Tyr Asn Lys Leu Tyr His Pro Thr Pro Glu Thr
               1700                1705                1710
   Leu Glu Asn Ile Leu Ala Asn Pro Ile Lys Ser Asn Asp Lys Lys Thr
           1715                1720                1725
   Leu Asn Asp Tyr Cys Ile Gly Lys Asn Val Asp Ser Ile Met Leu Pro
       1730                1735                1740
   Leu Leu Ser Asn Lys Lys Leu Ile Lys Ser Ser Ala Met Ile Arg Thr
   1745                1750                1755                1760
   Asn Tyr Ser Lys Gln Asp Leu Tyr Asn Leu Phe Pro Met Val Val Ile
                   1765                1770                1775
   Asp Arg Ile Ile Asp His Ser Gly Asn Thr Ala Lys Ser Asn Gln Leu
               1780                1785                1790
   Tyr Thr Thr Thr Ser His Gln Ile Ser Leu Val His Asn Ser Thr Ser
           1795                1800                1805
   Leu Tyr Cys Met Leu Pro Trp His His Ile Asn Arg Phe Asn Phe Val
       1810                1815                1820
   Phe Ser Ser Thr Gly Cys Lys Ile Ser Ile Glu Tyr Ile Leu Lys Asp
   1825                1830                1835                1840
   Leu Lys Ile Lys Asp Pro Asn Cys Ile Ala Phe Ile Gly Glu Gly Ala
                   1845                1850                1855
   Gly Asn Leu Leu Leu Arg Thr Val Val Glu Leu His Pro Asp Ile Arg
               1860                1865                1870
   Tyr Ile Tyr Arg Ser Leu Lys Asp Cys Asn Asp His Ser Leu Pro Ile
           1875                1880                1885
   Glu Phe Leu Arg Leu Tyr Asn Gly His Ile Asn Ile Asp Tyr Gly Glu
       1890                1895                1900
   Asn Leu Thr Ile Pro Ala Thr Asp Ala Thr Asn Asn Ile His Trp Ser
   1905                1910                1915                1920
   Tyr Leu His Ile Lys Phe Ala Glu Pro Ile Ser Leu Phe Val Cys Asp
                   1925                1930                1935
   Ala Glu Leu Ser Val Thr Val Asn Trp Ser Lys Ile Ile Ile Glu Trp
               1940                1945                1950
   Ser Lys His Val Arg Lys Cys Lys Tyr Cys Ser Ser Val Asn Lys Cys
           1955                1960                1965
   Met Leu Ile Val Lys Tyr His Ala Gln Asp Asp Ile Asp Phe Lys Leu
       1970                1975                1980
   Asp Asn Ile Thr Ile Leu Lys Thr Tyr Val Cys Leu Gly Ser Lys Leu
   1985                1990                1995                2000
   Lys Gly Ser Glu Val Tyr Leu Val Leu Thr Ile Gly Pro Ala Asn Ile
                   2005                2010                2015
   Phe Pro Val Phe Asn Val Val Gln Asn Ala Lys Leu Ile Leu Ser Arg
               2020                2025                2030
   Thr Lys Asn Phe Ile Met Pro Lys Lys Ala Asp Lys Glu Ser Ile Asp
           2035                2040                2045
   Ala Asn Ile Lys Ser Leu Ile Pro Phe Leu Cys Tyr Pro Ile Thr Lys
       2050                2055                2060
   Lys Gly Ile Asn Thr Ala Leu Ser Lys Leu Lys Ser Val Val Ser Gly
   2065                2070                2075                2080
   Asp Ile Leu Ser Tyr Ser Ile Ala Gly Arg Asn Glu Val Phe Ser Asn
                   2085                2090                2095
   Lys Leu Ile Asn His Lys His Met Asn Ile Leu Lys Trp Phe Asn His
               2100                2105                2110
   Val Leu Asn Phe Arg Ser Thr Glu Leu Asn Tyr Asn His Leu Tyr Met
           2115                2120                2125
   Val Glu Ser Thr Tyr Pro Tyr Leu Ser Glu Leu Leu Asn Ser Leu Thr
       2130                2135                2140
   Thr Asn Glu Leu Lys Lys Leu Ile Lys Ile Thr Gly Ser Leu Leu Tyr
   2145                2150                2155                2160
   Asn Phe His Asn Glu
                   2165
<210> 60
   <211> 47
   <212> DNA
   <213> ARTIFICIAL SEQUENCE
<220>
   <221> CDS
   <222> (5)..(43)
<220>
   <223> Description of artificial sequence: RT/PCR Product
<400> 60
   acaa atg acc atg cca aaa ata atg ata cta cct gac aaa taa gctt 47
   Met Thr Met Pro Lys Ile Met Ile Leu Pro Asp Lys
   1 5 10
<210> 61
   <211> 12
   <212> PRT
   <213> ARTIFICIAL SEQUENCE
<220>
   <223> Description of artificial sequence: RT/PCR Product
<400> 61
   Met Thr Met Pro Lys Ile Met Ile Leu Pro Asp Lys
   1 5 10
<210> 62
   <211> 11
   <212> PRT
   <213> ARTIFICIAL SEQUENCE
<220>
   <223> Description of artificial sequence: RT/PCR Product
<400> 62
   Thr Asn Asp His Ala Lys Asn Asn Asp Thr Thr
   1 5 10
<210> 63
   <211> 16
   <212> DNA
   <213> ARTIFICIAL SEQUENCE
<220>
   <223> Description of artificial sequence: RT/PCR Product
<400> 63
   aagctttaag cttcaa 16
<210> 64
   <211> 9
   <212> DNA
   <213> ARTIFICIAL SEQUENCE
<220>
   <223> Description of artificial sequence: RT/PCR Product
<400> 64
   aagcttcaa 9
<210> 65
   <211> 35
   <212> DNA
   <213> ARTIFICIAL SEQUENCE
<220>
   <223> Description of Artificial Sequence: Primer
<400> 65
   ggatccacaa taacattggg gcaaatgcaa ccatg 35
<210> 66
   <211> 19
   <212> DNA
   <213> ARTIFICIAL SEQUENCE
<220>
   <223> Description of Artificial Sequence: Primer
<400> 66
   agttacataa aacggatcc 19

## Description

### 1. INTRODUCTION

The present invention relates to recombinant negative strand virus RNA templates which may be used to express heterologous gene products in appropriate host cell systems and/or to construct recombinant viruses that express, package, and/or present the heterologous gene product. The expression products and chimeric viruses may advantageously be used in vaccine formulations. In particular, the present invention relates to methods of generating recombinant respiratory syncytial viruses and the use of these recombinant viruses as expression vectors and vaccines. The invention is described by way of examples in which recombinant respiratory syncytial viral genomes are used to generate infectious viral particles.

### 2. BACKGROUND OF THE INVENTION

A number of DNA viruses have been genetically engineered to direct the expression of heterologous proteins in host cell systems (e.g., vaccinia virus, baculovirus, etc.). Recently, similar advances have been made with positive-strand RNA viruses (e.g., poliovirus). The expression products of these constructs, i.e., the heterologous gene product or the chimeric virus which expresses the heterologous gene product, are thought to be potentially useful in vaccine formulations (either subunit or whole virus vaccines). One drawback to the use of viruses such as vaccinia for constructing recombinant or chimeric viruses for use in vaccines is the lack of variation in its major epitopes. This lack of variability in the viral strains places strict limitations on the repeated use of chimeric vaccinia, in that multiple vaccinations will generate host-resistance to the strain so that the inoculated virus cannot infect the host. Inoculation of a resistant individual with chimeric vaccinia will, therefore, not induce immune stimulation.

By contrast, negative-strand RNA viruses such as influenza virus and respiratory syncytial virus, demonstrate a wide variability of their major epitopes. Indeed, thousands of variants of influenza have been identified; each strain evolving by antigenic drift. The negative-strand viruses such as influenza and respiratory syncytial virus would be attractive candidates for constructing chimeric viruses for use in vaccines because its genetic variability allows for the construction of a vast repertoire of vaccine formulations which will stimulate immunity without risk of developing a tolerance.

### 2.1. RESPIRATORY SYNCYTIAL VIRUS

Virus families containing enveloped single-stranded RNA of the negative-sense genome are classified into groups having non-segmented genomes (Paramyxoviridae, Rhabdoviridae) or those having segmented genomes (Orthomyxoviridae, Bunyaviridae and Arenaviridae). Paramyxoviridae have been classified into three genera: paramyxovirus (sendai virus, parainfluenza viruses types 1-4, mumps, newcastle disease virus); morbillivirus (measles virus, canine distemper virus and rinderpest virus); and pneumovirus (respiratory syncytial virus and bovine respiratory syncytial virus).

Human respiratory syncytial virus (RSV) is the leading cause of severe lower respiratory tract disease in infants and young children and is responsible for considerable morbidity and mortality. Two antigenically diverse RSV subgroups A and B are present in human populations. RSV is also recognized as an important agent of disease in immuno-compromised adults and in the elderly. Due to the incomplete resistance to RSV reinfection induced by natural infection, RSV may infect multiple times during childhood and life. The goal of RSV immunoprophylaxis is to induce sufficient resistance to prevent the serious disease which may be associated with RSV infection. The current strategies for developing RSV vaccines principally revolve around the administration of purified viral antigen or the development of live attenuated RSV for intranasal administration. However, to date there have been no approved vaccines or highly effective antiviral therapy for RSV.

Infection with RSV can range from an unnoticeable infection to severe pneumonia and death. RSV possesses a single-stranded nonsegmented negative-sense RNA genome of 15,221 nucleotides (Collins, 1991, In The paramyxoviruses pp. 103-162, D.W. Kingsbury (ed.) Plenum Press, New York). The genome of RSV encodes 10 mRNAs (Collins et al., 1984, J. Virol. 49: 572-578). The genome contains a 44 nucleotide leader sequence at the 3' termini followed by the NS1-NS2-N-P-M-SH-G-F-M2-L and a 155 nucleotide trailer sequence at the 5' termini (Collins. 1991, *supra*)*.* Each gene transcription unit contains a short stretch of conserved gene start (GS) sequence and a gene end (GE) sequences.

The viral genomic RNA is not infectious as naked RNA. The RNA genome of RSV is tightly encapsidated with the major nucleocapsid (N) protein and is associated with the phosphoprotein (P) and the large (L) polymerase subunit. These proteins form the nucleoprotein core, which is recognized as the minimum unit of infectivity (Brown et al., 1967, J. Virol. 1: 368-373). The RSV N, P, and L proteins form the viral RNA dependent RNA transcriptase for transcription and replication of the RSV genome (Yu et al., 1995, J. Virol. 69:2412-2419; Grosfeld et al., 1995, J. Virol. 69:5677-86). Recent studies indicate that the M2 gene products (M2-1 and M2-2) are involved and are required for transcription (Collins et al., 1996, Proc. Natl: Acad. Sci. 93:81-5).

The M protein is expressed as a peripheral membrane protein, whereas the F and G proteins are expressed as integral membrane proteins and are involved in virus attachment and viral entry into cells. The G and F proteins are the major antigens that elicit neutralizing antibodies in vivo (as reviewed in McIntosh and Chanock, 1990 "Respiratory Syncytial Virus" 2nd ed. Virology (D. M. Knipe et al., Ed.) Raven Press, Ltd., N.Y.). Antigenic dimorphism between the subgroups of RSV A and B is mainly linked to the G glycoprotein, whereas the F glycoprotein is more closely related between the subgroups.

Despite decades of research, no safe and effective RSV vaccine has been developed for the prevention of severe morbidity and mortality associated with RSV infection. A formalin-inactivated virus vaccine has failed to provide protection against RSV infection and its exacerbated symptoms during subsequent infection by the wild-type virus in infants (Kapikian et al., 1969, Am. J. Epidemiol. 89:405-21; Chin et al., 1969, Am. J. Epidemiol. 89:449-63) Efforts since have focused on developing live attenuated temperature-sensitive mutants by chemical mutagenesis or cold passage of the wild-type RSV (Gharpure et al., 1969, J. Virol. 3: 414-21; Crowe et al., 1994, Vaccine 12: 691-9). However, earlier trials yielded discouraging results with these live attenuated temperature sensitive mutants. Virus candidates were either underattenuated or overattenuated (Kim et al., 1973, Pediatrics 52:56-63; Wright et al., 1976, J. Pediatrics 88:931-6) and some of the vaccine candidates were genetically unstable which resulted in the loss of the attenuated phenotype (Hodes et al., 1974, Proc. Soc. Exp. Biol. Med. 145:1158-64).

Attempts have also been made to engineer recombinant vaccinia vectors which express RSV F or G envelope glycoproteins. However, the use of these vectors as vaccines to protect against RSV infection in animal studies has shown inconsistent results (Olmsted et al. 1986, Proc. Natl. Acad. Sci. 83:7462-7466; Collins et al., 1990, Vaccine 8:164-168).

Thus, efforts have turned to engineering recombinant RSV to generate vaccines. For a long time, negative-sense RNA viruses were refractory to study. Only recently has it been possible to recover negative strand RNA viruses using a recombinant reverse genetics approach (U.S. Patent No. 5,166,057 to Palese et al.). Although this method was originally applied to engineer influenza viral genomes (Luytjes et al. 1989, Cell 59:1107-1113; Enami et al. 1990, Proc. Natl. Acad. Sci. USA 92: 11563-11567), it has been successfully applied to a wide variety of segmented and nonsegmented negative strand RNA viruses, including rabies (Schnell et al. 1994, EMBO J. 13: 4195-4203); VSV (Lawson et al., 1995, Proc. Natl. Acad. Sci USA 92: 4477-81); measles virus (Radecke et al., 1995, EMBO J. 14:5773-84); rinderpest virus (Baron & Barrett, 1997, J. virol. 71: 1265-71); human parainfluenza virus (Hoffman & Banerjee, 1997, J. Virol. 71:3272-7; Dubin et al., 1997, Virology 235:323=32); SV5 (He et al., 1997, Virology 237:249-60); respiratory syncytial virus (Collins et al. 1991, Proc. Natl. Acad. Sci. USA 88: 9663-9667) and Sendai virus (Park et al. 1991, Proc. Natl. Acad. Sci. USA 88:5537-5541; Kato et al. 1996, Genes to Cells 1:569-579). Although this approach has been used to successfully rescue RSV, a number of groups have reported that RSV is still refractory to study given several properties of RSV which distinguish it from the better characterized paramyxoviruses of the genera Paramyxovirus, Rubulavirus, and Morbillivirus. These differences include a greater number of RNAs, an unusual gene order at the 3' end of the genome, extensive strain-to-strain sequence diversity, several proteins not found in other nonsegmented negative strand RNA viruses and a requirement for the M2 protein (ORF1) to proceed with full processing of full length transcripts and rescue of a full length genome (Collins et al. PCT WO97/12032; Collins, P.L. et al. pp 1313-1357 of volume 1, Fields Virology, et al., Eds. (3rd ed., Raven Press, 1996).

### 3. SUMMARY OF THE INVENTION

The present invention relates to genetically engineered recombinant RS viruses and viral vectors which contain heterologous genes which for the use as vaccines. In accordance with the present invention, the recombinant RS viral vectors and viruses are engineered to contain heterologous genes, including genes of other viruses, pathogens, cellular genes, tumor antigens, or to encode combinations of genes from different strains of RSV.

The present invention in a first aspect provides a isolated infectious respiratory syncytial virus particle having an attenuated phenotype comprising a respiratory syncycial virus antigenome or genome wherein said genome or antigenome has: (a) a heterologous sequence encoding a G and F protein, from RSV strain B9320; and (b) a deletion of the M2-2 open reading frame.

Recombinant negative-strand viral RNA templates are described which may be used to transfect transformed cell that express the RNA dependent RNA polymerase and allow for complementation. Alternatively, a plasmid expressing the components of the RNA polymerase from an appropriate promoter can be used to transfect cells to allow for complementation of the negative-strand viral RNA templates. Complementation may also be achieved with the use of a helper virus or wild-type virus to provide the RNA dependent RNA polymerase. The RNA templates are prepared by transcription of appropriate DNA sequences with a DNA-directed RNA polymerase. The resulting RNA templates are of negative-or positive-polarity and contain appropriate terminal sequences which enable the viral RNA-synthesizing apparatus to recognize the template. Bicistronic mRNAs can be constructed to permit internal initiation of translation of viral sequences and allow for the expression of foreign protein coding sequences from the regular terminal initiation site, or vice versa.

As demonstrated by the examples described herein, recombinant RSV genome in the positive-sense or negative-sense orientation is co-transfected with expression vectors encoding the viral nucleocapsid (N) protein, the associated nucleocapsid phosphoprotein (P), the large (L) polymerase subunit protein, with or without the M2/ORF1 protein of RSV to generate infectious viral particles. Plasmids encoding RS virus polypeptides are used as the source of proteins which were able to replicate and transcribe synthetically derived RNPs. The minimum subset of RSV proteins needed for specific replication and expression of the viral RNP was found to be the three polymerase complex proteins (N, P and L). This suggests that the entire M2-1 gene function, supplied by a separate plasmid expresing M2-1, may not be absolutely required for the replication, expression and rescue of infectious RSV.

The expression products and/or chimeric virions obtained may advantageously be utilized in vaccine formulations. In particular, recombinant RSV genetically engineered to demonstrate an attenuated phenotype may be utilized as a live RSV vaccine. In accordance with the invention, recombinant RSV are engineered to express heterologous G and F polypeptides from RSV strain B9320 to generate a chimeric RSV to serve as a vaccine, that is able to elicit both vertebrate humoral and cell-mediated immune responses. The use of recombinant RSV for this purpose is especially attractive since these viruses demonstrate tremendous strain variability allowing for the construction of a vast repertoire of vaccine formulations. The ability to select from thousands of virus variants for constructing chimeric viruses obviates the problem of host resistance encountered when using other viruses such as vaccinia.

The present invention further relates to the attenuation of human respiratory syncytial virus by deletion of the M2-2 open reading frame.

### 3.1. DEFINITIONS

As used herein, the following terms will have the meanings indicated:
cRNA = anti-genomic RNA
HA = hemagglutinin (envelope glycoprotein)
HIV = human immunodefiency virus
L = large polymerase subunit
M = matrix protein (lines inside of envelope)
MDCK = Madin Darby canine kidney cells
MDBK = Madin Darby bovine kidney cells
moi = multiplicity of infection
N = nucleocapsid protein
NA = neuraminidase (envelope glycoprotein)
NP = nucleoprotein (associated with RNA and required for polymerase activity)
NS = nonstructural protein (function unknown)
nt = nucleotide
P = nucleocapsid phosphoprotein
PA, PB 1, PB2 = RNA-directed RNA polymerase components
RNP = ribonucleoprotein (RNA, PB2, PB1, PA and NP)
rRNP = recombinant RNP
RSV = respiratory syncytial virus
vRNA = genomic virus RNA
viral polymerase complex = PA, PB1, PB2 and NP
WSN = influenza A/WSN/33 virus
-HK virus: reassortment virus containing seven genes from WSN virus and the NA gene from influenza. A/HK/8/68 virus

### 4. DESCRIPTION OF THE FIGURES

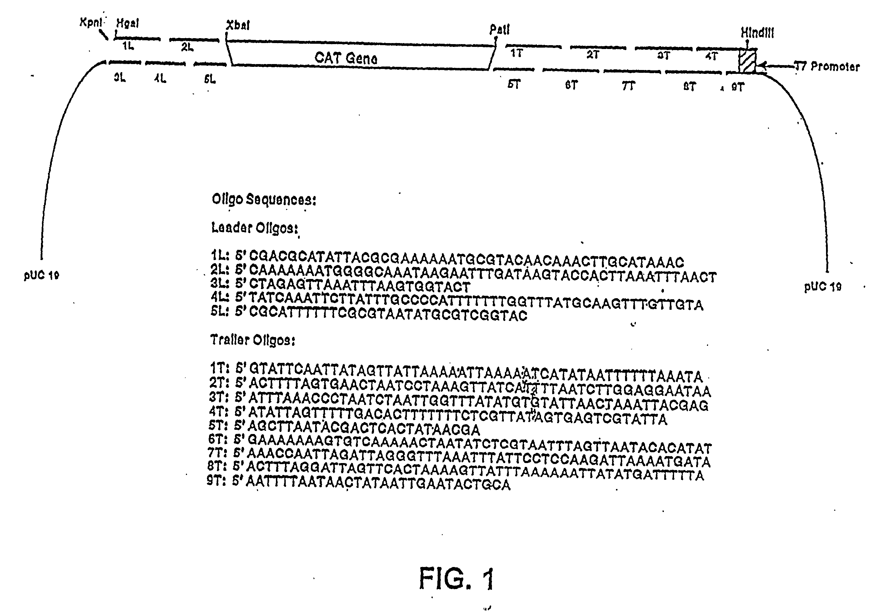

FIG. 1. Schematic representation of the RSV/CAT construct (pRSVA2CAT) used in rescue experiments. The approximate 100 nt long leader and 200 nt long trailer regions of RSV were constructed by the controlled annealing of synthetic oligonucleotides containing partial overlapping complementarity. The overlapping leader oligonucleotides are indicated by the 1 L - 5L shown in the construct. The overlapping trailer nucleotides are indicated by the 1T - 9T shown in the construct. The nucleotide sequences of the leader and trailer DNAs were ligated into purified CAT gene DNA at the indicate XbaI and PstI sites respectively. This entire construct was then ligated into KpnI/HindIII digested pUC19. The inclusion of a T7 promoter sequence and a HgaI site flanking the trailer and leader sequences, respectively, allowed in vitro synthesis of RSV/CAT RNA transcripts containing the precise genomic sequence 3' and 5' ends.

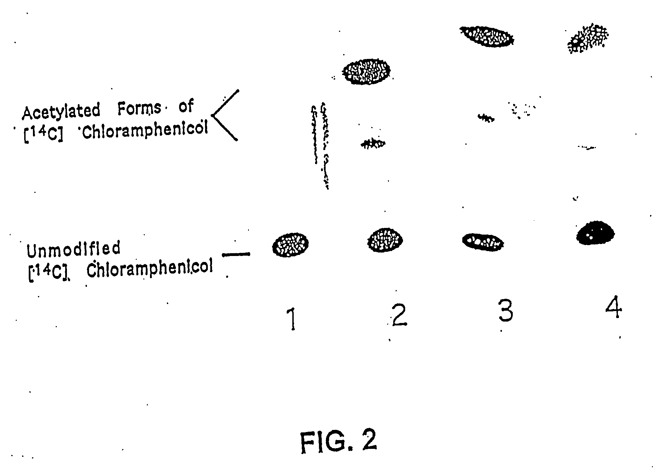

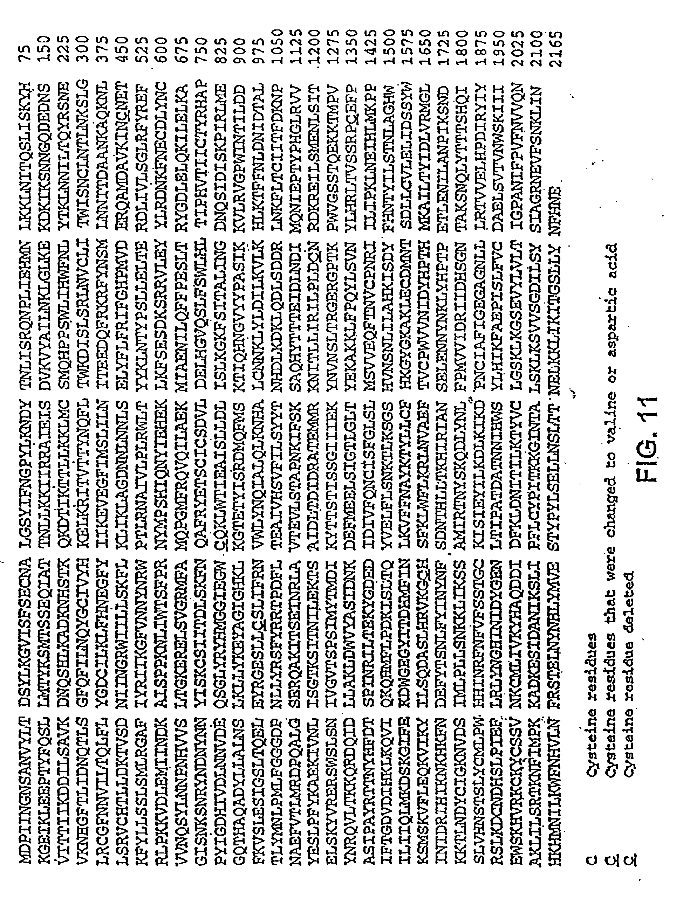

FIG. 2. Thin layer chromatogram (TLC) showing the CAT activity present in 293 cell extracts following infection and transfection with RNA transcribed from the RSV/CAT construct shown in Figure 11. Confluent monolayers of 293 cells in six-well plates (-10⁶ cells) were infected with either RSV A2 or B9320 at an m.o.i. of 0.1-1.0 pfu cell. At 1 hour post infection cells were transfected with 5-10 µg of CAT/RSV using the Transfect-Act^{™} protocol of Life Technologies. At 24 hours post infection the infected/transfected monolayers were harvested and processed for subsequence CAT assay according to Current Protocols in Molecular Biology, Vol. 1, Chapter 9.6.2; Gorman, et al., (1982) Mol. Cell. Biol. 2:1044-1051. Lanes 1, 2, 3 and 4 show the CAT activity present in (1) uninfected 293 cells, transfected with CAT/RSV-A2 infected 293 cells, co-infected with supernatant from (2) above. The CAT activity observed in each lane was produced from 1/5 of the total cellular extract from 10⁶ cells.

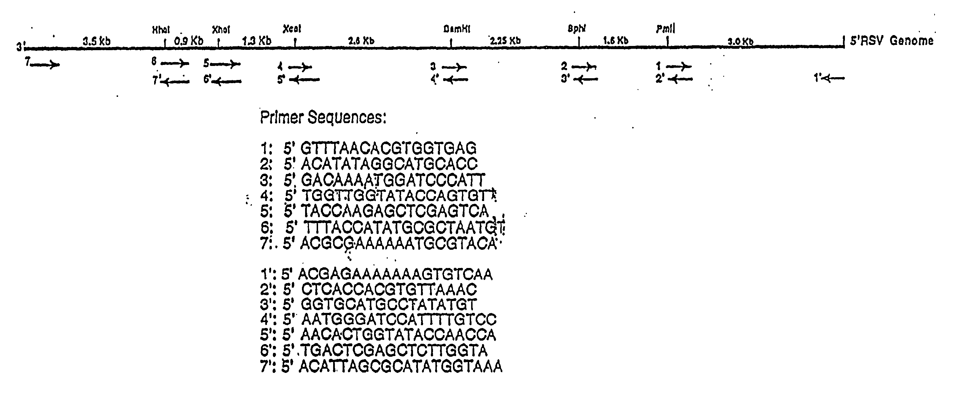

FIG. 3. Schematic representation of the RSV strain A2 genome showing the relative positions of the primer pairs used for the synthesis of cDNAs comprising the entire genome. The endonuclease sites used to splice these clones together are indicated; these sites were present in the native RSV sequence and were included in the primers used for cDNA synthesis. Approximately 100 ng of viral genomic RNA was used in RT/PCR reactions for the separate synthesis of each of the seven cDNAs. The primers for the first and second strand cDNA synthesis from the genomic RNA template are also shown. For each cDNA, the primers for the first strand synthesis are nos. 1-7 and the primers for the second strand synthesis are nos. 1'-7'.

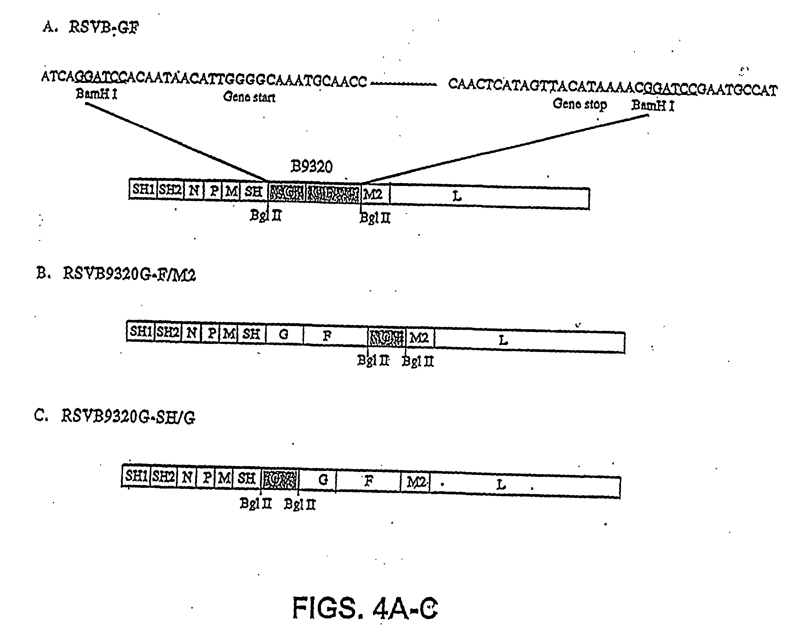

FIG. 4. Schematic representation of the RSV subgroup B strain B9320. BamH1 sites were created in the oligonucleotide primers used for RT/PCR in order to clone the G and F genes from the B9320 strain into RSV subgroup A2 antigenomic cDNA (FIG. 4A). A cDNA fragment which contained G and F genes from 4326 nucleotides to 9387 nucleotides of A2 strain was first subcloned into pUC 19 (pUCRVH). Bgl II sites were created at positions of 4630 (SH/G intergenic junction) (FIG. 4B) and 7554 (F/M2 intergenic junction (FIG. 4C). B93260 A-G and -F cDNA inserted into pUCR/H which is deleted of the A-G and F genes. The resulting antigenomic cDNA clone was termed as pRSVB-GF and was used to transfect Hep-2 cells to generate infectious RSVB-GF virus.

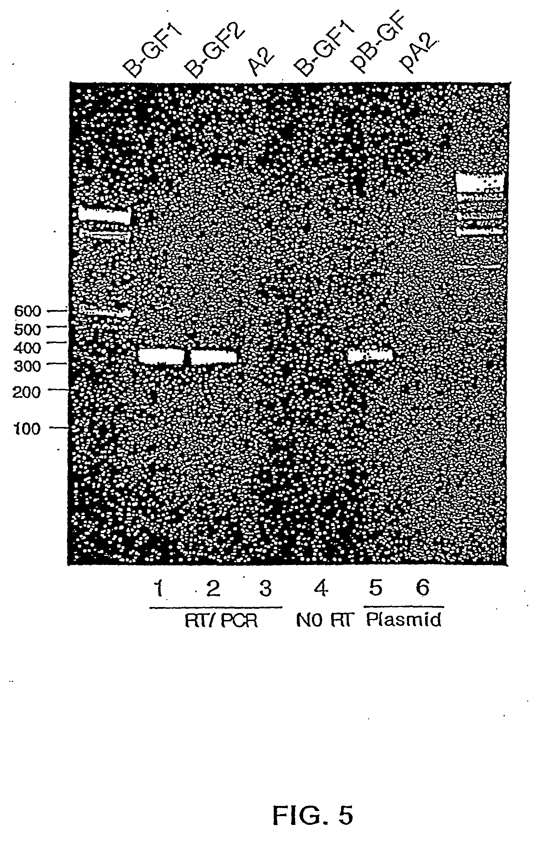

FIG. 5. Recombinant RSVB-GF virus was characterized by RT/PCR using RSV subgroup B specific primers. RSV subgroup B specific primers in the G region were incubated with aliquots of the recombinant RSV viral genomes and subjected to PCR. The PCR products were analyzed by electrophoresis on a 1% agarose gel and visualized by staining with ethidium bromide. As shown, no DNA product was produced in the RT/PCR reaction using RSV A2 as a template. However, a predicted product of 254 base pairs was seen in RT/PCR of RSVB-GF RNA and PCR control of plasmid pRSV-GF DNA as template, indicating the rescued virus contained G and F genes derived from B9320 virus.

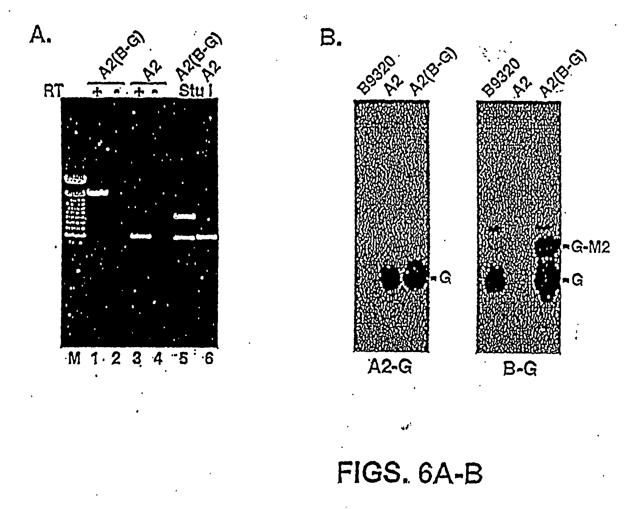

FIG. 6. Identification of chimeric rRSVA2(B-G) by RT/PCR and Northern blot analysis of RNA expression. FIG. 6A. RT/PCT analysis of chimeric rRSV A2(B-G), in comparison with wild-type A2(A2). Virion RNA extracted from rRSVA2(B-G) (lanes 1, 2) and rRSVA2 (lanes 3,4) was reverse transcribed using a primer annealed to (-) sense vRNA in the RSV F gene in the presence (+) or absence (-) of reverse transcriptase (RT), followed by PCR with a primer fair flanking the B-G insertion site. No DNA was detected in RT/PCR when reverse transcriptase (RT) was absent (lanes 2,4). A cDNA fragment, which is about 1kb bigger than the cDNA derived from A2, was produced from rRSVA(B-G). This longer PCR DNA product was digested by *Stu I* restriction enzyme unique to the inserted B-G gene (lane 5). 100 bp DNA size marker is indicated (M). FIG. 6B. Northern blot analysis of G mRNA expression. Hep-2 cells were infected with RSV B9320, rRSVA2 and chimeric rRSVA2(B-G). At 48 hr postinfection, total cellular RNA was extracted and electrophoresed on a 1.2% agarose gel containing formaldehyde. RNA was transferred to Hybond Nylon membrane and the filter was hybridized with a ³² P-labeled oligonucleotide probe specific for A2-G or specific for B9320-G mRNA. Both A2 G specific and B9320 G specific transcripts were detected in the rRSVA2 (B-G) infected cells. The run-off RNA transcript (G-M2) from rRSV A2 (B-G) infected cells is also indicated.

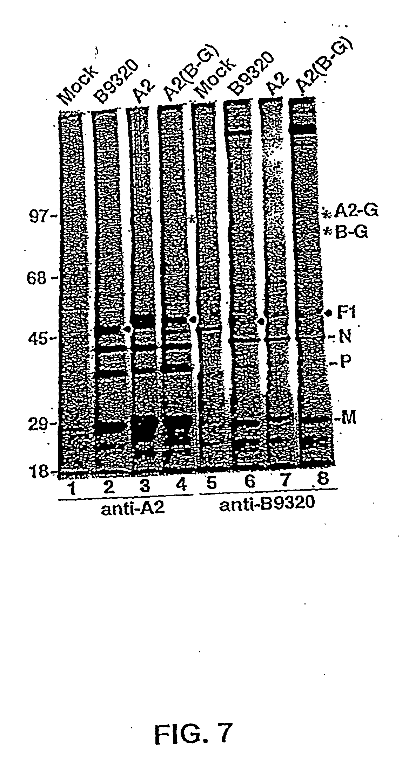

FIG. 7. Analysis of protein expression by rRSVA2 (B-G). Hep-2 cells were mock-infected (lanes 1, 5), infected with RSV B9320 (lanes 2, 6), rRSVA2 (lanes 3, 7) and rRSV A2 (B-G) (lanes 4, 8). At 14-18 hr postinfection, infected cells were labeled with ³⁵S-promix and polypeptides were immunoprecipitated by goat polyclonal antiserum against RSV A2 strain (lanes 1-5) or by mouse polyclonal antiserum against RSV B9320 strain (lanes 5-8). Immunoprecipitated polypeptides were separated on a 10% polyacrylamide gel. Both RSV A2 specific G protein and RSV B9320 specific G protein were produced in rRSV A2 (B-G) infected cells. The G protein migration is indicated by *. Mobility of the F 1 glycoprotein, and N, P, and M is indicated. Molecular sizes are shown on the left in kilodaltons.

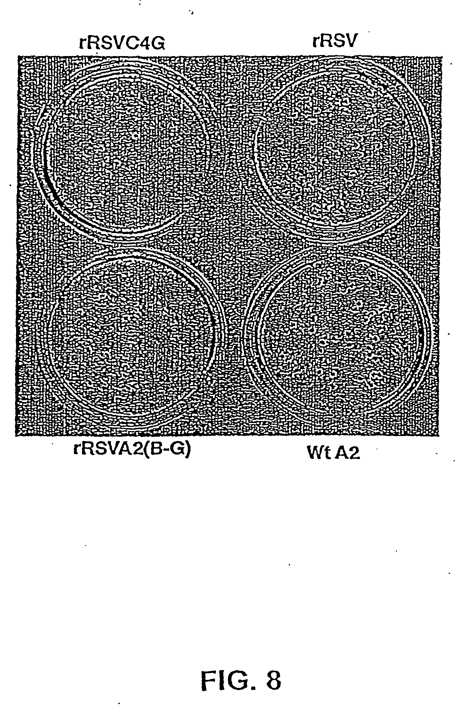

FIG. 8. Plaque morphology of rRSV, rRSVC4G, rRSVA2(B-G) and wild-type A2 virus (wt A2). Hep-2 cells were infected with each virus and incubated at 35°C for six days. The cell monolayers were fixed, visualized by immunostaining, and photographed.

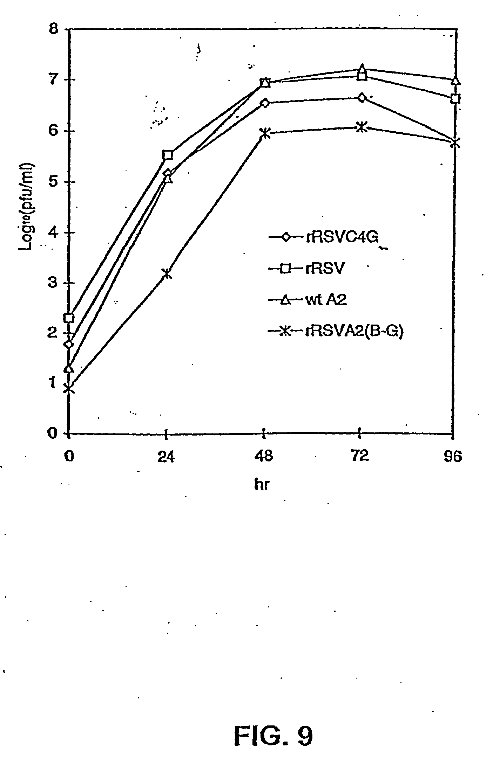

FIG. 9. Growth curve of rRSV, rRSVC4G, wild-type A2 RSV (wt A2) and chimeric rRSVA2(B-G). Hep-2 cells were infected with either virus at a moi of 0.5 and the medium was harvested at 24 hr intervals. The titer of each virus was determined in duplicate by plaque assay on Hep-2 cells and visualized by immunostaining.

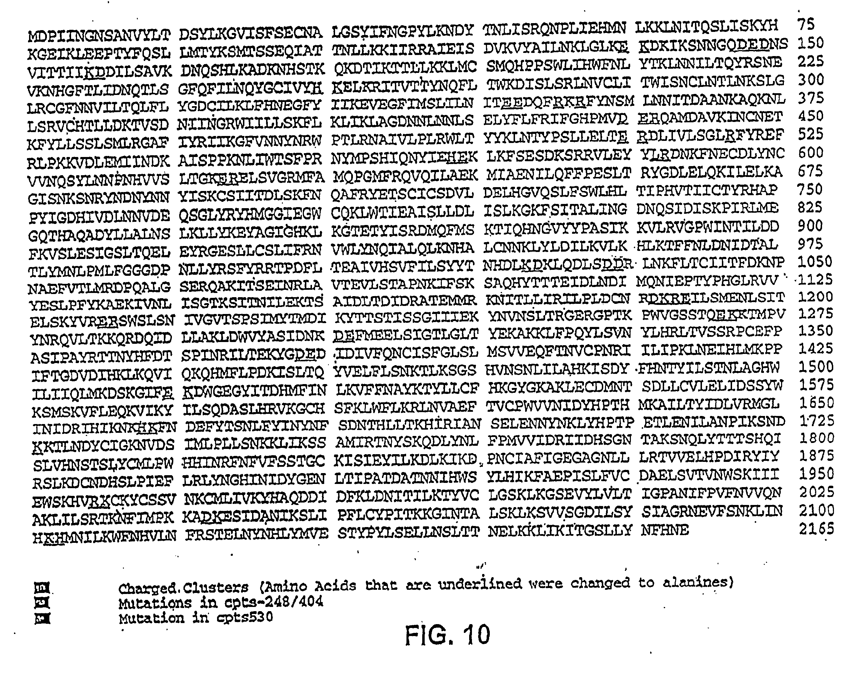

FIG. 10. RSV L protein charged residue clusters targeted for site-directed mutagenesis. Contiguous charged amino acid residues in clusters were converted to alanines by site-directed mutagenesis of the RSV L gene using the QuikChange site-directed mutagenesis kit (Stratagene).
FIG. 11. RSV L protein cysteine residues targeted for site-directed mutagenesis. Cysteine residues were converted to alanine-residues by site-directed mutagenesis of the RSV L gene using the QuikChange site-directed mutagenesis kit (Stratagene).

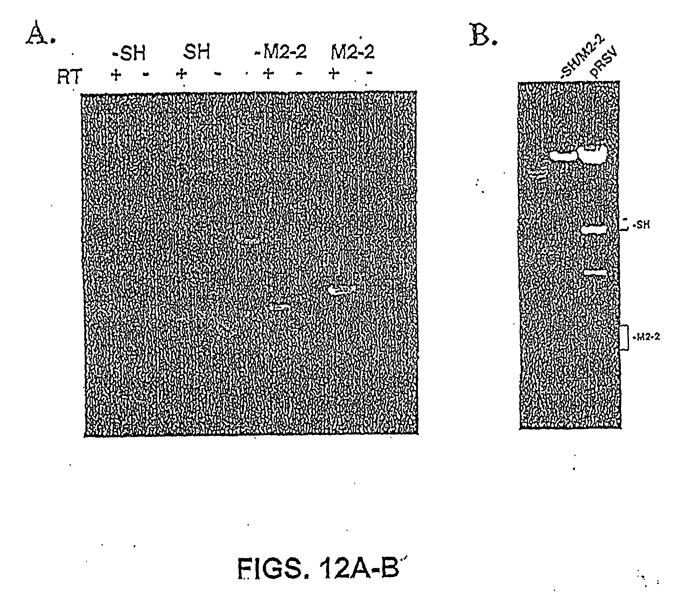

FIG. 12. Identification RSV M2-2 and SH deletion mutants. Deletions in M2-2 were generated by Hind III digestion of pET(S/B) followed by recloning of a remaining Sac I to BamHI fragment into a full-length clone. Deletions in SH were generated by Sac I digestion of pET(A/S) followed by recloning of a remaining Avr II Sac I fragment into a full-length clone. FIG. 12A. Identification of the recovered rRSVΔSH and rRSVΔM2-2 was performed by RT/PCR using primer pairs specific for the SH gene or M2-2 gene, respectively. Fig. 12B rRSVΔSHΔM2-2 was also detected by RT/PCR using primer pairs specific for the M2-2 and SH genes. RT/PCR products were run on an ethidium bromide agarose gel and bands were visualized by ultraviolet (UV) light.

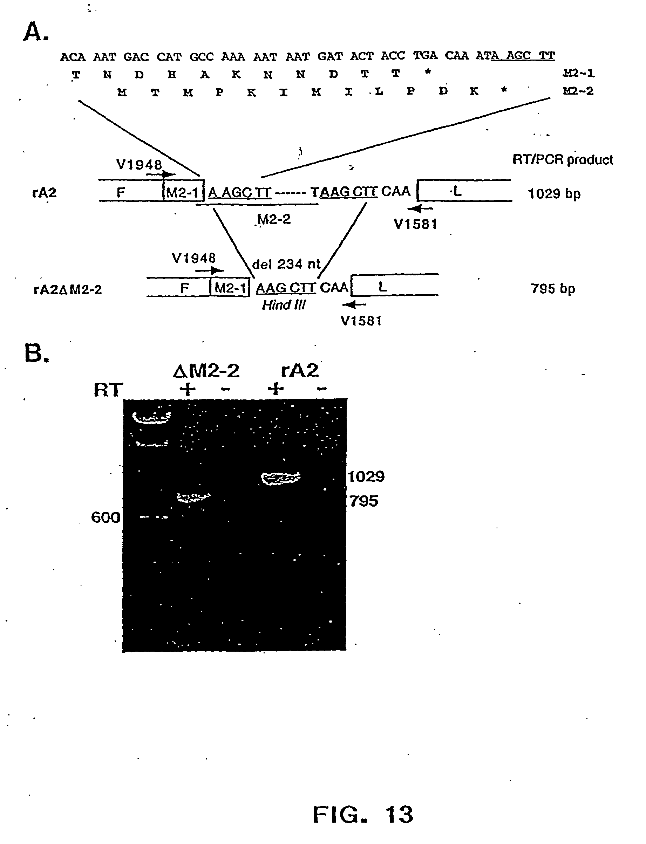

FIG. 13. Structure of rA2ΔM2-2 genome and recovery of rA2ΔM2-2. (A). Sequences shown is the region of the M2 gene that M2-1 and M2-2 open reading frames overlap. Total of 234 nt that encode the C-terminal 78 amino acids of M2-2 was deleted through the introduced *Hind III* sites (underlined). The N-terminal 12 amino acid residues of the M2-2 open reading frame are maintained as it overlaps with the M2-1 gene. (B). RT/PCR products of rA2ΔM2-2 and rA2 viral RNA using primers V1948 and V1581 in the presence (+) or absence (-) of reverse transcriptase (RT). The size of the DNA product derived from rA2 or rA2ΔM2-2 is indicated.

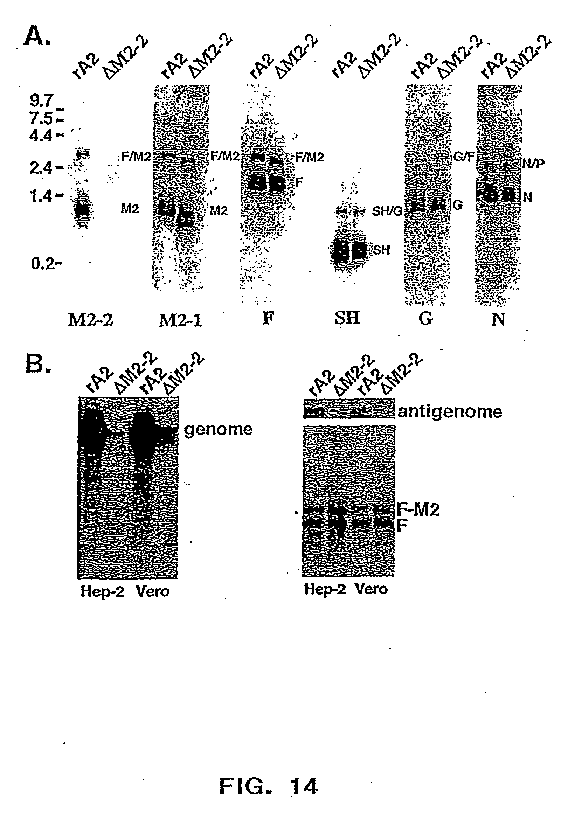

FIG. 14. Viral RNA expression by rA2ΔM2-2 and rA2. (A). Total RNA was extracted from rA2 or rA2ΔM2-2 infected Vero cells at 48 hr postinfection, separated by electrophoresis on 1.2% agarose/2.2 M formaldehyde gels and transferred to nylon membranes. Each blot was hybridized with a Dig-labeled riboprobe specific for the M2-2, M2-1, F, SH, G or N gene. The size of the RNA marker is indicated on the left. (B). Hep-2 and Vero cells were infected with rA2 or rA2ΔM2-2 for 24 hr and total cellular RNA was extracted. RNA Northern blot was hybridized with a ³² P-labeled riboprobe specific to the negative sense F gene to detect viral genomic RNA or a ³²P-labeled riboprobe specific to the positive sense F gene to detect viral antigenomic RNA and F mRNA. The top panel of the Northern blot on the right was taken from the top portion of the gel shown in the lower panel and was exposed for 1 week to show antigenome. The lower panel of the Northern blot was exposed for 3 hr to show the F mRNA. The genome, antigenome, F mRNA and dicistronic F-M2 RNA are indicated.

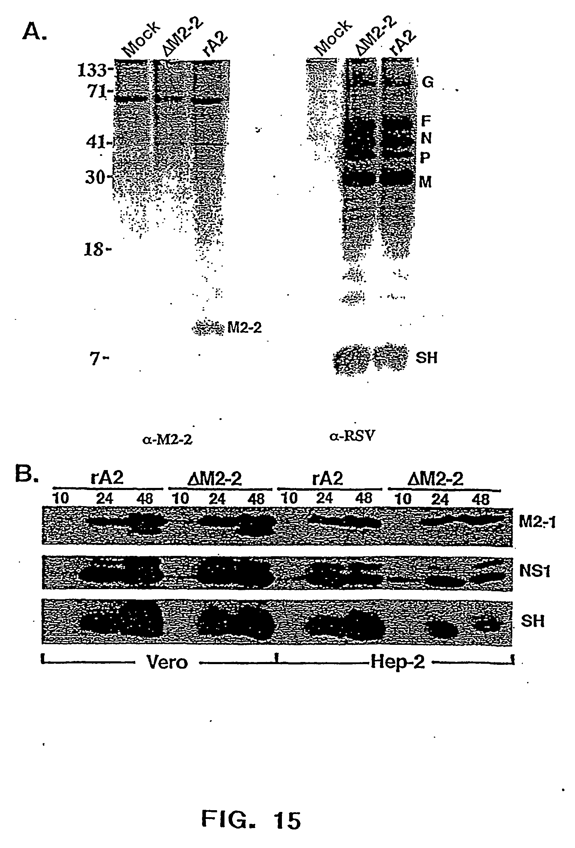

FIG. 15. Viral protein expression in rA2ΔM2-2 and rA2 infected cells. (A). Mock-infected, rA2ΔM2-2 and rA2 infected Vero cells were metabolically labeled with ³⁵ S-promix (100 µCi/ml) between 14 to 18 hr postinfection. Cell lysates were prepared for immunoprecipitation with goat polyclonal anti-RSV or rabbit polyclonal anti-M2-2 antisera. Immunoprecipitated polypeptides were separated on a 17.5% polyacrylamide gel containing 4 M urea and processed for autoradiography. The positions of each viral protein are indicated on the right and the molecular weight size markers are indicated on the left. (B). Protein synthesis kinetics in Hep-2 and Vero cells by Western blotting. Hep-2 and Vero cells were infected with rA2 or rA2ΔM2-2 and at 10 hr, 24 hr, or 48 hr postinfection, total infected cellular polypeptides were separated on a 17.5% polyacrylamide gel containing 4 M urea. Proteins were transferred to a nylon membrane and the blot probed with polyclonal antisera against M2-1, NS1 or SH as indicated.

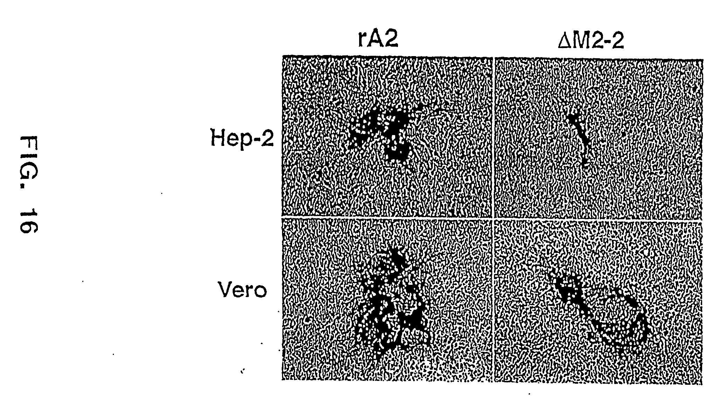

FIG. 16. Plaque morphology of rA2ΔM2-2 and rA2. Hep-2 or Vero cells were infected with rA2ΔM2-2 or rA2 under semisolid overlay composed of 1% methylcellulose and 1 X L 15 medium containing 2% FBS for 5 days. Virus plaques were visualized by immunostaining with a goat polyclonal anti-RSV antiserum and photographed under microscope.

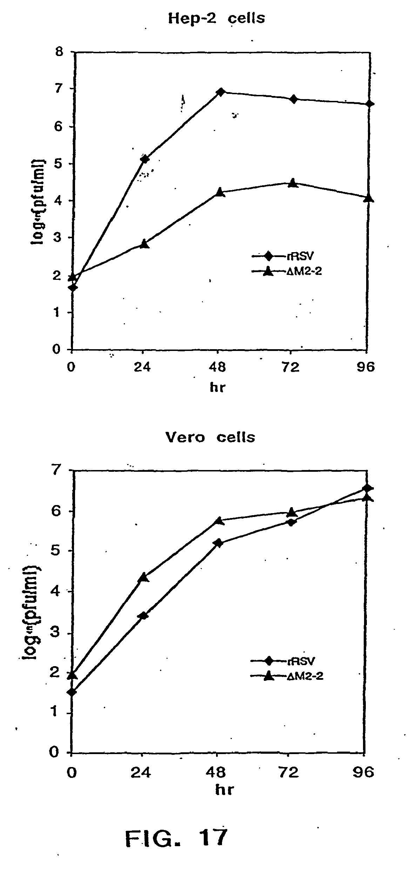

FIG. 17. Growth curves of rA2ΔM2-2 in Hep-2 and Vero cells. Vero cells (A) or Hep-2 cells (B) were infected with rA2ΔM2-2 or rA2 at m.o.i. of 0.5, and aliquots of medium were harvested at 24 hr intervals as indicated. The virus titers were determined by plaque assay in Vero cells. Virus titer at each time point is average of two experiments.

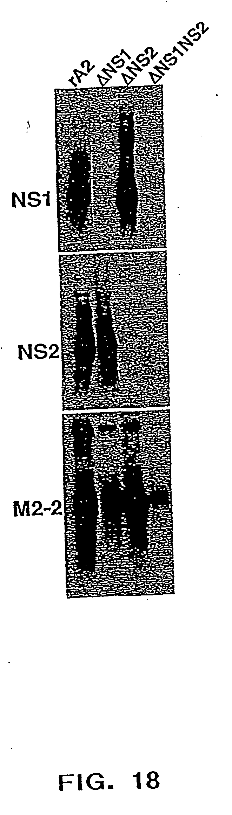

FIG. 18. Northern blot analysis of rA2ΔNS1, rA2ΔNS2 and rA2ΔNS1ΔNS2. Total cellular RNA was extracted from rA2, rA2ΔNS1, rA2ΔNS2 and rA2ΔNS1ΔNS2 infected Vero cells at 24 hr postinfection, separated by electrophoresis on 1.2% agarose/2.2 M formaldehyde gels and transferred to nylon membranes. Each blot was hybridized with a Dig-labeled riboprobe specific for the NS1, NS2, or M2-2 gene as indicated.

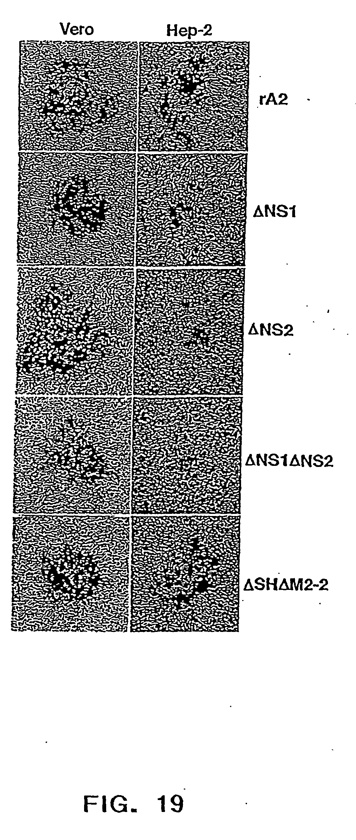

FIG. 19. Plaque morphology of deletion mutants. Hep-2 or Vero cells were infected with each deletion mutant as indicated under semisolid overlay composed of 1 % methylcellulose and 1 X L15 medium containing 2% FBS for 6 days. Virus plaques were visualized by immunostaining with a goat polyclonal anti-RSV antiserum and photographed under microscope.

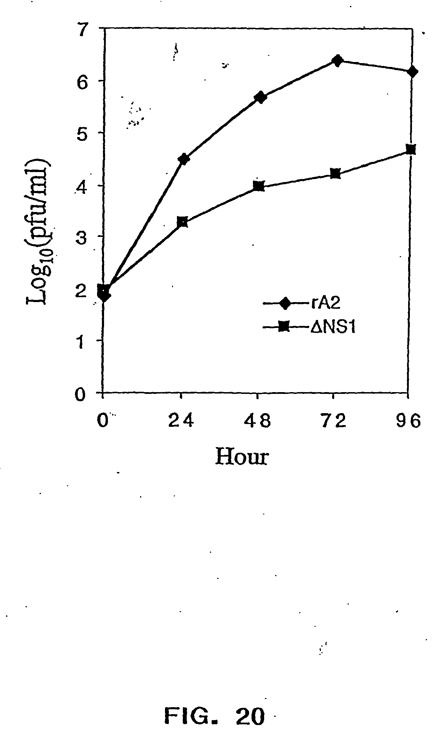

FIG. 20. Growth curves of rA2ΔNS1 in Vero cells. Vero cells were infected with rA2 ΔNS1 or rA2 at m.o.i. of 0.5, and aliquots of medium were harvested at 24 hr intervals as indicated. The virus titers were determined by plaque assay in Vero cells.

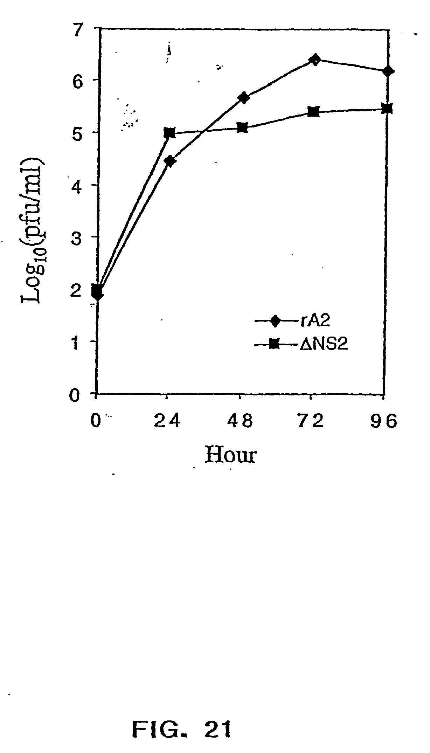

F1G. 21. Growth curves of rA2ΔNS2 in Vero cells. Vero cells were infected with rA2 ΔNS2 or rA2 at m.o.i. of 0.5, and aliquots of medium were harvested at 24 hr intervals as indicated. The virus titers were determined by plaque assay in Vero cells.

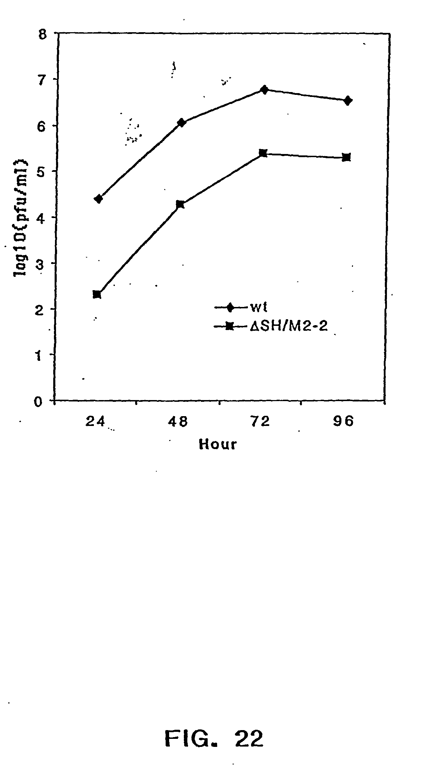

FIG. 22. Growth curves of rA2ΔSHΔM2-2 in Vero cells. Vero cells were infected with rA2ΔSHΔM2-2 or rA2 at m.o.i. of 0.5, and aliquots of medium were harvested at 24 hr intervals as indicated. The virus titers were determined by plaque assay in Vero cells.

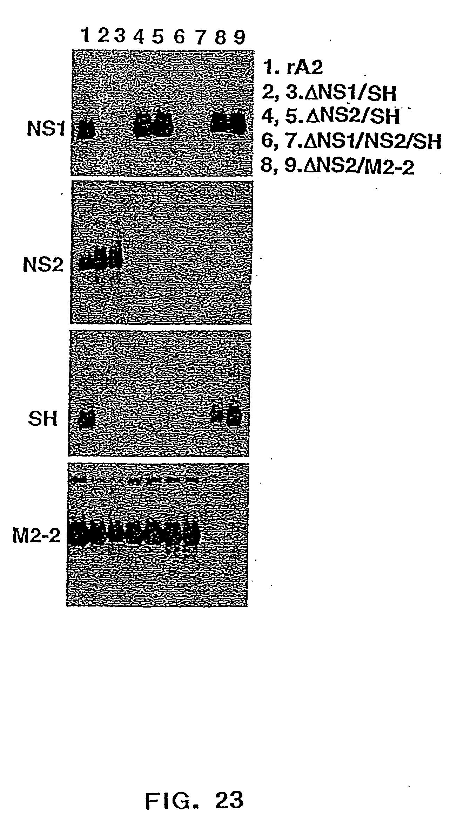

FIG. 23. Northern blot analysis of several deletion mutants. Total cellular RNA was extracted from Vero cells infected with each deletion mutant as indicated at 24 hr postinfection, separated by electrophoresis on 1.2% agarose/2.2 M formaldehyde gels and transferred to nylon membranes. Each blot was hybridized with a Dig-labeled riboprobe specific for the NS1, NS2, SH or M2-2 gene as indicated.

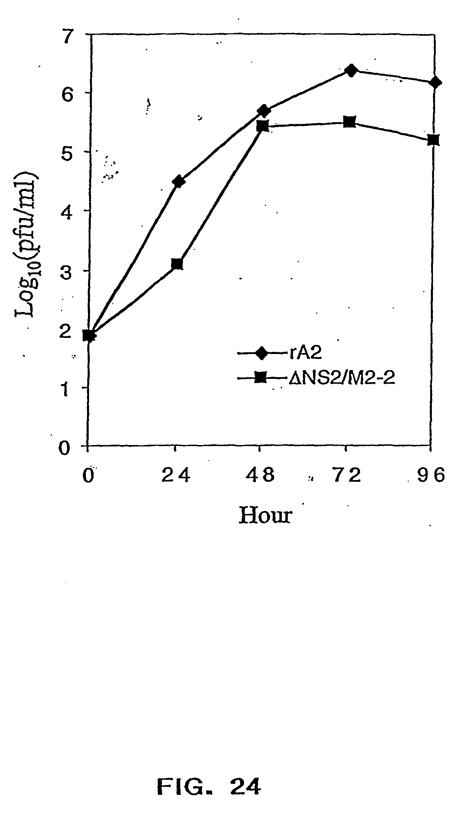

FIG. 24. Growth curves of rA2ΔNS2ΔM2-2 in Vero cells. Vero cells were infected with rA2ΔNS2ΔM2-2 or rA2 at m.o.i. of 0.5, and aliquots of medium were harvested at 24 hr intervals as indicated. The virus titers were determined by plaque assay in Vero cells.

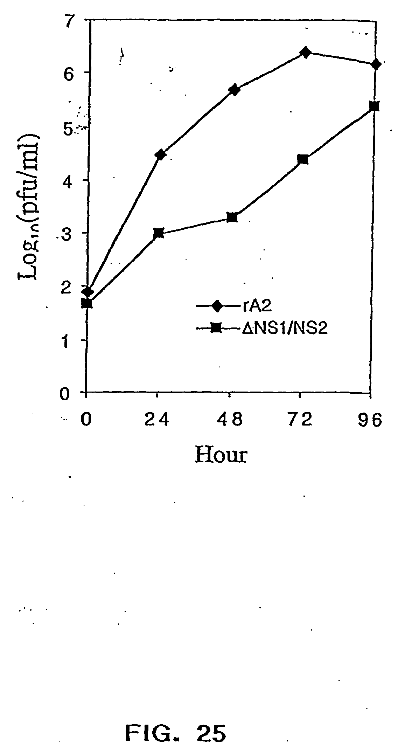

FIG. 25. Growth curves of rA2ΔNS1ΔNS2 in Vero cells. Vero cells were infected with rA2ΔNS1ΔNS2 or rA2 at m.o.i. of 0.5, and aliquots of medium were harvested at 24 hr intervals as indicated. The virus titers were determined by plaque assay in Vero cells.

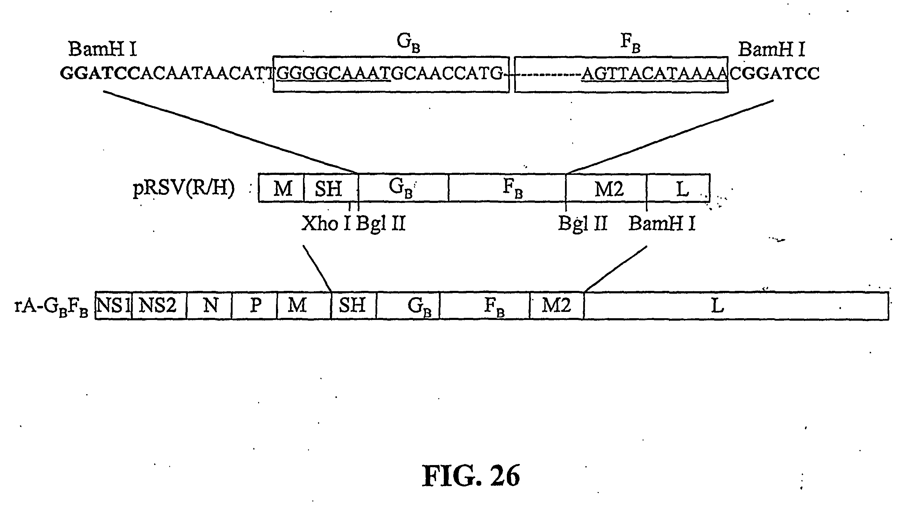

Fig.26. Insertion of the G and F genes of RSV B9320 strain into recombinant A2 strain. The G and F genes of B9320 were amplified by RT/PCR using primers that contained the BamH I restriction enzyme sites. A DNA cassette containing the G and F genes of B9320 was then introduced into the pRSV(R/H) subclone using the introduced Bgl II restriction enzyme sites that flanked the RSV G and F genes of the A2 strain. The cDNA fragment containing the G and F genes of B9320 was subsequently shuffled into the full-length A2 antigenomic cDNA by ligating at the Xho I and BamH I sites. The gene start signal of the G gene and the gene end signal of the F gene of B9320 are underlined and the restriction enzyme sites used for cloning are indicated.

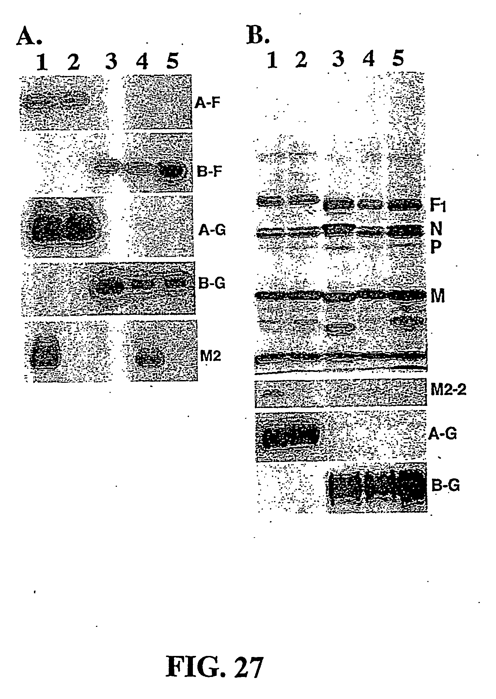

Fig. 27. Strain specific expression of the chimeric RSV rA-G_{B}F_{B} and rA-G_{B}F_{B}ΔM2-2. A. Viral RNA expression. Total cellular RNA were extracted from virus infected Vero cells and the Northern blots were hybridized with probes specific to the G or F gene of either subgroup A or subgroup B RSV. The M2-2 gene expression was examined by using a riboprobe specific to the M2-2 open reading frame. **B**. Viral protein expression. The infected Vero cells were labeled with ³⁵S-methionine and ³⁵S-cysteine and the cell lysate immunoprecipitated with anti-RSV polyclonal antibody or anti-M2-2 antibody. To detect the G protein expression, the infected cell extracts were subjected to western blotting using subgroup specific monoclonal antibody against the G protein. Both rA-G_{B}F_{B} and rA-G_{B}F_{B}ΔM2-2 expressed the subgroup B specific G and F proteins and retained normal expression of the other genes derived from the subgroup A2 backbone. No M2-2 protein was expressed in rA-G_{B}F_{B}ΔM2-2 infected cells. Lane 1: rA2, lane 2: rA2ΔM2-2, lane 3: B9320, lane 4: rA-G_{B}F_{B}, lane 5: rA-G_{B}F_{B}ΔM2-2.

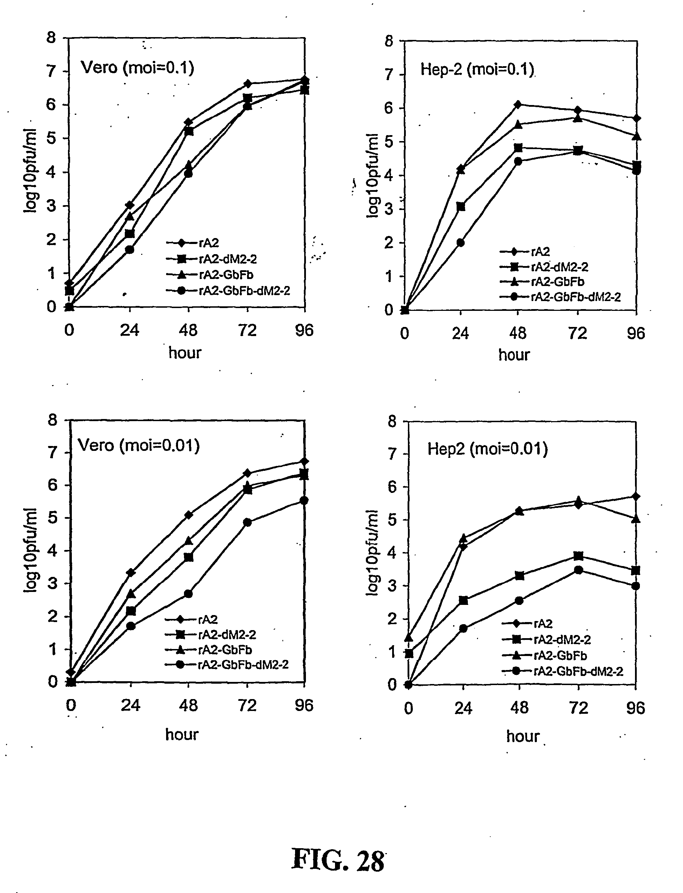

Fig. 28. Growth kinetics of the chimeric viruses in Hep-2 and Vero cells. Hep-2 or Vero cells were infected with viruses in duplicates at moi of either 0.1 or 0.01. At 24 hours intervals, the infected culture supernatants were harvested and virus titers determined by plaque assay in Vero cells.

### 5. DESCRIPTION OF THE INVENTION

The present invention relates to genetically engineered recombinant RS viruses and viral vectors which express heterologous G and F genes and comprise a deletion of the M2-2 open reading frame. The invention relates to the construction and use of recombinant negative strand RS viral RNA templates which may be used with viral RNA-directed RNA polymerase to express heterologous gene products in appropriate host cells and/or to rescue the heterologous gene in virus particles. The RNA templates of the present invention may be prepared by transcription of appropriate DNA sequences using a DNA-directed RNA polymerase such as bacteriophage T7, T3 or Sp6 polymerase. The recombinant RNA templates may be used to transfect continuous/transfected cell lines that express the RNA-directed RNA polymerase proteins allowing for complementation.

The invention is demonstrated by way of working examples in which infectious RSV is rescued from cDNA containing the RSV genome in the genomic or antigenomic sense introduced into cells expressing the N, P, and L proteins of the RSV polymerase complex. The background examples further demonstrate that expression of M2-1 expression plasmid is not required for recovery of infectious RSV from cDNA which is contrary to what has been reported earlier (Collins et al., 1995, Proc. Natl. Acad. Sci. USA 92:11563-7). Furthermore, the deletion of the M2-ORF2 from recombinant RSV cDNA results in the rescue of attenuated RSV particles. M2-2-deleted-RSV is an excellent vehicle to generate chimeric RSV encoding heterologous gene products, these chimeric viral vectors and rescued virus particles have utility as expression vectors for the expression of heterologous gene products and as live attenuated RSV vaccines expressing either RSV antigenic polypeptides or antigenic polypeptides of other viruses.

The invention is further demonstrated by way of working examples in which a cDNA clone which contained the complete genome of RSV, in addition to a T7 promoter, a hepatitis delta virus ribozyme and a T7 terminator, is used to generate an infectious viral particle when co-transfected with expression vectors encoding the N, P, L proteins of RSV. In addition, the working examples describe RNA transcripts of cloned DNA containing the coding region - in negative sense orientation - of the chloramphenicol-acetyl-transferase (CAT) gene or the green fluorescent protein (GFP) gene flanked by the 5' terminal and 3' terminal nucleotides of the RSV genome. The working examples further demonstrate that an RSV promoter mutated to have increased activity resulted in rescue of infectious RSV particles from a full length RSV cDNA with high efficiency. These results demonstrate the successful use of recombinant viral negative strand templates and RSV polymerase with increased activity to rescue RSV. This system is an excellent tool to engineer RSV viruses with defined biological properties, e.g. live-attenuated vaccines against RSV, and to use recombinant RSV as an expression vector for the expression of heterologous gene products.

This invention relates to the construction and use of recombinant negative strand viral RNA templates which may be used with viral RNA-directed RNA polymerase to express heterologous G and F gene products from RSV strain B9320 in appropriate host cells, to rescue the heterologous gene in virus particles and express mutated chimeric recombinant negative strand viral RNA templates (see U.S. Patent No. 5,166,057 to Palese *et al.,* incorporated herein by reference in its entirety). The heterologous gene product is a peptide or protein derived from RSV strain B9320. The RNA templates may be in the positive or negative-sense orientation and are prepared by transcription of appropriate DNA sequences using a DNA-directed RNA polymerase such as bacteriophage T7, T3 or the Sp6 polymerase.

The ability to reconstitute RNP's in vitro allows the design of novel chimeric influenza and RSV viruses which express foreign genes. One way to achieve this goal involves modifying existing viral genes. For example, the G or F gene may be modified to contain foreign sequences, such as the HA gene of influenza in its external domains. Where the heterologous sequence are epitopes or antigens of pathogens, these chimeric viruses may be used to induce a protective immune response against the disease agent from which these determinants are derived. For example, a chimeric RNA may be constructed in which a coding sequence derived from the gp120 coding region of human immunodeficiency virus was inserted into the coding sequence of RSV, and chimeric virus produced from transfection of this chimeric RNA segment into a host cell infected with wild-type RSV.

In addition to modifying genes coding for surface proteins, genes coding for nonsurface proteins may be altered. The latter genes have been shown to be associated with most of the important cellular immune responses in the RS virus system. Thus, the inclusion of a foreign determinant in the G or F gene of RSV may - following infection - induce an effective cellular immune response against this determinant. Such an approach may be particularly helpful in situations in which protective immunity heavily depends on the induction of cellular immune responses (e.g., malaria, etc.).

The present invention also further relates to the generation of attenuated recombinant RSV produced by introducing specific deletion of the M2-2, viral accessory gene. Additionally, the present invention specifically relates to the generation of attenuated recombinant RSV bearing a combination deletion of either the M2-2/SH viral accessory genes, the M2-2/NS2 viral accessory genes, or the NS1/NS2 viral accessory genes.

The invention is demonstrated by way of the working examples presented herein in which infectious attenuated RSV is rescued from RSV cDNA bearing deletions in the M2-2, viral accessory gene(s) either singly or in combination, such as M2-2/SH, M2-2/NS2, -deleted RSV represent excellent vehicles for the generation of live attenuated RSV vaccines.

### 5.1. CONSTRUCTION OF THE RECOMBINANT RNA TEMPLATES

Heterologous gene coding sequences flanked by the complement of the viral polymerase binding site/promoter, e.g. the complement of the 3'-RSV termini or the 3'- and 5'- RSV termini may be constructed using techniques known in the art. Heterologous gene coding sequences may also be flanked by the complement of the RSV polymerase binding site/promoter, e.g., the leader and trailer sequence of RSV using techniques known in the art. Recombinant DNA molecules containing these hybrid sequences can be cloned and transcribed by a DNA-directed RNA polymerase, such as bacteriophage T7, T3 or the Sp6 polymerase and the like, to produce the recombinant RNA templates which possess the appropriate viral sequences that allow for viral polymerase recognition and activity.

In a preferred embodiment of the present invention, the heterologous sequences are derived from the genome of another strain of RSV, e.g., the genome of RSV A strain is engineered to include the nucleotide sequences encoding the antigenic polypeptides G and F of RSV B9320 strain, or fragments thereof. In such an embodiment of the invention, heterologous coding sequences from another strain of RSV can be used to substitute for nucleotide sequences encoding antigenic polypeptides of the starting strain, or be expressed in addition to the antigenic polypeptides of the parent strain, so that a recombinant RSV genome is engineered to express the antigenic polypeptides of one, two or more strains of RSV.

One approach for constructing these hybrid molecules is to insert the heterologous coding sequence into a DNA complement of a RSV genomic RNA so that the heterologous sequence is flanked by the viral sequences required for viral polymerase activity; i.e., the viral polymerase binding site/promoter, hereinafter referred to as the viral polymerase binding site. In an alternative approach, oligonucleotides encoding the viral polymerase binding site, e.g., the complement of the 3'-terminus or both termini of the virus genomic segments can be ligated to the heterologous coding sequence to construct the hybrid molecule. The placement of a foreign gene or segment of a foreign gene within a target sequence was formerly dictated by the presence of appropriate restriction enzyme sites within the target sequence. However, recent advances in molecular biology have lessened this problem greatly. Restriction enzyme sites can readily be placed anywhere within a target sequence through the use of site-directed mutagenesis (e.g., see, for example, the techniques described by Kunkel, 1985, Proc. Natl. Acad. Sci. U.S.A. 82;488). Variations in polymerase chain reaction (PCR) technology, described infra, also allow for the specific insertion of sequences (i.e.. restriction enzyme sites) and allow for the facile construction of hybrid molecules. Alternatively, PCR reactions could be used to prepare recombinant templates without the need of cloning. For example, PCR reactions could be used to prepare double-stranded DNA molecules containing a DNA-directed RNA polymerase promoter (e.g., bacteriophage T3, T7 or Sp6) and the hybrid sequence containing the heterologous gene and the influenza viral polymerase binding site. RNA templates could then be transcribed directly from this recombinant DNA. In yet another embodiment, the recombinant RNA templates may be prepared by ligating RNAs specifying the negative polarity of the heterologous gene and the viral polymerase binding site using an RNA ligase. Sequence requirements for viral polymerase activity and constructs which may be used in accordance with the invention are described in the subsections below.

### 5.1.1. INSERTION OF THE HETEROLOGOUS GENES

The gene coding for the L protein contains a single open reading frame. The genes coding for M2 contain two open reading frames for ORF1 and 2, respectively. NS1 and NS2 are coded for by two genes, NS 1 and NS2. The G and F proteins, coded for by separate genes, are the major surface glycoproteins of the virus. Consequently, these proteins are the major targets for the humoral immune response after infection. Insertion of a foreign gene sequence into any of these coding regions could be accomplished by either an addition of the foreign sequences to be expressed or by a complete replacement of the viral coding region with the foreign gene or by a partial replacement. The heterologous sequences inserted into the RSV genome may be any length up to approximately 5 kilobases. Complete replacement would probably best be accomplished through the use of PCR-directed mutagenesis.

Alternatively, a bicistronic mRNA could be constructed to permit internal initiation of translation of viral sequences and allow for the expression of foreign protein coding sequences from the regular terminal initiation site. Alternatively, a bicistronic mRNA sequence may be constructed wherein the viral sequence is translated from the regular terminal open reading frame, while the foreign sequence is initiated from an internal site. Certain internal ribosome entry site (IRES) sequences may be utilized. The IRES sequences which are chosen should be short enough to not interfere with RS virus packaging limitations. Thus, it is preferable that the IRES chosen for such a bicistronic approach be no more than 500 nucleotides in length, with less than 250 nucleotides being preferred. Further, it is preferable that the IRES utilized not share sequence or structural homology with picornaviral elements. Preferred IRES elements include, but are not limited to the mammalian BiP IRES and the hepatitis C virus IRES.

### 5.2. EXPRESSION OF HETEROLOGOUS GENE PRODUCTS USING RECOMBINANT RNA TEMPLATE

The recombinant templates prepared as described above can be used in a variety of ways to express the heterologous gene products in appropriate host cells or to create chimeric viruses that express the heterologous gene products. In one embodiment, the recombinant template can be combined with viral polymerase complex purified infra, to produce rRNPs which are infectious. To this end, the recombinant template can be transcribed in the presence of the viral polymerase complex. Alternatively, the recombinant template may be mixed with or transcribed in the presence of viral polymerase complex prepared using recombinant DNA methods (e.g. see Kingsbury et al., 1987, Virology 156:396-403). In yet another embodiment, the recombinant template can be used to transfect appropriate host cells to direct the expression of the heterologous gene product at high levels. Host cell systems which provide for high levels of expression include continuous cell lines that supply viral functions such as cell lines superinfected with RSV, cell lines engineered to complement RSV viral functions, etc.

### 5.3. PREPARATION OF CHIMERIC NEGATIVE STRAND RNA VIRUS

In order to prepare chimeric virus, reconstituted RNPs containing modified RSV RNAs or RNA coding for foreign proteins may be used to transfect cells which are also infected with a "parent" RSV virus. Alternatively, the reconstituted RNP preparations may be mixed with the RNPs of wild type parent virus and used for transfection directly. Following transfection, the novel viruses may be isolated and their genomes identified through hybridization analysis. In additional approaches described herein for the production of infectious chimeric virus, rRNPs may be replicated in host cell systems that express the RSV or influenza viral polymerase proteins (e.g., in virus/host cell expression systems; transformed cell lines engineered to express the polymerase proteins, etc.), so that infectious chimeric virus are rescued; in this instance, helper virus need not be utilized since this function is provided by the viral polymerase proteins expressed. In a particularly desirable approach, cells infected with rRNPs engineered for all eight influenza virus segments may result in the production of infectious chimeric virus which contain the desired genotype; thus eliminating the need for a selection system.

Theoretically, one can replace any one of the genes of RSV, or part of any one of the RSV genes, with the foreign sequence. However, a necessary part of this equation is the ability to propagate the defective virus (defective because a normal viral gene product is missing or altered). A number of possible approaches exist to circumvent this problem.

A third approach to propagating the recombinant virus may involve co-cultivation with wild-type virus. This could be done by simply taking recombinant virus and co-infecting cells with this and another wild-type virus (preferably a vaccine strain). The wild-type virus should complement for the defective virus gene product and allow growth of both the wild-type and recombinant virus. This would be an analogous situation to the propagation of defective-interfering particles of influenza virus (Nayak et al., 1983, In: Genetics of Influenza Viruses, P. Palese and D. W. Kingsbury, eds., Springer-Verlag, Vienna, pp. 255-279). In the case of defective-interfering viruses, conditions can be modified such that the majority of the propagated virus is the defective particle rather than the wild-type virus. Therefore this approach may be useful in generating high titer stocks of recombinant virus. However, these stocks would necessarily contain some wild-type virus.

Alternatively, synthetic RNPs may be replicated in cells co-infected with recombinant viruses that express the RS virus polymerase proteins. In fact, this method may be used to rescue recombinant infectious virus in accordance with the invention. To this end, the RSV virus polymerase proteins may be expressed in any expression vector/host cell system, including, but not limited to, viral expression vectors (e.g., vaccinia virus, adenovirus, baculovirus, etc.) or cell lines that express the polymerase proteins (e.g., see Krystal et al., 1986, Proc. Natl. Acad. Sci. USA 83: 2709-2713).

### 5.4. GENERATION OF CHIMERIC VIRUSES WITH AN ATTENUATED PHENOTYPE BY DELETION OF M2-2

The methods of present invention may be used to introduce mutations or heterologous sequences to generate chimeric attenuated viruses which have many applications, including analysis of RSV molecular biology, pathogenesis, and growth and infection properties. In accordance with the present invention, heterologous G and F sequences may be introduced for example into the F or G protein coding sequences, NS1, NS2, M1ORF1, M2ORF2, N, P, or L coding sequences. In accordance with the present invention, the M2-2 gene, is eliminated to generate an attenuated phenotype.

In accordance with the present invention, an attenuated RSV exhibits a substantially lower degree of virulence as compared to a wild-type virus, including a slower growth rate, such that the symptoms of viral infection do not occur in an immunized individual.

### 5.5. VACCINE FORMULATIONS USING THE CHIMERIC VIRUSES

In a preferred embodiment, the present invention relates to bivalent RSV vaccines which confers protection against RSV-A and RSV-B. To formulate such a vaccine, a chimeric RS virus is used which expresses the antigenic polypeptides of both RSV-A and RSV-B subtypes.

Either a live recombinant viral vaccine or an inactivated recombinant viral vaccine can be formulated. A live vaccine may be preferred because multiplication in the host leads to a prolonged stimulus of similar kind and magnitude to that occurring in natural infections, and therefore, confers substantial, long-lasting immunity. Production of such live recombinant virus vaccine formulations may be accomplished using conventional methods involving propagation of the virus in cell culture or in the allantois of the chick embryo followed by purification.

In this regard, the use of genetically engineered RSV (vectors) for vaccine purposes may require the presence of attenuation characteristics in these strains. Current live influenza virus vaccine candidates for use in humans are either cold adapted, temperature sensitive, or passaged so that they derive several (six) genes from avian influenza viruses, which results in attenuation. The introduction of appropriate mutations (e.g., deletions) into the templates used for transfection may provide the novel viruses with attenuation characteristics. For example, specific missense mutations which are associated with temperature sensitivity or cold adaption can be made into deletion mutations. These mutations should be more stable than the point mutations associated with cold or temperature-sensitive mutants and reversion frequencies should be extremely low.

Alternatively, chimeric viruses with "suicide" characteristics may be constructed. Such viruses would go through only one or a few rounds of replication in the host. When used as a vaccine, the recombinant virus would go through a single replication cycle and induce a sufficient level of immune response but it would not go further in the human host and cause disease. Recombinant viruses lacking one or more of the essential RS virus genes would not be able to undergo successive rounds of replication. Such defective viruses can be produced by co-transfecting reconstituted RNPs lacking a specific gene(s) into cell lines which permanently express this gene(s). Viruses lacking an essential gene(s) will be replicated in these cell lines but when administered to the human host will not be able to complete a round of replication. Such preparations may transcribe and translate -- in this abortive cycle -- a sufficient number of genes to induce an immune response. Alternatively, larger quantities of the strains could be administered, so that these preparations serve as inactivated (killed) virus vaccines. For inactivated vaccines, it is preferred that the heterologous gene product be expressed as a viral component, so that the gene product is associated with the virion. The advantage of such preparations is that they contain native proteins and do not undergo inactivation by treatment with formalin or other agents used in the manufacturing of killed virus vaccines.

In another embodiment of this aspect of the invention, inactivated vaccine formulations may be prepared using conventional techniques to "kill" the chimeric viruses. Inactivated vaccines are "dead" in the sense that their infectivity has been destroyed. Ideally, the infectivity of the virus is destroyed without affecting its immunogenicity. In order to prepare inactivated vaccines, the chimeric virus may be grown in cell culture or in the allantois of the chick embryo, purified by zonal ultracentrifugation, inactivated by formaldehyde or β-propiolactone, and pooled. The resulting vaccine is usually inoculated intramuscularly.

Inactivated viruses may be formulated with a suitable adjuvant in order to enhance the immunological response. Such adjuvants may include but are not limited to mineral gels, e.g., aluminum hydroxide; surface active substances such as lysolecithin, pluronic polyols, polyanions; peptides; oil emulsions; and potentially useful human adjuvants such as BCG and Corynebacterium parvum.

Many methods may be used to introduce the vaccine formulations described above, these include but are not limited to oral, intradermal, intramuscular, intraperitoneal, intravenous, subcutaneous, and intranasal routes. It may be preferable to introduce the chimeric virus vaccine formulation via the natural route of infection of the pathogen for which the vaccine is designed. Where a live chimeric virus vaccine preparation is used, it may be preferable to introduce the formulation via the natural route of infection for influenza virus. The ability of RSV and influenza virus to induce a vigorous secretory and cellular immune response can be used advantageously. For example, infection of the respiratory tract by chimeric RSV or influenza viruses may induce a strong secretory immune response, for example in the urogenital system, with concomitant protection against a particular disease causing agent.

The following sections describe by way of example, and not by limitation, the manipulation of the negative strand RNA viral genomes using RSV as an example to demonstrate the applicability of the methods of the present invention to generate chimeric viruses for the purposes of heterologous gene expression, generating infectious viral particles and attenuated viral particles for the purposes of vaccination.

### 6. RESCUE OF INFECTIOUS RESPIRATORY SYNCYTIALVIRUSES (RSV) USING RNA DERIVED FROM SPECIFIC RECOMBINANT DNAS

This example describes a process for the rescue of infectious respiratory syncytial virus (RSV), derived from recombinant cDNAs encoding the entire RSV RNA genome into stable and infectious RSVs, as noted in Section 5 above. This process can be used in the production of chimeric RSV viruses which can express heterologous G and F genes. Another exemplary way to achieve the production of chimeric RSV involves modifying existing, native RSV genes, as is further described. Accordingly, this example also describes the utility of this process in the directed attenuation of RSV pathogenicity, resulting in production of a vaccine with defined, engineered biological properties for use in humans.

The first step of the rescue process involving the entire RSV RNA genome requires synthesis of a full length copy of the 15 kilobase (Kb) genome of RSV strain A2. This is accomplished by splicing together subgenomic double strand cDNAs (using standard procedures for genetic manipulation) ranging in size from 1 kb-3.5 kb, to form the complete genomic cDNA. Determination of the nucleotide sequence of the genomic cDNA allows identification of errors introduced during the assembly process; errors can be corrected by site directed mutagenesis, or by substitution of the error region with a piece of chemically synthesized double strand DNA. Following assembly, the genomic cDNA is positioned adjacent to a transcriptional promoter (e.g., the T7 promoter) at one end and DNA sequence which allows transcriptional termination at the other end, e.g., a specific endonuclease or a ribozyme, to allow synthesis of a plus or minus sense RNA copy of the complete virus genome *in vitro* or in cultured cells. The leader or trailer sequences may contain additional sequences as desired, such as flanking ribozyme and tandem T7 transcriptional terminators. The ribozyme can be a hepatitis delta virus ribozyme or a hammerhead ribozyme and functions to yield an exact 3' end free of non-viral nucleotides.

In accordance with this aspect of the invention, mutations, substitutions or deletions can be made to the native RSV genomic sequence which results in an increase in RSV promoter activity. Applicants have demonstrated that even an increase in RSV promoter activity greatly enhances the efficiency of rescue of RSV, allowing for the rescue of infectious RSV particles from a full-length RSV cDNA carrying the mutation. In particular, a point mutation at position 4 of the genome (C to G) results in a several fold increase in promoter activity and the rescue of infectious viral particles from a full-length RSV cDNA clone carrying the mutation.

The rescue process utilizes the interaction of full-length RSV strain A2 genome RNA, which is transcribed from the constructed cDNA, with helper RSV subgroup B virus proteins inside cultured cells. This can be accomplished in a number of ways. For example, full-length virus genomic RNA from RSV strain A2 can be transcribed *in vitro* and transfected into RSV strain B9320 infected cells, such as 293 cells using standard transfection protocols. In addition, *in vitro* transcribed genomic RNA from RSV strain A2 can be transfected into a cell line expressing the essential RSV strain A2 proteins (in the absence of helper virus) from stably integrated virus genes.

Alternatively, *in vitro* transcribed virus genome RNA (RSV strain A2) can also be transfected into cells infected with a heterologous virus (e.g., in particular vaccinia virus) expressing the essential helper RSV strain A2 proteins, specifically the N, P, L and/or M2-ORF1 proteins. In addition the *in vitro* transcribed genomic RNA may be transfected into cells infected with a heterologous virus, for example vaccinia virus, expressing T7 polymerase, which enables expression of helper proteins from transfected plasmid DNAs containing the helper N, P, and L genes.

As an alternative to transfection of *in vitro* transcribed genomic RNA, plasmid DNA containing the entire RSV cDNA construct may be transfected into cells infected with a heterologous virus, for example vaccinia virus, expressing the essential helper RSV strain A2 proteins and T7 polymerase, thereby enabling transcription of the entire RSV genomic RNA from the plasmid DNA containing the RSV cDNA construct. The vaccinia virus need not however, supply the helper proteins themselves but only the T7 polymerase; then helper proteins may be expressed from transfected plasmids containing the RSV N, P, and L genes, appropriately positioned adjacent to their own T7 promoters.

When replicating virus is providing the helper function during rescue experiments, the B9320 strain of RSV is used, allowing differentiation of progeny rescue directed against RSV B9320. Rescued RSV strain A2 is positively identified by the presence of specific nucleotide 'marker' sequences inserted in the cDNA copy of the RSV genome prior to rescue.

The establishment of a rescue system for native, i.e., 'wild-type' RSV strain A2 allows modifications to be introduced into the cDNA copy of the RSV genome to construct chimeric RSV containing sequences heterologous in some manner to that of native RSV, such that the resulting rescued virus may be attenuated in pathogenicity to provide a safe and efficacious human vaccine as discussed in Section 5.4 above.

In addition to alteration(s) (including alteration resulting from translocation) of the RSV genetic material to provide heterologous sequence, this process permits the insertion of 'foreign' genes (i.e., genes non-native to RSV) or genetic components thereof exhibiting biological function or antigenicity in such a way as to give expression of these genetic elements; in this way the modified, chimeric RSV can act as an expression system for other heterologous proteins or genetic elements, such as ribozymes, anti-sense.RNA, specific oligoribonucleotides, with prophylactic or therapeutic potential, or other viral proteins for vaccine purposes.

### 6.1. RESCUE OF THE LEADER AND TRAILER SEQUENCES OF RSV STRAIN A2 USING RSV STRAIN B9320 AS HELPER VIRUS

### 6.1.1. VIRUSES AND CELLS

Although RSV strain A2 was used in this Example, it is exemplary. It is within the skill in the art to use other strains of RSV subgroup A in accordance with the teachings of this Example. Methods which employ such other strains are encompassed by the invention.

RSV strain A2 and RSV strain B9320 were grown in Hep-2 cells and Vero cells respectively, and 293 cells were used as host during transfection/rescue experiments. All three cell lines were obtained from the ATCC (Rockville, Maryland).

### 6.1.2. CONSTRUCTION AND FUNCTIONAL ANALYSIS OF REPORTER PLASMIDS

### Plasmid pRSVA2CAT (Fig. 1) was constructed as described below.

The cDNAs of the 44 nucleotide leader and 155 nucleotide trailer components of RSV strain A2 (see Mink et al., Virology 185:615-624 (1991); Collins et al., Proc. Natl. Acad. Sci. 88:9663-9667 (1991)), the trailer component also including the promoter consensus sequence of bacteriophage T7 polymerase, were separately assembled by controlled annealing of oligonucleotides with partial overlapping complementarity (see Fig. 1). The oligonucleotides used in the annealing were synthesized on an Applied Biosystems DNA synthesizer (Foster City, CA). The separate oligonucleotides and their relative positions in the leader and trailer sequences are indicated in Fig. 1. The oligonucleotides used to construct the leader were:
1. 5'CGA CGC ATA TTA CGC GAA AAA ATG CGT ACA ACA AAC TTG CAT AAA C
2. 5'CAA AAA AAT GGG GCA AAT AAG AAT TTG ATA AGT ACC ACT TAA ATT TAA CT
3. 5'CTA GAG TTA AAT TTA AGT GGT ACT
4. 5'TAT CAA ATT CTT ATT TGC CCC ATT TTT TTG GTT TAT GCA AGT TTG TTG TA
5. 5'CGC ATT TTT TCG CGT AAT ATG CGT CGG TAC

The oligonucleotides used to construct the trailer were:
1. 5'GTA TTC AAT TAT ACT TAT TAA AAA TTA AAA ATC ATA TAA TTT TTT AAA TA
2. 5'ACT TTT ACT GAA CTA ATC CTA AAG TTA TCA TTT TAA TCT TGG AGG AAT AA
3. 5'ATT TAA ACC CTA ATC TAA TTG GTT TAT ATG TGT ATT AAC TAA ATT ACG AG
4. 5'ATA TTA GTT TTT GAC ACT TTT TTT CTC GTT ATA GTG AGT CGT ATT A
5. 5'AGC TTA ATA CGA CTC ACT ATA ACG A
6. 5'GAA AAA AAG TGT CAA AAA CTA ATA TCT CGT AAT TTA GTT AAT ACA CAT AT
7. 5'AAA CCA ATT AGA TTA GGG TTT AAA TTT ATT CCT CCA AGA TTA AAA TGA TA
8. 5'ACT TTA GGA TTA GTT CAC TAA AAG TTA TTT AAA AAA TTA TAT GAT TTT TA
9. 5'ATT TTT AAT AAC TAT AAT TGA ATA CTG CA

The complete leader and trailer cDNAs were then ligated to the chloramphenicol-acetyl-transferase (CAT) reporter gene XbaI and PstI sites respectively to form a linear - 1 kb RSV/CAT cDNA construct. This cDNA construct was then ligated into the Kpn I and Hind II sites of pUC 19. The integrity of the final pRSVA2CAT construct was checked by gel analysis for the size of the Xba I/Pst I and Kpn I/Hind II digestion products. The complete leader and trailer cDNAs were also ligated to the green fluorescent protein (GFP) gene using appropriate restriction enzyme sites to form a linear cDNA construct. The resulting RSV-GFP-CAT is a bicistronic reporter construct which expresses both CAT and GFP.

*In vitro* transcription of Hga I linearized pRSVA2CAT with bacteriophage T7 polymerase was performed according to the T7 supplier protocol (Promega Corporation, Madison, Wisconsin). Confluent 293 cells in six-well dishes (-1 x 10⁶ cells per well) were infected with RSV strain B9320 at 1 plaque forming units (p.f.u.) per cell and 1 hour later were transfected with 5-10 µg of the *in vitro* transcribed RNA from the pRSVA2CAT construct. The transfection procedure followed the transfection procedure of Collins et al., Virology 195:252-256 (1993) and employed Transect/ACT^{™} and Opti-MEM reagents according to the manufacturers specifications (Gibco-BRL, Bethesda, Maryland). At 24 hours post-infection the 293 cells were assayed for CAT activity using a standard protocol (Current Protocols in Molecular Biology, Vol. 1, Chapter 9.6.2; Gorman, et al., 1982) Mol. Cell Biol. 2: 1044-1051). The detection of high levels of CAT activity indicated that *in vitro* transcribed negative sense RNA containing the 'leader' and 'trailer' regions of the RSV A2 strain genome and the CAT gene can be encapsidated, replicated and expressed using proteins supplied by RSV strain B9320 (See Fig. 2). The level of CAT activity observed in these experiments was at least as high as that observed in similar rescue experiments where homologous RSV strain A2 was used as helper virus. The ability of an antigenically distinct subgroup B RSV strain B9320 to support the encapsidation, replication and transcription of a subgroup A RSV strain A2 RNA has to our knowledge hitherto not been formally reported.

### 6.2. CONSTRUCTION OF A cDNA REPRESENTING THE COMPLETE GENOME OF RSV

To obtain a template for cDNA synthesis, RSV genomic RNA, comprising 15,222 nucleotides, was purified from infected Hep-2 cells according to the method described by Ward et al., J. Gen. Virol. 64:167-1876 (1983). Based on the published nucleotide sequence of RSV, oligonucleotides were synthesized using an Applied Biosystems DNA synthesizer (Applied Biosystems, Foster City, CA) to act as primers for first and second strand cDNA synthesis from the genomic RNA template. The nucleotide sequences and the relative positions of the cDNA primers and key endonuclease sites within the RSV genome are indicated in Fig. 3. The production of cDNAs from virus genomic RNA was carried out according to the reverse transcription/polymerase chain reaction (RT/PCR) protocol of Perkin Elmer Corporation, Norwalk, Connecticut (see also Wang et al., (1989) Proc. Natl. Acad. Sci. 86:9717-9721); the amplified cDNAs were purified by electroelution of the appropriate DNA band from agarose gels. Purified DNA was ligated directly into the pCRII plasmid vector (Invitrogen Corp. San Diego), and transformed into either 'One Shot *E. coli* cells (Invitrogen) or 'SURE' *E. coli* cells (Stratagene, San Diego). The resulting, cloned, virus specific, cDNAs were assembled by standard cloning techniques (Sambrook et al., Molecular Cloning - A Laboratory Manual, Cold Spring Harbor laboratory Press (Cold Spring Harbor, NY, 1989) to produce a cDNA spanning the complete RSV genome. The entire cDNA genome was sequenced, and incorrect sequences were replaced by either site-directed mutagenesis or chemically synthesized DNA. Nucleotide substitutions were introduced at bases 7291 and 7294 (with base number 1 being at the start of the genomic RNA 3' end) in the 'F' gene, to produce a novel Stu I endonuclease site, and at positions 7423, 7424, and 7425 (also in the F gene) to produce a novel Pme I site. These changes were designed to act as definitive markers for rescue events. The bacteriophage T7 polymerase and the Hga I endonuclease site were placed at opposite ends of the virus genome cDNA such that either negative or positive sense virus genome RNA can be synthesized *in vitro.* The cDNAs representing the T7 polymerase promoter sequence and the recognition sequence for Hga I were synthesized on an Applied Biosystems DNA synthesizer and were separately ligated to the ends of the virus genome cDNA, or were added as an integral part of PCR primers during amplification of the terminal portion of the genome cDNA, where appropriate; the latter procedure was used when suitable endonuclease sites near the genome cDNA termini were absent, preventing direct ligation of chemically synthesized T7 promoter/Hga I site cDNA to the genome cDNA. This complete construct (genome cDNA and flanking T7 promoter/Hga I recognition sequence) was then cloned into the Kpn I/Not I sites of the Bluescript II SK phagemid (Stratagene, San Diego) from which the endogenous T7 promoter has been removed by site-directed mutagenesis. RNA transcribed from this complete genome construct may be rescued using RSV subgroup B helper virus to give infectious RSV in accordance with Example 6.1. This basic rescue system for the complete native, i.e., 'wild-type' RSV A2 strain genomic RNA can be employed to introduce a variety of modifications into the cDNA copy of the genome resulting in the introduction of heterologous sequences into the genome. Such changes can be designed to reduce viral pathogenicity without restricting virus replication to a point where rescue becomes impossible or where virus gene expression is insufficient to stimulate adequate immunity.

The following oligonucleotides were used to construct the ribozyme/T7 terminator sequence:
5'GGT*GGCCGGCATGGTCCCAGC
3'CCA CCGGCCGTACCAGGGTCG
CTCGCTGGCGCCGGCTGGGCAACA
GAGCGACCGCGGCCGACCCGTGTG
TTCCGAGGGGACCGTCCCCTCGGT
AAGGCTCCCCTGGCAGGGGAGCCA
AATGGCGAATGGGACGTCGACAGC
TTACCGCTTACCCTGCAGCTGTCG
TAACAAAGCCCGAAGGAAGCT
ATTGTTTCGGGCTTCCTTCGA
GAGTTGCTGCTGCCACCGTTG
CTCAACGACGACGGAGGCAAC
AGCAATAACTAGATAACCTTGGG
TCGTTATTGATCTATTGGAACCC
CCTCTAAACGGGTCTTGAGGGTCT
GGAGATTTGCCCAGAACTCCCAGA
TTTTGCTGAAAGGAGGAACTA
AAAACGACTTTCCTCCTTGAT
TATGCGGCCGCGTCGACGGTA
ATACGCCGGCGEAGCTGCCAT
CCGGGCCCGCCTTCGAAG3'
GGCCCGGGCGGAAGCTTC5'

A cDNA clone containing the complete genome of RSV a T7 promoter, a hepatitis delta virus ribozyme and a T7 terminator was generated. This construct can be used to generate antigenomic RNA or RSV *in vivo* in the presence of T7 polymerase. Sequence analysis indicated that the plasmid contained few mutations in RSV genome.

### 6.2.1. MODIFICATIONS OF THE RSV GENOME

For example, the F and G genes of the human subgroup B RSV Could be inserted into an otherwise RSV strain A genome (in place of, or in addition to the F and G genes of RSV strain A).

These modifications additionally comprise deletionof the M2-2 open reading frame.

### 6.3. RESCUE OF A cDNA REPRESENTING THE COMPLETE GENOME OF RSV

### 6.3.1. THE CONSTRUCTION AND FUNCTIONAL ANALYSIS OF EXPRESSION PLASMIDS

The RSV, N, P, and L genes encode the viral polymerase of RSV. The function of the RSV M genes is unknown. The ability of RSV, N, P, M, and L expression plasmids to serve the function of helper RSV strain A2 proteins was assessed as described below. The RSV, N, P, L, and M2-1 genes were cloned into the modified PCITE 2a(+) vector (Novagen, Madison, WI) under the control of the T7 promoter and flanked by a T7 terminator at it's 3' end. PCITE-2a(+) was modified by insertion of a T7 terminator sequence from PCITE-3a(+) into the Alwn I and Bgl II sites of pCITE-2a(+). The functionality of the N, P, and L expression plasmids was determined by their ability to replicate the transfected pRSVA2CAT. At approximately 80% confluency, Hep-2 cells in six-well plates were infected with MVA at a moi of 5. After 1 hour, the infected cells were transfected with pRSVA₂CAT (0.5mg), and plasmids encoding the N (0.4mg), P (0.4mg), and L (0.2mg) genes using lipofecTACE (Life Technologies, Gaithersburg, M.D.). The transfection proceeded for 5 hours or overnight and then the transfection medium was replaced with fresh MEM containing 2% (fetal bovine serum) FBS. Two days post-infection, the cells were lysed and the lysates were analyzed for CAT activity using Boehringer Mannheim's CAT ELISA kit. CAT activity was detected in cells that had been transfected with N, P, and L plasmids together with pRSVA₂CAT. However, no CAT activity was detected when any one of the expression plasmids was omitted. Furthermore, co-transfection of RSV-GFP-CAT with the N, P, and L expression plasmids resulted in expression of both GFP and CAT proteins. The ratios of different expression plasmids and moi of the recombinant vaccinia virus were optimized in the reporter gene expression system.

### 6.3.2. RECOVERY OF INFECTIOUS RSV FROM THE COMPLETE RSV cDNA

Hep-2 cells were infected with MVA (recombinant vaccinia virus expressing T7 polymerase) at an moi of one. Fifty minutes later, transfection mixture was added onto the cells. The transfection mixture consisted of 2 µg ofN expression vector, 2 µg of P expression vector, 1 µg of L expression vector, 1.25 µg of M2/ORF 1 expression vector, 2 µg of RSV genome clone with enhanced promoter, 50 µl of LipofecTACE (Life Technologies, Gaithersburg, M.D.) and 1 ml OPTI-MEM. One day later, the transfection mixture was replaced by MEM containing 2% FCS. The cells were incubated at 37°C for 2 days. The transfection supernatant was harvested and used to infect fresh Hep-2 cells in the presence of 40 µg/ml arac (drug against vaccinia virus). The infected Hep2 cells were incubated for 7 days. After harvesting the P1 supernatant, cells were used for immunostaining using antibodies directed against F protein of RSV A2 strain. Six positively stained loci with visible cell-cell-fusion (typical for RSV infection) were identified. The RNA was extracted from P1 supernatant, and used as template for RT-PCR analysis. PCR products corresponding to F and M2 regions were generated, both products contained the introduced markers. In control, PCR products derived from natural RSV virus lacked the markers.

A point mutation was created at position 4 of the leader sequence of the RSV genome clone (C residue to G) and this genome clone was designated pRSVC4GLwt. This clone has been shown in a reporter gene context to increase the promoter activity by several fold compared to wild-type. After introduction of this mutation into the full-length genome, infectious virus was rescued from the cDNA clone. The rescued recombinant RSV virus formed smaller plaques than the wild-type RSV virus (Figure 8).

This system allows the rescue mutated RSV. Therefore, it may be an excellent tool to engineer live-attenuated vaccines against RSV and to use RSV vector and viruses to achieve heterologous gene expression. It may be possible to express G protein of type B RSV into the type A background, so the vaccine is capable of protect both type A and type B RSV infection. It may also be possible to achieve attenuation and temperature sensitive mutations into the RSV genome, by changing the gene order or by site-directed mutagenesis of the L protein.

### 6.4. USE OF MONOCLONAL ANTIBODIES TO DIFFERENTIATE RESCUED VIRUS FROM HELPER VIRUS

In order to neutralize the RSV strain B9320 helper virus and facilitate identification of rescued A2 strain RSV, monoclonal antibodies against RSV strain B9320 were made as follows.

Six BALB/c female mice were infected intranasally (i.n.) with 10⁵ plaque forming units (p.f.u.) of RSV B9320, followed 5 weeks later by intraperitoneal (i.p.) inoculation with 10⁶-10⁷ pfu of RSV B9320 in a mixture containing 50% complete Freund's adjuvant. Two weeks after i.p. inoculation, a blood sample from each mouse was tested for the presence of RSV specific antibody using a standard neutralization assay (Beeler and Coelingh, J. Virol. 63:2941-2950 (1988)). Mice producing the highest level of neutralizing antibody were then further boosted with 10⁶ p.f.u. of RSV strain B9320 in phosphate buffered saline (PBS), injected intravenously at the base of the tail. Three days later, the mice were sacrificed and their spleens collected as a source of monoclonal antibody producing B-cells. Splenocytes (including B-cells) were teased from the mouse spleen through incisions made in the spleen capsule into 5 ml of Dulbecco's Modified Eagle's Medium (DME). Clumps of cells were allowed to settle out, and the remaining suspended cells were separately collected by centrifugation at 2000xg for 5 minutes at room temperature. These cell pellets were resuspended in 15 ml 0.83 (W/V) NH4Cl, and allowed to stand for 5 minutes to lyse red blood cells. Splenocytes were then collected by centrifugation as before through a 10 ml; cushion of fetal calf serum. The splenocytes were then rinsed in DME, repelleted and finally resuspended in 20 ml of fresh DME. These splenocytes were then mixed with Sp2/0 cells (a mouse myeloma cell line used as fusion partners for the immortalization of splenocytes) in a ratio of 10:1, spleen cells: Sp2/0 cells. Sp2/0 cells were obtained from the ATCC and maintained in DME supplemented with 10% fetal bovine serum. The cell mixture was then centrifuged for 8 minutes at 2000xg at room temperature. The cell pellet was resuspended in 1 ml of 50% polyethylene glycol 1000 mol. wt. (PEG 1000), followed by addition of equal volumes of DME at 1 minute intervals until a final volume of 25 ml was attained. The fused cells were then pelleted as before and resuspended at 3.5 x 10⁶ spleen cells ml¹ in growth medium (50% conditioned medium from SP2/0 cells, 50% HA medium containing 100 ml RPMI 25 ml F.C.S., 100 µgml gentamicin, 4 ml 50X Hypoxanthine, Thymidine, Aminopterin (HAT) medium supplied as a prepared mixture of Sigma Chem. Co., St. Louis, MO). The cell suspension was distributed over well plates (200 µl well⁻¹) and incubated at 37°C, 95 humidity and 5% CO₂. Colonies of hybridoma cells (fused splenocytes and Sp2/0 cells) were then subcultured into 24 well plates and grown until nearly confluent; the supernatant growth medium was then sampled for the presence of RSV strain B9320 neutralizing monoclonal antibody, using a standard neutralization assay (Beeler and Coelingh, J. Virol. 63:2941-50 (1988)). Hybridoma cells from wells with neutralizing activity were resuspended in growth medium and diluted to give a cell density of 0.5 cells per 100 µl and plated out in 96 well plates, 200 µl per well. This procedure ensured the production of monoclones (i.e. hybridoma cell lines derived from a single cell) which were then reassayed for the production of neutralizing monoclonal antibody. Those hybridoma cell lines which produced monoclonal antibody capable of neutralizing RSV strain B9320 but not RSV strain A2 were subsequently infected into mice, i.p. (10⁶ cells per mouse). Two weeks after the i.p. injection mouse ascites fluid containing neutralizing monoclonal antibody for RSV strain B9320 was tapped with a 19 gauge needle, and stored at -20°C.

This monoclonal antibody was used to neutralize the RSV strain B9320 helper virus following rescue of RSV strain A2 as described in Section 9.1. This was carried out by diluting neutralizing monoclonal antibody 1 in 50 with molten 0.4% (w/v) agar in Eagle's Minimal Essential Medium (EMEM) containing 1% F.C.S. This mixture was then added to Hep-2 cell monolayers, which had been infected with the progeny of rescue experiments at an m.o.i. of 0.1-0.01 p.f.u. per cell. The monoclonal antibody in the agar overlay inhibited the growth of RSV strain B9320, but allowed the growth of RSV strain A2, resulting in plaque formation by the A2 strain. These plaques were picked using a pasteur pipette to remove a plug a agar above the plaque and the infected cells within the plaque; the cells and agar plug were resuspended in 2 ml of EMEM, 1% FCS, and released virus was plaqued again in the presence of monoclonal antibody on a fresh Hep-2 cell monolayer to further purify from helper virus. The twice plaqued virus was then used to infect Hep-2 cells in 24 well plates, and the progeny from that were used to infect six-well plates at an m.o.i. of 0.1 p.f.u. per cell. Finally, total infected cell RNA from one well of a six-well plates was used in a RT/PCR reaction using first and second strand primers on either side of the 'marker sequences' (introduced into the RSV strain A2 genome to act as a means of recognizing rescue events) as described in Section 6.2 above. The DNA produced from the RT/PCR reaction was subsequently digested with Stu I and Pme I to positively identify the 'marker sequences' introduced into RSV strain A2 cDNA, and hence to establish the validity of the rescue process.

### 8. EXAMPLE: EXPRESSION OF RSV SUBGROUP B-G AND -F PROTEINS BY RSV A2 STRAIN

The following experiments were conducted to generate a chimeric RSV which expresses the antigenic polypeptides of more than one strain of RSV. Two main antigenic subgroups (A and B) of respiratory syncytial virus (RSV) cause human diseases. Glycoproteins F and G are the two major antigenic determinants of RSV. The F glycoproteins of subgroup A and B viruses are estimated to be 50% related, while the relationship of G glycoproteins is considerably less, about 1-5%. Infection of RSV subgroup A induces either partial or no resistance to replication of a subgroup B strain and vice versa. Both subgroup A and subgroup B RSV virus vaccines are needed to protect from RSV infection.

The first approach described herein is to make an infectious chimeric RSV cDNA clone expressing subgroup B antigens by replacing the current infectious RSV A2 cDNA clone G and F region with subgroup B-G and -F genes. The chimeric RSV would be subgroup B antigenic specific. The second approach described herein is to insert subgroup B-G gene in the current A2 cDNA clone so that one virus would express both subgroup A and B specific antigens.

### 8.1. SUBSTITUTION OF A2 G AND F BY B9320 G AND F GENES

RSV subgroup B strain B9320 G and F genes were amplified from B9320 vRNA by RT/PCR and cloned into pCRII vector for sequence determination. BamH I site was created in the oligonucleotide primers used for RT/PCR in order to clone the G and F genes from B9320 strain into A2 antigenomic cDNA (Fig. 4A). A cDNA fragment which contained G and F genes from 4326 nt to 9387 nt of A2 strain was first subcloned into pUC 19 (pUCR/H). Bgl II sites were created at positions of 4630 (SH/G intergenic junction) and 7554 (F/M2 intergenic junction), respectively by Quickchange site-directed mutagenesis kit (Stratagene, Lo Jolla, CA). B9320 G and F cDNA inserted in pCR.II vector was digested with BamH I restriction enzyme and then subcloned into Bgl II digested pUCR/H which had the A2 G and F genes removed. The cDNA clone with A2 G and F genes replaced by B9320 G and F was used to replace the Xho I to Msc I region of the full-length A2 antigenomic cDNA. The resulting antigenomic cDNA clone was termed pRSVB-GF and was used to transfect Hep-2 cells to generate infectious RSVB-GF virus.

Generation of chimeric RSVB-GF virus was as follows, pRSVB-GF was transfected, together with plasmids encoding proteins N, P, and L, into Hep-2 cells which had been infected with MVA, a recombinant vaccinia virus which expresses the T7 RNA polymerase. Hep-2 cells were split a day before transfection in six-well dishes. Monolayers of Hep-2 cells at 60% - 70% confluence were infected with MVA at moi of 5 and incubated at 35°C for 60 min. The cells were then washed once with OPTI-MEM (Life Technologies, Gaithersburg, MD). Each dish was replaced with 1 ml of OPTI-MEM and added with 0.2 ml of transfection medium. The transfection medium was prepared by mixing five plasmids in a final volume of 0.1 ml of OPTI-MEM medium, namely 0.6 µg of RSV antigenome pRSVB-GF, 0.4 µg ofN plasmid, 0.4 µg of P plasmid, and 0.2 µg of L plasmid. This was combined with 0.1 ml of OPTI-MEM containing 10 µl lipofecTACE (Life Technologies, Gaithersburg, MD U.S.A.). After a 15 minute incubation at room temperature, the DNA/lipofecTACE was added to the cells and the medium was replaced one day later by MEM containing 2% FBS. Cultures were further incubated at 35°C for 3 days and the supernatants harvested. Aliquots of culture supernatants were then used to infect fresh Hep-2 cells. After incubation for 6 days at 35°C, the supernatant was harvested and the cells were fixed and stained by an indirect horseradish peroxidase method using goat anti-RSV antibody (Biogenesis, Sandown, NH) followed by a rabbit anti-goat antibody linked to horseradish peroxidase. The virus infected cells were then detected by addition of substrate chromogen (DAKO, Carpinteria, CA, U.S.A.) according to the manufacturer's instructions. RSV-like plaques were detected in the cells which were infected with the supernatants from cells transfected with pRSVB-GF. The virus was further plaque purified twice and amplified in Hep-2 cells.

Recombinant RSVB-GF virus was characterized by RT/PCR using RSV subgroup B specific primers. Two independently purified recombinant RSVB-GF virus isolates were extracted with an RNA extraction kit (Tel-Test, Friendswood, TX) and RNA was precipitated by isopropanol. Virion RNAs were annealed with a primer spanning the RSV region from nt 4468 to 4492 and incubated for 1 hr under standard RT conditions (10 µl reactions) using superscript reverse transcriptase (Life Technologies, Gaithersburg, MD). Aliquots of each reaction were subjected to PCR (30 cycles at 94°C for 30 s, 55°C for 30 s and 72°C for 2 min) using subgroup B specific primers in G region (CACCACCTACCTTACTCAAGT and

TTTGTTTGTGGGTTTGATGGTTGG). The PCR products were analyzed by electrophoresis on 1% agarose gel and visualized by staining with ethidium bromide. As shown in Fig. 5, no DNA product was produced in RT/PCR reactions using RSV A2 strain as template. However, a predicted product of 254 bp was detected in RT/PCR reactions utilizing RSVB-GF RNA or the PCR control plasmid, pRSVB-GF DNA, as template, indicating the rescued virus contained G and F genes derived form B9320 virus.

### 8.2. EXPRESSION OF B9320G BY RSV A2 VIRUS

RSV subgroup B strain B9320 G gene was amplified from B9320 vRNA by RT/PCR and cloned into pCRII vector for sequence determination. Two Bgl II sites were incorporated into the PCR primers which also contained gene start and gene end signals (GATATCAAGATCTACAATAACATTGGGGCAAATGC and GCTAAGAGATCTTTTT GAATAACTAAGCATG). B9320G cDNA insert was digested with Bgl II and cloned into the SH/G (4630 nt) or F/M2 (7552 nt) intergenic junction of a A2 cDNA subclone (Fig. 4B and Fig. 4C). The Xho I to Msc I fragment containing B9320G insertion either at SH/G or F/M2 intergenic region was used to replace the corresponding Xho I to Msc I region of the A2 antigenomic cDNA. The resulting RSV antigenomic cDNA clone was termed as pRSVB9320G-SH/G or pRSVB9320G-F/M2.

Generation of RSV A2 virus which had B9320 G gene inserted at F/M2 intergenic region was performed similar to what has described for generation of RSVB-GF virus. Briefly, pRSVB9320G-F/M2 together with plasmids encoding proteins N, P and L were transfected, into Hep-2 cells, infected with a MVA vaccinia virus recombinant, which expresses the T7 RNA polymerase (Life Technologies, Gaithersburg, M.D.). The transfected cell medium was replaced by MEM containing 2% fetal bovine serum (FBS) one day after transfection and further incubated for 3 days at 35°C. Aliquots of culture supernatants (PO) were then used to infect fresh Hep-2 cells. After incubation for 6 days at 35°C, the supernatant was harvested and the cells were fixed and stained by an indirect horseradish peroxidase method using goat anti-RSV antibody (Biogenesis) followed by a rabbit anti-goat antibody linked to horseradish peroxidase. The virus infected cells were then detected by addition of substrate chromogen (Dako). RSV-like plaques were detected in the cells which were infected with the supernatants from cells transfected with pRSVB9320G/F/M2.

Characterization of pRSVB9320G-F/M2 virus was performed by RT/PCR using B9320G specific primers. A predicted PCR product of 410bp was seen in RT/PCR sample using pRSVB9320G-F/M2 RNA as template, indicating the rescued virus contained G gene derived from B9320. (Figure 6)

Expression of the inserted RSV B9320 G gene was analyzed by Northern blot using a ³²P-labeled oligonucleotide specific to A2-G or B-G mRNA. Total cellular RNA was extracted from Hep-2 cells infected with wild-type RSVB 9320, rRSVA2, or rRSVB9320G-F/M2 48 hours postinfection using an RNA extraction kit (RNA stat-60, Tel-Test). RNA was electrophoresed on a 1.2% agarose gel containing formaldehyde and transferred to a nylon membrane (Amersham). An oligonucleotide specific to the G gene of the A2 stain (5'TCTTGACTGTTGTGGATTGCAGGGTTGACTTGACTCCGATCGATCC-3') and an oligonucleotide specific to the B9320 G gene (5'CTTGTGTTGTTGTTGTATGGTGT GTTTCTGATTTTGTATTGATCGATCC-3') were labeled with ³²P-ATP by a kinasing reaction known to those of ordinary skill in the art. Hybridization of the membrane with one of the ³²P-labeled G gene specific oligonucletodies was performed at 65°C and washed according to standard procedure. Both A2-G and B9320-G specific RNA were detected in the rRSVB9320G-F/M2 infected Hep-2 Cells. (Figure 6B) These results demonstrate subtype specific RNA expression.

Protein expression of the chimeric rRSVA2(B-G) was compared to that of RSV B9320 and rRSV by immunoprecipitation of ³⁵S-labeled infected Hep-2 cell lysates. Briefly, the virus infected cells were labeled with ³⁵S-promix (100 µCi/ml ³⁵S-Cys and ³⁵S-Met, Amersham, Arlington Heights, IL) at 14 hours to 18 hours post-infection according to a protocol known to those of ordinary skill in the art. The cell monolayers were lysed by RIPA buffer and the polypeptides were immunoprecipitated with either polyclonal antiserum raised in goat against detergent disrupted RSV A2 virus (Fig. 7, lanes 1-4) or antiserum raised in mice against undisrupted B9320 virions (Fig. 7, lanes 5-8). The radio labeled immunoprecipitated polypeptides were electrophoresed on 10% polyacrylamide gels containing 0.1% SDS and detected by autoradiography. Anti-RSV A2 serum immunoprecipitated the major polypeptides of the RSV A2 strain, whereas anti-B9320 serum mainly reacted with RSV B9320 G protein and the conserved F protein of both A and B subgroups. As shown in Fig. 7, a protein which is identical to the A2-G protein (lane 3), was immunoprecipitated from the rRSVA2(B-G) infected cells (lane 4) by using an antiserum against RSV A2. The G protein of RSV B9320 strain was not recognized by the anti-A2 antiserum. A protein species, smaller than A2-G protein, was immunoprecipitated from both B9320 (lane 6) and rRSVA2(B-G)(lane 9) infected cells using the antiserum raised in mice against B9320 virions. This polypeptide was not present in the uninfected and RSV A2 infected cells and likely is to represent the G protein specific to the RSV B 9320 strain. Amino acid sequence comparison of both A2 and B9320 RSV G proteins indicated that two additional potential N-glycosylation sites (N-X-S/t) are present in the RSV A2G protein, which may contribute to slower migration of the A2 G protein under the conditions used. The F protein of RSV B9320 also migrated slightly faster than RSV A2 F protein. The P and M proteins also showed mobility differences between the two virus subtypes. The identity of the polypeptide near the top of the protein gel present in FSV B9320 and rRSVA2(B-G) infected cells is not known. Antisera raised in mice against the RSV B9320 virions poorly recognized the N, P and M proteins are compared to the goat antiserum raised against the RSV A2 strain. The data described above clearly indicate that chimeric rRSV

A2(B-G) expresses both the RSV A2 and B9320 specific G proteins.

### 8.2.1 REPLICATION OF RECOMBINANT RSV IN TISSUE CULTURE

Recombinant RS viruses were plaque purified three times and amplified in Hep-2 cells. Plaque assays were performed in Hep-2 cells in 12-well plates using an overlay of 1% methylcellulose and 1 x L15 medium containing 2% fetal bovine serum (FBS). After incubation at 35°C for 6 days, the monolayers were fixed with methanol and plaques were identified by immunostaining. Plaque size and morphology of rRSV was very similar to that of wild-type A2 RSV (Fig. 8). However, the plaques formed by rRSVC4G were smaller than rRSV and wild-type A2 virus. The only genetic difference between rRSV and rRSVC4 was a single nucleotide substitution in the RSV leader region. Therefore, the smaller plaque size of rRSV A2(B-G) was not distinguishable from that of rRSVC4G.

The growth curves of rRSV, rRSVC4G and rRSV A2 (B-G) were compared to that of the biologically derived wild-type A2 virus. Hep-2 cells were grown in T25 culture flasks and infected with rRSV, rRSVC4G, rRSVA2(B-G), or wild-type RSV A2 strain at a moi of 0.5. After 1 hour adsorption at 37°C, the cells were washed three times with MEM containing 2% FBS and incubated at 37°C in 5% CO₂. At 4 hour intervals post-infection, 250 µl of the culture supernatant was collected, and stored at -70°C until virus titration. Each aliquot taken was replaced with an equal amount of fresh medium. The titer of each virus was determined by plaque assay on Hep-2 cells and visualized by immunostaining (*vide supra*). As shown in Fig. 9, the growth kinetics of rRSV is very similar to that of wild-type A2 virus. Maximum virus titer for all the viruses were achieved between 48 hr to 72 hr. The virus titer of rRSVC4G was about 2.4-fold (at 48 hr) and 6.6-fold (at 72 hr) lower than rRSV and wild-type A2 RSV. The poor growth of rRSVC4G may also be due to the single nucleotide change in the leader region. The chimeric rRSV A2(B-G) showed slower kinetics and lower peak titer (Fig. 9).

### GENERATION OF HUMAN RESPIRATORY SYNCYTIAL VIRUS VACCINE (RSV) CANDIDATE BY DELETING THE VIRAL SH AND M2ORF2 GENE

### 10.1. M2-2 DELETION MUTANT

To delete M2-2 genes, two Hind III restriction enzyme sites were introduced at RSV nucleotides 8196 and 8430, respectively, in a cDNA subclone pET(S/B) which contained an RSV restriction fragment from 4478 to 8505. The RSV restriction fragment had been previously prepared by Quikchange site-directed mutagenesis (Strategene, Lo Jolla, CA). Digestion of pET(S/B) with Hind III restriction enzyme removed a 234 nucleotide sequence which contained the majority of the M2-2 open reading frame. The nucleotides encoding the first 13 amino acids at the N-terminus of the M2-2 gene product were not removed because this sequence overlaps M2-1. The cDNA fragment which contained M2-2 gene deletion was digested with SacI and BamHI and cloned back into a full-length RSV cDNA clone, designated pRSVΔM2-2

Infectious RSV with this M2-2 deletion was generated by transfecting pRSVΔM2-2 plasmid into MVA-infected Hep-2 cells expressing N, P and L genes. Briefly, pRSVΔM2-2 was transfected, together with plasmids encoding proteins N, P and L, into Hep-2 cells which had been infected with a recombinant vaccinia virus (MVA) expressing the T7 RNA polymerase.

Transfection and recovery of recombinant RSV was performed as follows. Hep-2 cells were split five hours or a day before the transfection in six-well dishes. Monolayers of Hep-2 cells at 70% - 80% confluence were infected with MVA at a multiplicity of infection (moi) of 5 and incubated at 35°C for 60 min. The cells were then washed once with OPTI-MEM (Life Technologies, Gaithersburg, M.D.). Each dish was replaced with 1 ml of OPTI-MEM and 0.2 ml transfection medium was added. The transfection medium was prepared by mixing 0.5 - 0.6 µg of RSV antigenome, 0.4 µg ofN plasmid, 0.4 µg of P plasmid, and 0.2 µg of L plasmid in a final volume of 0.1 ml OPTI-MEM medium. This was combined with 0.1 ml of OPTI-MEM containing 10 µl lipofecTACE (Life Technologies). After a 15 minute incubation at room temperature, the DNA/lipofecTACE mixture was added to the cells. The medium was replaced one day later with MEM containing 2% FBS. Cultures were further incubated at 35°C for 3 days and the supernatants harvested. Three days post-transfection, 0.3 - 0.4 ml culture supernatants were passaged onto fresh Hep-2 cells and incubated with MEM containing 2% FBS. After incubation for six days, the supernatant was harvested and the cells were fixed and stained by an indirect horseradish peroxidase method using goat anti-RSV antibody (Biogenesis) followed by a rabbit anti-goat antibody linked to horseradish peroxidase. The virus infected cells were then detected by addition of substrate chromogen (DAKO) according to the manufacturer's instructions. Recombinant RSV which contained M2-2 gene deletion was recovered from the transfected cells. Identification of rRSVΔM2-2 was performed by RT/PCR using primers flanking the deleted region. As shown in Fig. 12A, a cDNA fragment which is 234 nucleotides shorter than the wild-type RSV was detected in rRSVΔM2-2 infected cells.

No cDNA was detected in the RT/PCR reaction which did not contain reverse transcriptase in the RT reaction. This indicated that the DNA product was derived from viral RNA and not from contamination. The properties of the M2-2 deletion RSV are currently being evaluated.

### 10.3 GENERATION OF BOTH SH AND M2-2 DELETION MUTANT.

Both SH and M2-2 genes were deleted from the full-length RSV cDNA construct by cDNA subcloning. A Sac I to Bam HI fragment containing M2-2 deletion removed from cDNA subclone pET(SB)ΔM2-2RSV was cloned into pRSVΔSH cDNA clone. The double gene deletion plasmid pRSVΔSHΔM2-2 was confirmed by restriction enzyme mapping. As shown in Fig. 12B, the SH/M2-2 double deletion mutant is shorter than the wild-type pRSV cDNA.

Recovery of infectious RSV containing both the SH and M2-2 deletion was performed as described earlier. Infectious virus with both SH and M2-2 deleted was obtained from transfected Hep-2 cells.

### 11. EXAMPLE: GENERATION OF A HUMAN RESPIRATORY SYNCYTIAL VIRUS VACCINE (RSV) CANDIDATE BY DELETING A VIRAL ACCESSORY GENE(S) EITHER SINGLY OR IN COMBINATION

### Rationale:

Human respiratory syncytial virus is the major course of pneumonia and bronchiolitis in infants under one year of age. RSV is responsible for more than one in five pediatric hospital admissions due to respiratory tract disease and causes 4,500 deaths yearly in the USA alone. Despite decades of investigation to develop an effective vaccine against RSV, no safe and effective vaccine has been achieved to prevent the severe morbidity and significant mortality associated with RSV infection. Various approaches have been used to develop RSV vaccine candidates: formalin-inactivated virus, recombinant subunit vaccine of expressed RSV glycoproteins, and live attenuated virus. Recently, generation of live attenuated RSV mutants has been the focus for the RSV vaccine development. In the past, generation of live attenuated RSV mutant can only be achieved by in vitro passage and/or chemical mutagenesis. Virus was either underattenuated or overattenuated and was not genetically stable. The present investigation provides an immediate approach to generate genetically stable live attenuated RSV vaccines by deleting an accessory gene(s) individually or in combination. Gene deletions are considered to be a very powerful strategy for attenuating RSV because such deletions will not revert and the recombinant RSV deletion mutants are thus expected to be genetically very stable.

RSV is unique among the paramyxoviruses in its gene organization. In addition to the N, P, L, M, G and F genes which are common to all the paramyxoviruses, RSV contains four additional genes which encode five proteins: NS1, NS2, SH, M2-1 and M2-2. M2-1 and M2-2 are translated from two open reading frames that overlap in the middle of the M2 mRNA. M2-1 enhances mRNA transcriptional processivity and also functions as an antitermination factor by increasing transcriptional readthrough at the intergenic junctions (Collins, P. L. et al.Proc. Natl. Acad. Sci. U S A 93, 81-85 (1996); Hardy, R. W. et al. J. Virol. 72, 520-526 (1998)). However, the M2-2 protein was found to inhibit RSV RNA transcription and replication in vitro (Collins, P. L. et al.Proc. Natl. Acad Sci. USA 93, 81-85 (1996)). The accessory protein NS 1 was reported to be a potent transcription inhibitor (Atreya, P. L. et al., J. Virol. 72, 1452-1461 (1998)). The SH gene has been shown to be dispensable for RSV growth in tissue culture in a naturally occurring virus and in a recombinant RSV harboring an engineered SH deletion (Bukreyev, A.et al., J Virol 71(12), 8973-82 (1997); Karron, R. A. et al. J. Infect. Dis. 176, 1428-1436 (1997)). SH minus RSV replicates as well as the wild type RSV *in vitro.* Recently, it was reported that the NS2 gene could also be deleted (Teng, M. N., et al J Virol 73(1), 466-73 (1999); Buchholz, U. J. et al. J Virol 73(1), 251-9 (1999)). Deletion of M2-1, M2-2, and NS1 I has not been reported, neither was deletion of more than two nonessential genes reported.

Traditionally, live attenuated virus mutants were generated by passaging of RSV at lower temperature for many times and/or mutagenized by chemical reagents. The mutations are introduced randomly and the virus phenotype is difficult to maintain because revertants may develop. The ability to produce virus from an infectious cDNA makes it possible to delete gene or genes that are associated with virus pathogenesis. Gene deletion alone or in combination with mutations in the other viral genes (G, F, M, N, P and L) may yield a stably attenuated RSV vaccine to effectively protect RSV infection.

### 11.1 GENERATION OF A HUMAN RESPIRATORY SYNCYTIAL VIRUS VACCINE (RSV) CANDIDATE BY DELETING THE VIRAL M2-2 GENE

This example describes production of a recombinant RSV in which expression of the M2-2 gene has been ablated by removal of a polynucleotide sequence encoding the M2-2 gene and its encoded protein. The RSV M2-2 gene is encoded by M2-2 gene and its open reading frame is partially overlapped with the 5'-proximal M2-1 open reading frame by 12 amino acids (Collins, P. L. et al.Proc. Natl. Acad. Sci. USA 93, 81-85 (1996)). The predicted M2-2 polypeptide contains 90 amino acids, but the M2-2 protein has not yet been identified intracelluarly. The M2-2 protein down-regulates RSV RNA transcription and replication in a minigenome model system (Collins, P. L. et al. Proc. Natl. Acad. Sci. USA 92, 11563-11567 (1995)). The significance of this negative effect on RSV RNA transcription and replication in the viral replication cycle is not known.

### 11.1.1 RECOVERY OF RECOMBINANT RSV THAT LACKS THE M2-2 GENE

To produce a recombinant RSV that no longer expresses the M2-2 protein, the M2-2 gene was deleted from a parental RSV cDNA clone (Jin, H. et al. Virology 251, 206-214 (1998)). The antigenomic cDNA clone encodes a complete antigenomic RNA of strain A2 of RSV, which was used successfully to recover recombinant RSV. This antigenomic cDNA contains a single nucleotide change in the leader region at position 4 from C to G in its antigenomic sense. The construction of plasmid pA2ΔM2-2 involved a two step cloning procedure. Two *Hind III* restriction enzyme sites were introduced at RSV sequence of 8196 nt and 8430 nt respectively in a cDNA subclone (pET-S/B) that contained RSV *Sac I* (4477nt) to *BamH I* (8504nt) cDNA fragment using Quickchange mutagenesis kit (Strategene). Digestion of this cDNA clone with *Hind III* restriction enzyme removed the 234 nt *Hind III* cDNA fragment that contained the M2-2 gene. The remaining *Sac I* to *BamH I* fragment that did not contain the M2-2 gene was then cloned into a RSV antigenomic cDNA pRSVC4G. The resulting plasmid was designated as pA2ΔM2-2.

To recover recombinant RSV with the M2-2 open reading frame deleted, pA2ΔM2-2 was transfected, together with plasmids encoding the RSV N, P, and L proteins under the control of T7 promoter, into Hep-2 cells which had been infected with a modified vaccinia virus encoding the T7 RNA polymerase (MVA-T7). After 5 hours incubation of the transfected Hep-2 cells at 35°C, the medium was replaced with MEM containing 2% FBS and the cells were further incubated at 35°C for 3 days. Culture supernatants from the transfected Hep-2 cells were used to infect the fresh Hep-2 or Vero cells to amplify the rescued virus. Recovery of rA2ΔM2-2 was indicated by syncytial formation and confirmed by positive staining of the infected cells using polyclonal anti-RSV A2 serum. Recovered rA2ΔM2-2 was plaque purified three times and amplified in Vero cells. To confirm that rA2ΔM2-2 contained the M2-2 gene deletion, viral RNA was extracted from virus and subjected to RT/PCR using a pair of primers spanning the M2-2 gene. Viral RNA was extracted from rA2ΔM2-2 and rA2 infected cell culture supernatant by an RNA extraction kit (RNA STAT-50, Tel-Test, Friendswood, TX). Viral RNA was reverse transcribed with reverse transcriptase using a primer complementary to viral genome from 7430 nt to 7449 nt. The cDNA fragment spanning the M2-2 gene was amplified by PCR with primer V 1948 (7486 nt to 7515 nt at positive-sense) and primer V 1581 (8544 nt to 8525 nt at negative sense). The PCR product was analyzed on a 1.2 % agarose gel and visualized by EtBr staining. As shown in Fig. 13B, wild type rA2 yielded a PCR DNA product corresponding to the predicted 1029 nt fragment, whereas rA2ΔM2-2 yielded a PCR product of 795 nt, 234 nt shorter. Generation of RT/PCR product was dependent on the RT step, indicating that they were derived from RNA rather than from DNA contamination.

### 11.1.2 RNA SYNTHESIS OF rA2ΔM2-2

mRNA expression from cells infected with rA2ΔM2-2 or rA2 was analyzed by Northern blot hybridization analyses. Total cellular RNA was extracted from rA2ΔM2-2 or rA2 infected cells by an RNA extraction kit (RNA STAT-60, Tel-Test, Friendswood, TX). RNA was electrophoresed on a 1.2% agarose gel containing formaldehyde and transferred to a nylon membrane (Amersham Pharmacia Biotech, Piscataway, NJ). The membrane was hybridized with a RSV gene specific riboprobe labeled with digoxigenin (Dig). The hybridized RNA bands were visualized by using Dig-Luminescent Detection Kit for Nucleic Acids (Boehringer Mannheim, Indianapolis, IN). Hybridization of the membranes with riboprobes was done at 65°C, membrane washing and signal detection were performed according to the standard procedures. To examine mRNA synthesis from rA2ΔM2-2 and rA2, accumulation of the M2 mRNA and the other viral mRNA products in infected Vero cells was analyzed by Northern blot hybridization. Hybridization of the blot with a probe specific to the M2-2 open reading frame did not detect any signal in rA2ΔM2-2 infected cells. Instead, a shorter M2 mRNA was detected in rA2ΔM2-2 infected cells using a riboprobe specific to the M2-1 gene (Fig. 14A). These observations confirmed that the M2-2 gene was deleted from rA2ΔM2-2. Accumulation of the other nine RSV mRNA transcripts was also examined and the amounts of each mRNA were found to be comparable between rA2ΔM2-2 and rA2 infected cells. Examples of Northern blots probed with N, SH, G and F are also shown in Fig. 14A. Slightly faster migration of F-M2 bicistronic mRNA was also discernible due to the deletion of the M2-2 region.

The M2-2 protein was previously reported to be a potent transcriptional negative regulator in a minigenome replication assay. However, deletion of the M2-2 gene from virus did not appear to affect viral mRNA production in infected cells. To determine if levels of viral antigenome and genome RNA of rA2ΔM2-2 were also similar to rA2, the amount of viral genomic and antigenomic RNA produced in infected Vero and Hep-2 cells was examined by Northern hybridization. Hybridization of the infected total cellular RNA with a ³²P-labeled F gene riboprobe specific to the negative genomic sense RNA indicated that much less genomic RNA was produced in cells infected with rA2ΔM2-2 compared to rA2 (Fig. 14B). A duplicate membrane was hybridized with a ³²P-labeled F gene riboprobe specific to the positive sense RNA. Very little antigenomic RNA was detected in cells infected with rA2ΔM2-2, although the amount of the F mRNA in rA2ΔM2-2 infected cells was comparable to rA2. Therefore, it appears that RSV genome and antigenome synthesis was down-regulated due to deletion of the M2-2 gene. This down-regulation was seen in both Vero and Hep-2 cells and thus was not cell type dependent.

### 11.1.3 PROTEIN SYNTHESIS OF rA2ΔM2-2

Since the putative M2-2 protein has not been identified in RSV infected cells previously, it was thus necessary to demonstrate that the M2-2 protein is indeed encoded by RSV and produced in infected cells. A polyclonal antiserum was produced against the M2-2 fusion protein that was expressed in a bacterial expression system. To produce antiserum against the M2-2 protein of RSV, a cDNA fragment encoding the M2-2 open reading frame from 8155nt to 8430nt was amplified by PCR and cloned into the pRSETA vector (Invitrogen, Carlsbad, CA). pRSETA/M2-2 was transformed into BL21-Gold(DE3)plysS cells (Strategene, La Jolla, CA) and expression of His-tagged M2-2 protein was induced by IPTG. The M2-2 fusion protein was purified through HiTrap affinity columns (Amersham Pharmacia Biotech, Piscataway, NJ) and was used to immunize rabbits. Two weeks after a booster immunization, rabbits were bled and the serum collected.

Viral specific proteins produced from infected cells were analyzed by immunoprecipitation of the infected cell extracts or by Western blotting. For immunoprecipitation analysis, the infected Vero cells were labeled with ³⁵S-promix (100 µCi/ml ³⁵S-Cys and ³⁵S-Met, Amersham, Arlington Heights, IL) at 14 hr to 18 hr postinfection. The labeled cell monolayers were lysed by RIPA buffer and the polypeptides immunoprecipitated by polyclonal anti-RSV A2 serum (Biogenesis, Sandown, NH) or anti-M2-2 serum. Immunoprecipitation of rA2 infected Vero cell lysates with anti-M2-2 antibody produced a protein band of approximately 10 kDa, which is the predicated size for the M2-2 polypeptide. This polypeptide was not detected in rA2ΔM2-2 infected cells (Fig. 15A), confirming that M2-2 is a protein product produced by RSV and its expression was ablated from rA2ΔM2-2. The overall polypeptide pattern of rA2ΔM2-2 was indistinguishable from that of rA2. However, it was noted that slightly more P and SH proteins were produced in rA2ΔM2-2 infected Vero cells by immunoprecipitation. Nevertheless, by Western blotting analysis, a comparable amount of SH was produced in cells infected with rA2ΔM2-2 or rA2 (Fig.15B).

Immunoprecipitated polypeptides were electrophoresed on 17.5% polyacrylamide gels containing 0.1 % SDS and 4 M urea, and detected by autoradiography. For Western blotting analysis, Hep-2 and Vero cells were infected with rA2ΔM2-2 or rA2. At various times postinfection, virus infected cells were lysed in protein lysis buffer and the cell lysates were electrophoresed on 17.5% polyacrylamide gels containing 0.1% SDS and 4 M urea. The proteins were transferred to a nylon membrane. Immunoblotting was performed as described in Jin *et al.* (Jin, H. et al. Embo J 16(6), 1236-47 (1997)), using polyclonal antiserum against M2-1, NS1, or SH.

Western blotting was used to determine the protein synthesis kinetics of rA2ΔM2-2 in both Vero and Hep-2 cell lines. Hep-2 or Vero cells were infected with rA2ΔM2-2 or rA2 at moi of 0.5 and at various times of postinfection, the infected cells were harvested and the polypeptides separated on a 17.5% polyacrylamide gel containing 4 M urea. The proteins were transferred to a nylon membrane and probed with polyclonal antisera against the three accessory proteins: M2-1, NS1 and SH. Protein expression kinetics for all three viral proteins were very similar for rA2ΔM2-2 and rA2 in both Hep-2 and Vero cells (Fig.15B). Synthesis of the NS1 protein was detected at 10 hr postinfection, which was slightly earlier than M2-1 and SH because the NS 1 protein is the first gene translated and is a very abundant protein product in infected cells. Similar protein synthesis kinetics was also observed when the membrane was probed with a polyclonal antiserum against RSV (data not shown). Comparable M2-1 was detected in rA2ΔM2-2 infected cells, indicating that deletion of the M2-2 open reading frame did not affect the level of the M2-1 protein that is translated by the same M2 mRNA.

### 11.1.4 GROWTH ANALYSIS OF RECOMBINANT RSV IN TISSUE CULTURE

To compare plaque morphology of rA2ΔM2-2 with rA2, Hep-2 or Vero cells were infected with each virus and overlayed with semisolid medium composed of 1% methylcellulose and 1 X L15 medium with 2% FBS. Five days after infection, infected cells were immunostained with antisera against RSV A2 strain. Plaque size was determined by measuring plaques from photographed microscopic images. Plaque formation of rA2ΔM2-2 in Hep-2 and Vero cells was compared with rA2. As shown in Fig. 16, rA2ΔM2-2 formed pin point sized plaques in Hep-2 cells, with a reduction of about 5-fold in virus plaque size observed for rA2ΔM2-2 compared to rA2. However, only a slight reduction in plaque size (30%) was seen in Vero cells infected with rA2ΔM2-2.

A growth kinetics study of rA2ΔM2-2 in comparison with rA2 was performed in both Hep-2 and Vero cells. Cells grown in 6-cm dishes were infected with rA2 or rA2ΔM2-2 at a moi of 0.5. After I hr adsorption at room temperature, infected cells were washed three times with PBS, replaced with 4 ml of OPTI-MEM and incubated at 35°C incubator containing 5% CO₂. At various times post-infection, 200µl culture supernatant was collected, and stored at -70°C until virus titration. Each aliquot taken was replaced with an equal amount of fresh medium. Virus titer was determined by plaque assay in Vero cells on 12-well plates using an overlay of 1% methylcellulose and 1 X L15 medium containing 2% FBS. As seen in Fig. 17, rA2ΔM2-2 showed very slow growth kinetics and the peak titer of rA2ΔM2-2 was about 2.5-3 log lower than that of rA2 in Hep-2 cells. In Vero cells, rA2ΔM2-2 reached a peak titer similar to rA2. To analyze virus replication in different host cells, each cell line grown in 6-well plates was infected with rA2ΔM2-2 or rA2 at moi of 0.2. Three days postinfection, the culture supernatants were collected and virus was quantitated by plaque assay. rA2ΔM2-2 was examined for its growth properties in various cell lines that derived from different hosts with different tissue origins (Table 3). Significantly reduced replication of rA2ΔM2-2, two orders of magnitude less than rA2, was observed in infected Hep-2, MRC-5, and Hela cells, all of human origin. However, replication of rA2ΔM2-2 was only slightly reduced in MDBK and LLC-MK2 cells that are derived from bovine and rhesus monkey kidney cells, respectively.

**Table 3. Replication levels of rA2 M2-2 and rA2 in various cell lines**

| Cell lines | Host | Tissue origin | Virus titer [log₁₀(pfu/ml)] | |
|---|---|---|---|---|
| | | | rA2 | rA2ΔM2-2 |
| Vero | Monkey | Kidney | 6.1 | 6.1 |
| Hep-2 | Human | Larynx | 6.2 | 4.3 |
| MDBK | Bovine | Kidney | 6.1 1 | 5.5 |
| MRC-5 | Human | Lung | 5.5 | 3.0 |
| Hela | Human | Cervix | 6.6 | 4.5 |
| LLC-MK2 | Monkey | Kidney | 6.7 | 6.1 |

### 11.1.5 REPLICATION OF rA2ΔM2-2

### IN MICE AND COTTON RATS

Virus replication *in vivo* was determined in respiratory pathogen-free 12-week-old Balb/c mice (Simonsen Lab., Gilroy, CA) and S. Hispidus cotton rats (Virion Systems, Rockville, MD). Mice or cotton rats in groups of 6 were inoculated intranasally under light methoxyflurane anesthesia with 10⁶ pfu per animal in a 0.1-ml inoculum of rA2 or rA2ΔM2-2. On day 4 postinoculation, animals were sacrificed by CO₂ asphyxiation and their nasal turbinates and lungs were obtained separately. Tissues were homogenized and virus titers were determined by plaque assay in Vero cells. To evaluate immunogenicity and protective efficacy, three groups of mice were inoculated intranasally with rA2, rA2ΔM2-2 or medium only at day 0. Three weeks later, mice were anesthetized, serum samples were collected, and a challenge inoculation of 10⁶ pfu of biologically derived wild type RSV strain A2 was administered intranasally. Four days post-challenge, the animals were sacrificed and both nasal turbinates and lungs were harvested and virus titer determined by plaque assay. Serum antibodies against RSV A2 strain were determined by 60% plaque reduction assay (Coates, H.V. et al., AM. J. Epid. 83:299-313 (1965)) and by immunostaining of RSV infected cells.

**Table 4. Replication of rA2AM2-2 and rA2 in cotton rats**

| Virus | Virus titer (mean log₁₀ pfu/g tissue _ SE)^{a} | |
|---|---|---|
| | Nasal turbinates | Lung |
| rA2 | 4.0 0.33 | 5.5 0.12 |
| rA2ΔM2-2 | < 1.4 | < 1.4 |

| | | |
|---|---|---|
| ^{a} Groups of six cotton rats were immunized intranasally with 10⁶ pfu of the indicated virus on day 0. The level of infected virus replication at day 4 was determined by plaque assay on indicated specimens, and the mean log₁₀ titer _ standard error (SE) per gram tissue were determined. | | |

To evaluate levels of attenuation and immunogenicity of rA2ΔM2-2, replication of rA2ΔM2-2 in the upper and lower respiratory tract of mice and cotton rats was examined. Cotton rats in groups of 6 were inoculated with 10⁶ pfu ofrA2AM2-2 or rA2 intranasally. Animals were sacrificed at 4 days postinoculation, their nasal turbinates and lung tissues were harvested, homogenized, and levels of virus replication in these tissues were determined by plaque assay. rA2ΔM2-2 exhibited at least 2 log reduction of replication in both nasal turbinates and lungs of the infected cotton rats (Table 4). No virus replication was detected in cotton rats infected with rA2ΔM2-2, whereas a high level of wild type rA2 virus replication was detected in both the upper and lower respiratory tract of cotton rats. Attenuation of rA2ΔM2-2 was also observed in mice. Geometric mean titer of virus replication and standard error obtained from two experiments are shown in Table 5. rA2ΔM2-2 replication was only detected in one or two of 12 infected mice. The replication was limited, only a few plaques were observed at 10⁻¹ dilution of the tissue homogenates. Despite its restricted replication in mice, rA2ΔM2-2 induced significant resistance to challenge with wild type A2 RSV (Table 5). When mice previously inoculated with rA2ΔM2-2 or rA2 were inoculated intranasally with 10⁶ pfu dose of wild type A2 strain, no wild type A2 virus replication was detected in the upper and lower respiratory tract of mice. Therefore, rA2ΔM2-2 was fully protective against wild type A2 virus challenge.

The immunogenicity of rA2ΔM2-2 was also examined. Two groups of mice were infected with rA2ΔM2-2 or rA2, and three weeks later, serum samples were collected. The serum neutralization titer was determined by 50% plaque reduction titer. The neutralization titer from rA2ΔM2-2 infected mice was comparable to that of rA2, both had 60% plaque reduction titer at 1:16 dilution. The serum obtained from rA2ΔM2-2 infected mice was also able to immunostain RSV plaques, confirming that RSV-specific antibodies were produced in rA2ΔM2-2 infected mice.

**Table 5. Replication of rA2AM2-2 and rA2 in mice, and protection against wild type A2 RSV challenge**

| Immunizing | Virus replication^{a} | | RSV A2 replication after challenge^{b} | |
|---|---|---|---|---|
| Virus | (Mean log₁₀ pfu/g tissue _ SE) | | (Mean log₁₀ pfu/g tissue _ SE) | |

| | Nasal turbinates | Lung | Nasal turbinates | Lung |
|---|---|---|---|---|
| rA2 | 3.72_0.33 | 4.0_0.13 | < 1.4 | < 1.4 |
| rA2ΔM2-2 | < 1.4 | < 1.4 | < 1.4 | < 1.4 |
| Control | < 1.4 | < 1.4 | 3.53_0.17 | 4.10_0.13 |

| | | | | |
|---|---|---|---|---|
| ^{a} Groups of 12 Balb/c mice were immunized intranasally with 10⁶ pfu of the indicated virus on day 0. The level of infected virus in indicated tissues was determined by plaque assay at day 4, and the mean log₁₀ titer _ standard error (SE) per gram tissue were determined. ^{b} Groups of 6 Balb/c Mice were intranasally administered with 10⁶ pfu of RSV A2 on day 21 and sacrificed 4 days later. Replication of wild type RSV A2 in tissues as indicated was determined by plaques assay, and the mean log₁₀ titer_ standard error (SE) per gram tissue were determined. | | | | |

The two RSV antigenic subgroups, A and B, exhibit a relatively high degree of conservation in M2-2 proteins, suggesting functional importance for the M2-2 protein. Transcriptional analysis for rA2 and rA2ΔM2-2 yielded important findings within the present example. Although overall mRNA transcriptional levels were substantially the same for both viruses, Northern blot analysis revealed dramatic reduction of virus genome and antigenome RNA for rA2ΔM2-2 compared to wild type rA2. This finding is contradictory with what has been reported for the negative transcriptional regulation of the M2-2 protein in a minigenome system. It thus appears that the functional role of M2-2 in the virus life cycle is more complicated than previously thought. Nevertheless, the reduction in the level of genome and antigenome of rA2ΔM2-2 did not appear to affect virus yields in infected Vero cells.

The finding that rA2ΔM2-2 exhibited host range restricted replication in different cell lines provided a good indication that deletion of a nonessential gene is a good means to create a host range mutant, which can be a very important feature for vaccine strains. rA2ΔM2-2 did not replicate well in several cell lines that are derived from human origin, lower virus yield was produced from these cell lines. However, the levels of protein synthesis in Hep-2 cells were similar to Vero cells that produced high levels of rA2ΔM2-2. This indicated that the defect in virus release was probably due to a defect in a later stage, probably during the virus assembly process.

The finding that the M2-2 minus virus grows well in Vero cells and exhibits attenuation in the upper and lower respiratory tracts of mice and cotton rats presents novel advantages for vaccine development. The reduced replication in respiratory tracts of rodents did not affect immunogenicity and protection against challenging wild type virus replication, indicating that this M2-2 minus virus may serve as a good vaccine for human use. The nature of the M2-2 deletion mutation, involving a 234 nt deletion, represents a type of mutation that will be highly refractory to reversion.

### 11.5 GENERATION OF A HUMAN RESPIRATORY SYNCYTIAL VIRUS VACCINE (RSV) CANDIDATE BY DELETING THE VIRAL M2-2 AND SH GENES

This example describes production of a recombinant RSV in which expression of two RSV genes, M2-2 and SH, has been ablated by removal of polynucleotide sequences encoding the M2-2 and SH genes and their encoded proteins. As described earlier, the M2-2 or SH gene is dispensable for RSV replication *in vitro.* It is possible that deletion of two accessory genes will produce a recombinant RSV with a different attenuation phenotype. The degree of attenuation from deletion of two genes can be increased or decreased.

SH and M2-2 genes were deleted from the full-length RSV cDNA construct through cDNA cloning. A *Sac I* to *BamH I* fragment that contained M2-2 deletion in the pET(SB) subclone as described earlier was removed by digestion with *Sac I* and *BamH I* restriction enzymes and was cloned into the full-length RSV antigenomic cDNA clone that contained the SH gene deletion (pA2ΔSH). The resulting plasmid that contained deletion of SH and M2-2 was designated pA2ΔSHΔM2-2. Deletion of SH and M2-2 in pA2ΔSHΔM2-2 plasmid was confirmed by restriction enzyme mapping.

Generation of rA2ΔSHΔM2-2 mutant was performed as described above (see Section 7). Recombinant RSV that contained a deletion of the SH and M2-2 genes (rA2ΔSHΔM2-2) was recovered from MVA-infected cells that had been co-transfected with pA2ΔSHΔM2-2 together with three plasmids that expressed the N, P and L proteins, respectively. Recovery of infectious RSV deletion mutant was indicated by syncytial formation and confirmed by immunostaining with an antibody against RSV.

Deletion of the SH and M2-2 genes in rA2ΔSHΔM2-2 was confirmed by RT/PCR using two sets of primers that flanked the SH gene and the M2-2 gene, respectively. mRNA expression from cells infected with rA2ΔSHΔM2-2 or rA2 was analyzed by Northern blot hybridization analyses as described earlier. Both SH and M2-2 mRNAs were not detected in cells infected with rA2ΔSHΔM2-2 using a probe that was specific to the SH gene or M2-2 gene. The fact that two RSV genes (SH and M2-2) can be deleted from RSV indicates that the SH and M2-2 proteins are dispensable for RSV replication. In contrast to rA2ΔM2-2 that formed very small plaques in Hep-2 cells, rA2ΔSHΔM2-2 had a plaque size larger than rA2ΔM2-2 (Fig. 19).

A growth kinetics study of rA2ΔSHΔM2-2 in comparison with rA2 was performed in Vero cells. Cells grown in 6-cm dishes were infected with rA2 or rA2ΔSHΔM2-2 at a moi of 0.2. As seen in Fig. 22, rA2ΔSHΔM2-2 showed slower growth kinetics and its peak titer was about 1.5 log lower than that of rA2. This indicated that rA2ΔSHΔM2-2 is attenuated in tissue culture.

To evaluate the level of attenuation of rA2ΔSHΔM2-2, replication of rA2ΔSHΔM2-2 in the lower respiratory tracts of mice was examined. Mice in groups of 6 were inoculated with 10⁶ pfu of rA2ΔSHΔM2-2 or rA2 intranasally. Animals were sacrificed at 4 days postinoculation, their nasal turbinates and lung tissues were harvested, homogenized, and levels of virus replication in these tissues were determined by plaque assay. rA2ΔSHΔM2-2 exhibited about a 2 log reduction of replication in lungs of the infected mice (Table 11). This data indicated that rA2ΔSHΔM2-2 is attenuated in mice, although the degree of attenuation is not as significant as rA2ΔM2-2.

**Table 11. Replication of rA2ΔSHΔM2-2 and rA2 in mice**

| Virus | Virus titer in lung (mean log₁₀ pfu/g tissue _ SE)^{a} |
|---|---|
| rA2 | 4.2 _ 0.08 |
| rA2ΔSHΔM2-2 | 2.4_1.2 |

| | |
|---|---|
| ^{a} Groups of six mice were immunized intranasally with 10⁶ pfu of the indicated virus on day 0. The level of infected virus replication at day 4 was determined by plaque assay on indicated specimens, and the mean log₁₀ titer _ standard error (SE) per gram tissue were determined. | |

### 11.6 GENERATION OF A HUMAN RESPIRATORY SYNCYTIAL VIRUS VACCINE (RSV) CANDIDATE BY DELETING THE VIRAL M2-2 AND NS1 GENES

This example describes production of a recombinant RSV in which expression of two different RSV genes, NS1 and M2-2, has been ablated by removal of polynucleotide sequences encoding the NS1 and M2-2 genes and their encoded proteins. As described earlier, NS1 and M2-2 gene alone is dispensable for RSV replication *in vitro.* This example provided a different attenuating method by deletion of two accessory genes from RSV.

NS1 and M2-2 genes were deleted from the full-length RSV cDNA construct through cDNA cloning. A *Xma I* to *Avr II* fragment that contained NS1 deletion in pET(X/A) subclone was removed by digestion with *Xma I* and *Avr II* restriction enzymes and was cloned into the full-length RSV antigenomic cDNA clone that contained the M2-2 gene deletion (pA2ΔM2-2). The resulting plasmid that contained deletion of both NS1 and M2-2 was designated pA2ΔNS1 ΔM2-2. Deletion of NS1 and M2-2 in pA2ΔNS1ΔM2-2 plasmid was confirmed by restriction enzyme mapping.

Generation rA2ΔNS 1ΔM2-2 mutant was performed as described above (see section 11.2). Recombinant RSV that contained deletion of NS1 and M2-2 genes was recovered from MVA-infected cells that had been co-transfected with pA2ΔNS 1ΔM2-2 together with three plasmids that expressed the N, P and L proteins, respectively. Recovery of infectious RSV was indicated by syncytial formation and confirmed by immunostaining with an antibody against RSV. Identification of the recovered rA2ΔNS I ΔM2-2 was confirmed by RT/PCR using a pair of primers flanking the NS 1 gene and the M2-2 gene.

Replication of rA2ΔNS 1ΔM2-2 in tissue culture cell lines and in small animal models is being studied. Preliminary *in vitro* data indicated that rA2ΔNS1ΔM2-2 is very attenuated in tissue culture cells and recombinant RSV containing deletion of NS1 and M2-2 genes is more attenuated than rA2ΔSHΔM2-2.

### 11.7 GENERATION OF A HUMAN RESPIRATORY SYNCYTIAL VIRUS VACCINE (RSV) CANDIDATE BY DELETING THE VIRAL NS2 AND M2-2 GENES

This example describes production of a recombinant RSV in which expression of two different RSV genes, NS2 and M2-2, has been ablated by removal of polynucleotide sequences encoding the NS2 and M2-2 genes and their encoded proteins. As described earlier, NS2 or M2-2 gene is dispensable for RSV replication *in vitro.* It is possible that deletion of two accessory genes from RSV will produce a recombinant RSV with a different attenuation phenotype.

NS2 and M2-2 genes were deleted from the full-length RSV cDNA construct through cDNA cloning. A *Xma I* to *Avr II* fragment that contained NS2 deletion in pET(X/A) subclone was removed by digestion with *Xma I* and *Avr II* restriction enzymes and was cloned into the full-length RSV antigenomic cDNA clone that contained the M2-2 gene deletion (pA2ΔM2-2). The resulting plasmid that contained deletion of both NS2 and M2-2 was designated pA2ΔNS2 ΔM2-2. Deletion of NS2 and M2-2 in pA2ΔNS2ΔM2-2 plasmid was confirmed by restriction enzyme mapping.

Generation of rA2ΔNS2ΔM2-2 mutant was performed as described above (see Section 7). Recombinant RSV that contained deletion in the NS2 and M2-2 genes (rA2ΔNS2ΔM2-2) was recovered from MVA-infected cells that had been co-transfected with pA2ΔNS2ΔM2-2 together with three plasmids that expressed the N, P and L proteins, respectively. Recovery of infectious RSV was indicated by syncytial formation and confirmed by immunostaining with an antibody against RSV. Identification of the recovered rA2ΔNS2ΔM2-2 was confirmed by RT/PCR using two pairs of primers flanking the NS2 or M2-2 gene, respectively.

mRNA expression from cells infected with rA2ΔNS2ΔM2-2 or rA2 was analyzed by Northern blot hybridization analyses. As shown in Fig. 23, neither NS2 nor M2-2 mRNA was detected in cells infected with rA2ΔNS2ΔM2-2 using a probe that was specific to the NS2 gene or to the M2-2 gene. Comparable levels of NS 1 and SH mRNA expression was observed in cells infected with rA2ΔNS2ΔM2-2 Northern blot data confirmed that expression of both NS2 and M2-2 was ablated in rA2ΔNS2ΔM2-2.

A growth kinetics study of rA2ANS2AM2-2 in comparison with rA2 was performed in Vero cells. Cells grown in 6-cm dishes were infected with rA2 or rA2ΔNS2ΔM2-2 at a moi of 0.2. As seen in Fig. 24, rA2ΔNS2ΔM2-2 showed very slow growth kinetics and its peak titer was about 10-fold lower than that of rA2. To analyze virus replication in different host cells, each cell line grown in 6-well plates was infected with rA2ΔNS2ΔM2-2 or rA2 at moi of 0.2. Three days postinfection, the culture supernatants were collected and virus was quantitated by plaque assay. rA2ΔNS2ΔM2-2 had about a few fold reduction in virus titer compared to rA2 in Vero cells. However, a 2-3 log reduction in virus titer was observed in Hep-2, MDBK, Hela, MRC5and LLC-MK2 cells (Table 12). Therefore, replication of rA2ΔNS2ΔM2-2 exhibits a substantial host range effect, which is an indication of attenuation.

**Table 12. Growth comparison of rA2ΔNS2/M2-2 and rA2 in different cell lines**

| | Virus titer [log₁₀(pfu/ml)] | |
|---|---|---|
| Cell lines | rA2 | rA2ΔNS2/M2-2 |
| Vero | 6.4 | 5.7 |
| Hep-2 | 6.7 | 3.5 |
| MDBK | 6.7 | 3.7 |
| MRC-5 | 5.9 | 2.0 |
| Hela | 6.5 | 2.9 |
| LLC-MK2 | 6.7 | 4.8 |

Replication of rA2ΔNS2/M2-2 *in vivo* was determined in respiratory pathogen-free 4-week old cotton rats. Cotton rats in groups of 5 were inoculated intranasally under light methoxyflurane anesthesia with 10⁵ pfu per animal in a 0.1-ml inoculum of rA2 or rA2 ΔNS2ΔM2-2. On day 4 postinoculation, animals were sacrificed by CO₂ asphyxiation and their nasal turbinates and lungs were obtained separately. Tissues were homogenized and virus titers were determined by plaque assay in Vero cells. As shown in Table 13, no virus replication was detected in the upper and lower respiratory tracts of cotton rats that were infected with rA2 ΔNS2ΔM2-2. This indicated that deletion of the NS2 and M2-2 genes severely attenuated RSV. Thus, this recombinant RSV with an NS2 and M2-2 deletion might serve as a good vaccine candidate for human use.

**Table 13. Replication of rA2ΔNS2/M2-2 and rA2 in cotton rats**

| Virus | Virus titer (mean log₁₀ pfu/g tissue _ SE) | |
|---|---|---|
| | Nasal turbinates | Lung |
| rA2 | 2.30_0.26 | 4.23_0.10 |
| rA2ΔNS2/M2-2 | < 1.4 | < 1.4 |

| | | |
|---|---|---|
| ^{a} Groups of five cotton rats were immunized intranasally with 10⁵ pfu of the indicated virus on day 0. The level of infected virus replication at day 4 was determined by plaque assay on indicated specimens, and the mean log₁₀ titer _ standard error (SE) per gram tissue were determined. | | |

In summary, 11 different gene deletion mutants 9not all in accordance with the invention) have been obtained as summarized in Table 17. Four RSV accessory genes have been deleted either individually or in combination. These different deletion mutants showed different plaque formation and growth properties. A good correlation was demonstrated between plaque size *in vitro* and attenuation *in vivo.* These different RSV deletion mutants provide several choices for use as potential RSV vaccine candidates.

**Table 17. Summary of RSV gene deletion mutants**

| Virus | Recovered |
|---|---|
| ΔM2-2 | Yes |
| ΔSH | Yes |
| ΔNS1 | Yes |
| ΔNS2 | Yes |
| ΔM2-2ΔSH | Yes |
| ΔM2-2ΔNS1 | ND^{a} |
| ΔM2-2ΔNS2 | Yes |
| ΔNS1ΔNS2 | Yes |
| ΔSHΔNS1 | Yes |
| ΔSHΔNS2 | Yes |
| ΔSHΔNS1ΔNS2 | Yes |

| | |
|---|---|
| ^{a} ND Not determined. Replication of rA2ΔM2-2ΔNS 1 was not detected in tissue culture. | |

### 13. EXAMPLE: CHIMERIC SUBGROUP A RESPIRATORY SYNCYTIAL VIRUS (RSV) WITH THE GLYCOPROTEINS OF SUBGROUP B AND RSV WITHOUT THE M2-2 GENE ARE ATTENUATED IN AFRICAN GREEN MONKEYS

### 13.1 INTRODUCTION

In this study, rA2ΔM2-2 was evaluated for its attenuation, immunogenicity, and protective efficacy against subsequent wild type RSV challenge in African green monkeys. The replication of rA2ΔM2-2 was more than 1000-fold restricted in both the upper and lower respiratory tracts of the infected monkeys and it induced titers of serum anti-RSV neutralizing antibody that were slightly lower than those induced by wild type rA2. When rA2ΔM2-2-infected monkeys were challenged with wild type A2 virus, the replication of the challenge virus was reduced by approximately 100-fold in the upper respiratory tract and 45,000-fold in the lower respiratory tracts. To further attenuate rA-G_{B}F_{B}, the M2-2 open reading frame was removed from rA-G_{B}F_{B}. As described for rA2ΔM2-2, rA-G_{B}F_{B}ΔM2-2 was restricted for growth in Hep-2 cells and was attenuated in cotton rats. rA2 and rA-G_{B}F_{B}_bearing a deletion of the M2-2 gene could represent a bivalent RSV vaccine composition for protection against multiple strains from the two RSV subgroups.

African green monkeys (AGM) were evaluated as a non-human primate model for assessing the attenuation, immunogenicity and protective efficacy of RSV vaccine candidates. We showed that rA2 replicated to high titers in both the upper and lower respiratory tracts of AGM, whereas rA2ΔM2-2 and rA-G_{B}F_{B} replicated poorly in the respiratory tracts of monkeys. Both rA2ΔM2-2 and rA-G_{B}F_{B} induced neutralizing antibodies which protected the animals from experimental challenge.

### 13.2 MATERIALS AND METHODS

### Cells and viruses

Monolayer cultures of HEp-2 and Vero cells (obtained from American Type Culture Collections, ATCC) were maintained in minimal essential medium (MEM) containing 5% fetal bovine serum (FBS). Wild type RSV strains, A2 and B9320, were obtained from ATCC and grown in Vero cells. Modified vaccinia virus Ankara (MVA-T7) expressing bacteriophage T7 RNA polymerase was grown in CEK cells.

### Construction of chimeric cDNA clone

The wild type RSV B9320 was grown in Vero cells and the viral RNA was extracted from infected cell culture supernatant. A cDNA fragment containing the G and F genes of RSV B9320 was obtained by RT/PCR using the following primers: ATCA*GGATCC*ACAATAACATTGGGGCAAATGCAACC and CTGGCATTC*GGATCC*GTTTTATGTAACTATGAGTTG (the BamH I sites engineered for cloning is in italics and B9320 specific sequences are underlined). BamH I restriction enzyme sites were introduced upstream of the gene start sequence of G and downstream of the gene end sequence of F. The PCR product was first introduced into the T/A cloning vector (Invitrogen) and the sequences were confirmed by DNA sequencing. The BamH I restriction fragment containing the G and F gene cassette of B9320 was then transferred into a RSV cDNA subclone pRSV(R/H) that contained RSV sequences from nt 4326 to nt 9721 through the introduced Bgl II sites at nt 4655 (upstream of the gene start signal of G) and at nt 7552 (downstream of the gene end signal of F). Introduction of these two Bgl II sites were made by PCR mutagenesis using the QuickChange mutagenesis kit (Strategene, La Jolla, CA). BamH I and Bgl II restriction enzyme sites have compatible ends but ligation obliterates both restriction sites. The Xho I (nt 4477) to BamH I (nt 8498) restriction fragment containing the G and F genes of B9320 was then shuttled into the infectious RSV antigenomic cDNA clone pRSVC4G (Jin et al., 1998). The chimeric antigenomic cDNA was designated pRSV -G_{B}F_{B}. To delete the M2-2 gene from pRSV-G_{B}F_{B}, the Msc I (nt 7692) to BamH I (nt 8498) fragment from rA2ΔM2-2 which contained the M2-2 deletion (Jin et al., 2000a) was introduced into pRSV-G_{B}F_{B}. The chimeric cDNA clone that lacks the M2-2 gene was designated pRSV-G_{B}F_{B}ΔM2-2.

### Recovery of recombinant RSV

Recovery of recombinant RSV from cDNA is described herein. Briefly, HEp-2 cells in 6-well plate at 80% confluence were infected with MVA at an m.o.i. of 5 pfu/cell for 1 h and then were transfected with full-length antigenomic plasmids (pRSV-G_{B}F_{B} or pRSV-G_{B}F_{B}ΔM2-2), together with plasmids expressing the RSV N, P, and L proteins using LipofecTACE reagent (Life Technologies, Gaithersburg, MD). After incubating the transfected cells at 35°C for three days, the culture supernatants were passaged in Vero cells for six days to amplify rescued virus. The recovered recombinant viruses were biologically cloned by three successive plaque purifications and further amplified in Vero cells. Virus recovered from pRSV -G_{B}F_{B} transfected cells was designated rA-G_{B}F_{B} and that from pRSV-G_{B}F_{B}ΔM2-2 transfected cells was designated rA-G_{B}F_{B}OM2-2. Virus titer was determined by plaque assay and plaques were visualized by immunostaining using polyclonal anti-RSV A2 serum (Biogenesis, Sandown, NH).

### Virus characterization

The expression of viral RNA for each recovered chimeric RSV was analyzed by Northern blotting. Total cellular RNA was extracted from virus infected cells at 48 hr post-infection. The RNA blot was hybridized with a γ-³²P-ATP labeled oligonucleotide probe specific for the F gene of B9320 (GAGGTGAGGTACAATGCATTAATAGCAAGATGGAGGAAGA) or a γ-³²P-ATP labeled probe specific for the F gene of A2 (CAGAAGCAAAACAAAATGTGACTGCAGTGAGGATTGTGGT). To detect the G mRNA of the chimeric viruses, RNA blots were hybridized with a 190-nt riboprobe specific to the G gene of B9320 or a 130nt riboprobe specific to the G gene of A2. Both riboprobes were labeled with α-³²P-CTP. Hybridization was performed at 65°C in Express Hyb solution (Clontech, Palo Alto, CA) overnight. Membranes were washed at 65°C under stringent condition and exposed to film.

Viral specific proteins from infected cells were analyzed by immunoprecipitation of the infected cell extracts or by Western blotting. To immunoprecipitate viral proteins, Vero cells were infected with virus at an moi of 1.0 and labeled with ³⁵S-promix (100 µCi/ml ³⁵S-Cys and ³⁵S-Met, Amersham, Arlington Heights, IL) from 14 hr to 18 hr postinfection. The labeled cell monolayers were lysed with RIPA buffer and the polypeptides immunoprecipitated by polyclonal goat anti-RSV A2 serum (Biogenesis, Sandown, NH) or by a polyclonal antibody against the M2-2 protein. Immunoprecipitated polypeptides were electrophoresed on SDS-PAGE and detected by autoradiography. For Western blotting analysis, virus infected Vero cells were lysed in protein lysis buffer and the proteins were separated on 12% SDS-PAGE. The proteins were transferred to a nylon membrane and immunoblotting was performed as described herein, using a monoclonal antibody against the G protein of B9320 or a monoclonal antibody against the G protein of A2 (Storch and Park, 1987 J. Med. Virol. 22:345-356).

Growth of chimeric RSV in vitro was compared with wild type recombinant A2 (rA2) and rA2ΔM2-2. Growth cycle analysis was performed in both HEp-2 and Vero cells. Cells grown in 6-cm dishes were infected with each virus at a moi of 0.01 or 0.1. After 1 hr adsorption at room temperature, the infected cell monolayers were washed three times with PBS, and incubated at 35°C with 4 ml of Opti-MEM in an incubator containing 5% CO₂. At various times post-infection, 200 µl of the culture supernatant was collected, and stored at -70°C for virus titration. Each aliquot removed was replaced with an equal amount of fresh medium. Virus titer was determined by plaque assay in Vero cells on 12-well plates using an overlay of 1% methylcellulose and 1 X L 15 medium containing 2% FBS.

### Virus replication in cotton rats

Virus replication *in vivo* was determined in respiratory pathogen-free *S. Hispidus* cotton rats. Cotton rats in groups of 12 were inoculated intranasally under light methoxyflurane anesthesia with 10^{5.5} pfu of virus per animal in a 0.1-ml inoculum. On day 4 post-inoculation, six animals were sacrificed by CO₂ asphyxiation and their nasal turbinates and lungs were harvested separately. Tissues were homogenized and virus titers determined by plaque assay in Vero cells. Three weeks later, the remaining 6 animals were anesthetized, their serum samples were collected, and a challenge inoculation of 10⁶ pfu of biologically derived wild type RSV strain A2 or B9320 administered intranasally. Four days post-challenge, the animals were sacrificed and both nasal turbinates and lungs were harvested, homogenized and virus titer determined by plaque assay. Serum neutralizing antibodies against RSV A2 or B9320 strain were determined by a 50% plaque reduction assay (Coates et al., 1966, Am. J. Epidemiol. 83(2):299-313).

### Virus replication in AGM

Recombinant RSV was evaluated for their replication, immunogenicity and protective efficacy in AGM (Cercopithecus aethiops). AGM, obtained from St. Kitts with an average age of 4.2 years and body weight ranging from 2.2 to 4.3 kg, were used in the first study (study A) to compare the replication of rA2 with wild type A2. The second study (study B) used AGM with ages ranging from 5.3 to 8.4 years and an average body weight of 4.15 kg. None of the monkeys had detectable serum neutralizing antibodies for RSV B9320 or A2 (titer < 1:10). Groups of 4 monkeys were inoculated with either wild type A2, rA2, rA2ΔM2-2, wild type B9320 or rA-G_{B}F_{B} by both intranasal and intratracheal route with a dose of 10^{5.5} pfu in a 1.0 ml inoculum at each site. Following inoculation, daily nasopharyngeal (NP) swabs were collected from each monkey for 12 days under Telazol anesthesia and tracheal lavage (BAL) were collected on days 3, 5, 7 and 10 post-infection (Kakuk et al., 1993, J. Infect. Dis. 167(3):553-561). On day 28 post-infection, serum samples were collected from each infected monkey and the monkeys were challenged with either wild type A2 or B9320 at both the intranasal and intratracheal sites with a dose of 10^{5.5} pfu in a 1.0-ml inoculum. Replication of the challenge virus in the upper and lower respiratory tracts of monkeys was examined by quantitation of virus shed in NP and tracheal lavage specimens. The NP samples were collected daily for 10 days and BAL samples were collected on days 3, 5, 7 and 10 post-challenge. Fourteen days after wild type virus challenge, serum samples were collected for measurement of RSV neutralizing antibody by the 50% plaque reduction assay using wild type A2 or B9320 viruses. The virus shed in the NP and BAL samples were quantitated by plaque assay using Vero cells.

### 13.3 RESULTS

### Construction of cDNA and recovery of RSV A/B chimeric virus

Previously, we constructed an infectious antigenomic cDNA encoding wt RSV strain A2 and its derivative bearing a deletion of the M2-2 gene. Here, these cDNAs were modified by replacing the G and F genes of the A2 strain with those of B9320 to produce chimeric viruses expressing RSV subgroup B antigens. The gene start and gene end sequences are very conserved between the two RSV subgroups. Therefore, the complete G and F genes of B9320 including their own gene start and gene end signals were transferred to the A2 cDNA backbone (Fig. 26). The cDNA encoding the G and F genes of B9320 was obtained by RT/PCR and confirmed by sequence analysis. The constructed chimeric cDNA was designated pRSVA-G_{B}F_{B}. pRSVA-G_{B}F_{B}ΔM2-2 was constructed by deleting the M2-2 gene from pRSVA-G_{B}F_{B}. The M2 gene containing the deletion of the M2-2 open reading frame from rA2ΔM2-2 was introduced into pRSVA-G_{B}F_{B} through the unique Msc I and BamH I restriction enzyme sites. Both chimeric viruses (rA-G_{B}F_{B} and rA-G_{B}F_{B}ΔM2-2) were recovered from cDNA using the previously described rescue system. The recovered recombinant viruses were plaque-purified and amplified in Vero cells.

### Characterization of the recombinant chimeric viruses in vitro

Expression of the subgroup specific proteins by the chimeric viruses was analyzed by Northern and Western blotting. Using strain specific probes, B9320-specific G and F mRNAs were detected in cells infected with rA-G_{B}F_{B} and rA-G_{B}F_{B}AM2-2 (Fig. 27A). The M2-2 gene was not detected in cells infected with rA-G_{B}F_{B}ΔM2-2, confirming that the M2-2 gene was deleted from this chimeric virus. The B9320 strain specific protein expression of the two chimeric viruses was also compared with that of rA2, rA2ΔM2-2 and wild type B9320 (Fig. 27B). The F1 protein of rA-G_{B}F_{B} and rA-G_{B}F_{B}ΔM2-2 showed the same rate of migration mobility as that of B9320, both migrated faster than that of A2. Western blotting analysis using strain specific monoclonal antibodies confirmed that the G protein of subgroup B was expressed by rA-G_{B}F_{B} and rA-G_{B}F_{B}ΔM2-2 (Fig. 27B). Western blotting using a polyclonal antibody specific to the M2-2 protein further confirmed the ablation of the M2-2 gene in rA2ΔM2-2 and rA-G_{B}F_{B}ΔM2-2.

Replication of chimeric viruses, rA-G_{B}F_{B} and rA-G_{B}F_{B}ΔM2-2, was compared to rA2 and rA2ΔM2-2 in both the HEp-2 and Vero cells (Fig. 28). In Vero cells, infected at an moi of 0.1, both rA-G_{B}F_{B} and rA-G_{B}F_{B}ΔM2-2 reached peak titers similar to that seen with wild type rA2 and rA2ΔM2-2 respectively. At a lower moi of 0.01, the peak titer of rA-G_{B}F_{B} was slightly reduced compared to rA2; the level of replication of rA-G_{B}F_{B}AM2-2 was reduced by about 10-fold compared to rA-G_{B}F_{B}. In HEp-2 cells, at moi of 0.1, rA-G_{B}F_{B} showed a slightly lower peak titer compared to wt A2 whereas the replication of rA-G_{B}F_{B}ΔM2-2 was reduced by about 100-fold. At moi of 0.01, the peak titer of rA-G_{B}F_{B} was reduced by about 10-fold compared to rA2 and the peak titer ofrA-G_{B}F_{B}AM2-2 was reduced by 100-fold. Therefore, similar to that observed for rA2ΔM2-2, rA-G_{B}F_{B}AM2-2 also exhibited restricted replication in HEp-2 cells, whereas its replication in Vero cells was less impaired.

### Replication of chimeric RSV in cotton rats

Cotton rats are susceptible to both subgroup A and B RSV infection. The levels of replication of rA-G_{B}F_{B} and rA-G_{B}F_{B}ΔM2-2 in the nasal turbinates and lungs of cotton rats were compared with rA2, rA2ΔM2-2 and wild type B9320 (Table 21). The replication of rA-G_{B}F_{B} was below the limit of detection by plaque assay in the nasal turbinates, its replication in lung tissue was reduced by about 3.6 log₁₀ compared to wild type B9320 and by about 2.0 log₁₀ relative to rA2. The replication of rA2ΔM2-2 was not detected in the nasal turbinates and was 1.6 log lower in the lung compared to rA2. Removal of M2-2 from rA-G_{B}F_{B} further attenuated the chimeric virus. No virus replication was detected in either the nasal turbinates or lungs of cotton rats infected with rA-G_{B}F_{B}ΔM2-2.

Although rA-G_{B}F_{B} and rA-G_{B}F_{B}ΔM2-2 were attenuated in cotton rats, both chimeric viruses induced sufficient immunity against RSV to protect the animals from challenge (Table 21). The level of serum anti-RSV neutralizing antibody induced by rA-G_{B}F_{B} was 2.85-fold lower The level of replication of chimeric rA-G_{B}F_{B} was compared with that of wild type B9320. RSV sero-negative AGM were inoculated with 5.5 log₁₀pfu of rA-G_{B}F_{B} or B9320 by intranasal and intratracheal instillation. The throat swab and tracheal lavage samples were collected over 12 days for virus quantitation. B9320 replicated to a level similar to that of wild type A2 virus (Table 22). The peak titer of rA-G_{B}F_{B} in the respiratory tracts of the infected monkeys was about 1000-fold reduced compared to that of B9320. Animals infected with rA-G_{B}F_{B} shed virus for a shorter period than those infected with B9320. Despite its significantly attenuated replication, rA-G_{B}F_{B} provided complete protection when challenged with wild type B9320. No challenge virus was detected in either the upper or lower respiratory tracts of the monkeys previously immunized with rA-G_{B}F_{B}(Table 23). Consistent with the level of protection seen in monkeys immunized with rA-G_{B}F_{B}, the level of serum anti-RSV neutralizing antibody from these monkeys was only marginally reduced (about 2-fold) compared to that observed for wild type B9320 infected animals. The level of serum anti-RSV neutralizing antibody induced by rA-G_{B}F_{B} was augmented by subsequent wild type RSV infections

### 13.4 DISCUSSION

To expedite vaccine development for subgroup B RSV, a recombinant A2 virus was used as a vector to express subgroup B RSV surface antigens. The chimeric virus should elicit a balanced immune response and provide protection against subgroup B RSV infection. As an approach to expressing RSV subgroup B antigens, we constructed a different chimeric virus in which the G and F genes of the A2 strain were completely replaced by the G and F genes of the B9320 strain. The chimeric RSV was then further attenuated using a strategy developed for attenuating the A2 virus.

The recovered chimeric RSV (rA-G_{B}F_{B}) replicated efficiently in Vero cells, but its growth in HEp-2 cells was reduced by 5- to 10-fold relative to rA2. rA-G_{B}F_{B} was attenuated in both the upper and lower respiratory tracts of cotton rats. To determine if the attenuation of rA-G_{B}F_{B} was host specific, this chimeric virus was further evaluated in AGM that are genetically more closely related to humans than rodents. RSV infection in AGM is less well characterized and there is a wide range in the reported peak titer (Crowe et al., 1996, J. Infect. Dis. 173:829-839); (Kakuk et al., 1993, J. Infect. Dis. 167:553-561). Therefore, RSV infection was first tested in AGM using wild type viruses. Both subgroup A and subgroup B RSV were shown to replicate equally well in AGM and virus titers recovered from the upper and lower respiratory tracts of relative to that induced by wild type B9320. Serum anti-RSV neutralizing antibody induced by rA-G_{B}F_{B}ΔM2-2 was approximately 4-fold lower compared to that induced by B9320 and 1.5-fold lower than that of rA-G_{B}F_{B}. By comparison, the level of serum anti-RSV neutralizing antibody induced by rA2ΔM2-2 was similarly reduced by approximately 2-fold compared to that of rA2.

### Replication of wt RSV and rA2AM2-2 in AGM

In order to investigate RSV attenuation and immunogenicity in primates, replication of recombinant RSV was further studied in AGM. Study A examined the replication of recombinant A2 and wild type A2 virus in the respiratory tracts of AGM. RSV sero-negative AGM were infected with 5.5 log₁₀pfu of rA2 or wt A2 intranasally and intratracheally and virus shedding was monitored over a period of 12 days in both the upper and lower respiratory tracts. As shown in Table 22, rA2 replicated well in both the upper and lower respiratory tracts of AGM. rA2 reached a peak titer of 4.18 and 4.28 log₁₀pfu/ml at each site respectively and shed virus over the same length of time as the wild type A2 virus (Table 22, study A), though the peak titer of rA2 in the respiratory tracts of AGM was slightly lower than that obtained for wild type A2 virus. Having confirmed a high level of replication of rA2 in AGM, rA2ΔM2-2 was evaluated for its attenuation, immunogenicity, and protective efficacy in AGM. In a separate study (study B, Table 22), rA2ΔM2-2 showed a greatly reduced level of replication in both the nasopharynx and trachea compared to rA2. Its peak titer in nasopharynx had a reduction of 3.1 log₁₀ while the peak titer in the trachea was reduced by 3.25 log₁₀. Despite the much lower level of replication in the respiratory tracts, rA2ΔM2-2 induced a significant level of serum anti-RSV neutralizing antibody. The antibody titer induced by rA2ΔM2-2 was about 4-fold lower than that induced by rA2 at three weeks post-infection (Table 23). When challenged with wild type A2 virus, rA2ΔM2-2 provided partial protection against wild type RSV replication in the upper respiratory tract and virtually complete protection in the lower respiratory tract of immunized monkeys. Monkeys inoculated with rA2 were fully protected in both the upper and lower respiratory tracts (Table 23). Although rA2ΔM2-2 did not provide complete protection in the respiratory tracts of immunized monkeys, it reduced virus shedding by 5 days. Two weeks after challenge, the level of serum anti-RSV neutralizing antibody from rA2ΔM2-2 infected monkeys approached that induced by rA2.

### Replication of chimeric rA-G_{B}F_{B} and wild type B9320 in AGM

AGM were comparable to those observed in infected Chimpanzees (Crow et al., 1994, Vaccine 12:783-790). When rA-G_{B}F_{B} was evaluated in AGM, it showed a mean peak titer reduction of 3.0 log₁₀ in the upper respiratory tract and a reduction of 2.59 log₁₀ in the lower respiratory tract.

The level of attenuation of rA-G_{B}F_{B} in AGM was consistent with those levels observed in cotton rats. However, this result was somewhat different from that reported for a recently described chimeric RSV in which the G and F genes of A2 were replaced with those of RSV B1 strain (rAB1, Whitehead et al., 1999, J. Virol. 73:9773-80). Though rAB1 and rA-G_{B}F_{B} are similarly attenuated in cotton rats, rAB1 was not attenuated in Chimpanzees. In contrast to rAG_{B}F_{B}, rAB1 replicated better than wt RSV B 1 in both the upper and lower respiratory tracts of Chimpanzees (Whitehead et al., 1999, J. Virol. 73:9773-80). Part of this discrepancy may be explained by the semi-permissiveness of Chimpanzees to wild type subgroup B RSV infection. However, there exists the possibility that rA-G_{B}F_{B} is more attenuated than rAB1 because of differences in the subgroup B strain surface antigens or constellation effects when these antigens are introduced into an A2 background. Therefore, it appears that chimerization of closely related different heterologous proteins can result in different phenotypes. Chimerization of surface antigens resulting in an attenuated virus has been reported for several paramyxoviruses. A chimeric measles virus with the HN and F proteins replaced by the G protein of VSV was highly restricted in replication in vitro (Spielhofer et al., 1998, J. Virol. 72:2150-2159). A chimeric Rinderpest virus in which the F and H proteins were replaced by the heterologous surface proteins of a closely related peste-des-petits-ruminants virus was attenuated in vitro, as indicated by slow virus growth and low virus yield (Das et al., 2000, J. Virol. 74:9039-9047). Most recently, it was reported that the PIV3-PIV2 chimeric virus, in which the F and HN genes of PIV3 were replaced by those of PIV2 was not attenuated in vitro, but it was severely attenuated in hamsters, AGM and Chimpanzees (Tao et al., 2000, J. Virol 74:6448-6458). On the other hand, the chimeric PIV3-PIV1 was not attenuated in vivo (Tao et al., 1998, J. Virol. 72:2955-2961; Tao et al., 1999, Vaccine 17:1100-1108). Though attenuated in AGM, rA-G_{B}F_{B} induced significant levels of anti-RSV neutralizing antibody and provided complete protection against subsequent challenge with wild type subgroup B RSV.

In this study, rA2ΔM2-2 was evaluated for its attenuation, immunogenicity and protection against wild type RSV challenge in AGM. rA2ΔM2-2 was shown to be attenuated in the respiratory tracts of AGM and following challenge, much reduced replication of wild type RSV was observed in animals previously infected with rA2ΔM2-2. The level of replication and protection observed for rA2ΔM2-2 in AGM is very similar to that reported in a Chimpanzee study for a similar recombinant RSV that had the M2-2 protein expression silenced (Bermingham and Collins, 1999, pNAS USA 96:11259-11264; Teng et al., 2000, J. Virol. 74: 9317-9321). rA2ΔM2-2 may prove to be more attenuated in humans than a previously tested vaccine candidate cpts248/404 (Teng et al., 2000, J. Virol. 74: 9317-9321). cpts248/404 was neither sufficiently attenuated nor genetically stable in naive infants (Crowe et al., 1994, Vaccine 12:783-790; Wright et al., 2000, J. Infect. Dis. 182:1331-1342). The serum anti-RSV neutralizing antibody titer induced by rA2ΔM2-2 was slightly lower than that induced by the wild type RSV infection. However, the augmentation of neutralizing antibody titer after the challenge suggests that the immunogenicity of rA2ΔM2-2 could be enhanced by repeat administrations.

Since rA2ΔM2-2 exhibits many of the desired features in a live attenuated vaccine, the deletion of the M2-2 gene was considered as an appropriate way to further attenuate the chimeric rAG_{B}F_{B}. In vitro study indicated that rA-G_{B}F_{B}ΔM2-2 had similar level of attenuation as rA2ΔM2-2, exhibiting increased syncytial formation, reduced growth in HEp-2 cells and unbalanced RNA transcription to replication. As the chimeric rA-G_{B}F_{B} virus is already attenuated in both the cotton rats and AGM, rA-G_{B}F_{B}ΔM2-2 is expected to be more attenuated than rA2ΔM2-2. However, cotton. rats studies indicated that rA-G_{B}F_{B}ΔM2-2 was still capable of inducing a level of serum RSV neutralizing antibody approaching that induced by rA-G_{B}F_{B} and provided complete protection against subsequent experimental challenge. Therefore, rA-G_{B}F_{B}ΔM2-2 may represent a suitable vaccine candidate for protecting against subgroup B RSV infection

The present invention is not to be limited in scope by the specific embodiments described which are intended as single illustrations of individual aspects of the invention, and any constructs, viruses or enzymes which are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claims.

## Claims

1. An isolated infectious respiratory syncytial virus particle having an attenuated phenotype comprising a respiratory syncycial virus antigenome or genome wherein said genome or antigenome has: (a) a heterologous sequence encoding a G and F protein, from RSV strain B9320; and (b) a deletion of the M2-2 open reading frame.

2. The isolated infectious respiratory syncytial virus particle according to claim 1 wherein the heterologous G and F genes replace the G and F genes of the host virus genome.

3. The isolated infectious respiratory syncytial virus particle of claim 1, wherein said heterologous sequence is derived from a different strain of respiratory syncytial virus.

4. An isolated cDNA encoding an infectious respiratory syncytial virus particle having an attenuated phenotype comprising a respiratory syncycial virus antigenome or genome wherein said genome or antigenome has: (a) a heterologous sequence encoding a G and F protein, from RSV strain B9320; and (b) a deletion of the M2-2 open reading frame.

5. The isolated cDNA encoding an infectious respiratory syncytial virus particle according to claim 4 wherein the heterologous G and F genes replace the G and F genes of the host virus genome.

6. A vaccine comprising (a) the isolated infectious respiratory syncytial virus of any one of claims 1 to 3; and (b) a pharmaceutically acceptable carrier.

7. A pharmaceutical composition comprising (a) the isolated infectious respiratory syncytial virus of any one of claims 1 to 3; and (b) a pharmaceutically acceptable carrier.

## Patentansprüche

1. Isolierter infektiöser RSV ("Respiratory Syncytial Virus")- Partikel, der einen attenuierten Phänotyp aufweist, umfassend ein RSV-Antigenom oder-Genom, wobei das Genom oder Antigenom Folgendes aufweist: (a) eine heterologe Sequenz, die für G- und F- Protein von dem RSV-Stamm B9320 codieren; und (b) eine Deletion des offenen Leserahmens M2-2.

2. Der isolierte infektiöse RSV-Partikel entsprechend Anspruch 1, wobei die heterologen G- und F- Gene die G- und F- Gene des Wirts-Virusgenoms ersetzen.

3. Der isolierte infektiöse RSV-Partikel nach Anspruch 1, wobei die heterologe Sequenz von einem anderen RSV-Stamm abgeleitet ist.

4. Isolierte cDNA, die für einen infektiösen RSV-Partikel codiert, der einen attenuierten Phänotyp aufweist, umfassend ein RSV-Antigenom oder-Genom, wobei das Genom oder Antigenom folgendes aufweist: (a) eine heterologe Sequenz, die für G- und F-Proteine von dem Stamm B9320 codieren; und (b) eine Deletion des offenen Leserahmens M2-2.

5. Die isolierte cDNA, die für einen RSV-Partikel codiert, nach Anspruch 4, wobei die heterologen G- und F-Gene die G- und F-Gene des Wirts-Virusgenoms ersetzen.

6. Ein Impfstoff bzw. Vakzin umfassend (a) den isolierten infektiösen RSV nach einem der Ansprüche 1 bis 3; und (b) einen pharmazeutischen annehmbaren Trägerstoff.

7. Pharmazeutische Zusammensetzung umfassend (a) den isolierten infektiösen RSV nach einem der Ansprüche 1-3; und (b) einen pharmazeutisch annehmbaren Trägerstoff.

## Revendications

1. Particule du virus respiratoire syncytial infectieux isolée possédant un phénotype atténué comprenant un antigénome ou génome du virus respiratoire syncytial dans lequel ledit génome ou antigénome possède : (a) une séquence hétérologue codant une protéine G et F, de la souche de RSV B9320 ; et (b) une délétion du cadre ouvert de lecture M2-2

2. Particule du virus respiratoire syncytial infectieux isolé selon la revendication 1, dans laquelle les gènes G et F hétérologues remplacent les gènes G et F du génome du virus hôte.

3. Particule du virus respiratoire syncytial infectieux isolé selon la revendication 1, dans laquelle ladite séquence hétérologue est dérivée depuis une souche différente du virus respiratoire syncytial.

4. ADNc isolé codant une particule du virus respiratoire syncytial infectieux possédant un phénotype atténué comprenant un antigénome ou génome du virus respiratoire syncytial dans lequel ledit génome ou antigénome possède : (a) une séquence hétérologue codant une protéine G et F, de la souche de VRS B9320 ; et (b) une délétion du cadre ouvert de lecture M2-2.

5. ADNc isolé codant une particule du virus respiratoire syncytial infectieux selon la revendication 4, dans lequel les gènes G et F hétérologues remplacent les gènes G et F du génome du virus hôte.

6. Vaccin comprenant (a) le virus respiratoire syncytial infectieux isolé selon l'une quelconque des revendications 1 à 3 ; et (b) un excipient pharmaceutiquement acceptable.

7. Composition pharmaceutique comprenant (a) le virus respiratoire syncytial infectieux isolé selon l'une quelconque des revendications 1 à 3 ; et (b) un excipient pharmaceutiquement acceptable.
